# EUROPEAN PATENT APPLICATION

(11) **EP 4 850 693 A1**
(43) Date of publication of application: **09.12.2026**
(21) Application number: 25748179.6
(22) Date of filing: 28.01.2025
(51) Int. Cl.: A61K 45/00, A61K 31/337, A61K 31/4745, A61K 31/7068, A61K 39/395, A61K 47/68, A61P 35/00, A61P 43/00, C07K 16/28, C12N 15/13

(54) **COMBINATION OF ANTI-HER2 ANTIBODY-DRUG CONJUGATE WITH OTHER AGENTS**

(30) Priority: 29.01.2024 JP 2024010751
(71) Applicant: Daiichi Sankyo Company, Limited, Tokyo 103-8426 (JP)
(72) Inventor: HARA Kyoko, Tokyo 103-8426 (JP); ISHIZAKI Masayuki, Tokyo 103-8426 (JP); IWATA Tomomi, Tokyo 103-8426 (JP); WADA Teiji, Tokyo 103-8426 (JP)
(74) Representative: Marks & Clerk LLP
(86) International application number: PCT/JP2025/002542
(87) International publication number: WO 2025/164597

(57) **Abstract**

[Object] To provide a novel drug combination therapy for diseases, particularly for cancer. [Solution Means] To provide administration in combination of an anti-HER2 antibody-drug conjugate and an additional agent, such as an immune checkpoint inhibitor.

## Description

### Technical Field

The present invention relates to a pharmaceutical composition in which a specific anti-HER2 antibody-drug conjugate and an additional agent, such as an immune checkpoint inhibitor, are used in combination; a therapeutic method in which a specific anti-HER2 antibody-drug conjugate and an additional agent, such as an immune checkpoint inhibitor, are administered in combination to a subject; and the like.

### Background Art

In cancer treatment, curing or life-extending effects are expected by removing a cancerous part through surgery, or by killing cancer cells with anticancer agents or radiation. However, a single anticancer agent treatment alone cannot provide sufficient effects for many cancers; thus, treatment is being attempted through combination with various pharmaceutical agents. Examples of anticancer agents include chemotherapeutic agents, such as paclitaxel, capecitabine, irinotecan, doxorubicin, carboplatin, taxol, or camptothecin (Non-Patent Literature 1); molecular-targeted drugs, such as imatinib, crizotinib, dasatinib, and lapatinib; therapeutic antibodies for cancers, such as trastuzumab (Patent Document 1), bevacizumab, cetuximab, and ramucirumab; and antibody-drug conjugates, such as trastuzumab emtansine (Patent Document 2). The anticancer agent to be used and its combination therapy are determined according to the type of cancer, the degree of progression, and the treatment status.

Immune checkpoint inhibitors, which are becoming standard-of-care drugs in recent years, are agents that inhibit the immunosuppressive system and activate anti-tumor immunity (Non-Patent Literature 2 to 4). Known examples of immune checkpoint inhibitors include anti-PD-1 antibodies, such as nivolumab (Patent Document 3), pembrolizumab (Patent Document 4), spartalizumab (Patent Document 5), cemiplimab (Patent Document 6), and dostarlimab; anti-PD-L1 antibodies, such as atezolizumab (Patent Document 7), durvalumab (Patent Document 8), and avelumab (Patent Document 9); anti-CTLA-4 antibodies, such as ipilimumab (Patent Document 10) and tremelimumab (Patent Document 11); anti-TIGIT antibodies, such as tiragolumab (Patent Document 12) and vibostolimab (Patent Document 13); and anti-LAG-3 antibodies, such as relatlimab (Patent Document 14).

A stimulator of interferon genes (STING) is a transmembrane adaptor protein localized in the endoplasmic reticulum (Non-Patent Literature 5). STING functions as a central molecule in innate immune activation in mammals and serves as the first line of defense against the invasion of pathogens, such as bacteria and viruses. STING activation is known to be triggered by signals generated when plural cytoplasmic DNA sensors senses exogenous and endogenous DNA (Non-Patent Literature 6 to 8).

A recent study has shown that STING promotes not only host defense against microorganisms but also anti-tumor immunity (Non-Patent Literature 9).

Patent Documents 15 to 18 describe conjugates in which a STING agonist is used as an immunostimulatory compound and is bound to an antibody via a linker, and include examples of in vivo administration.

Patent Document 19 describes an example of in vivo administration of a conjugate in which a STING agonist and an antibody are bound via a linker, in combination with trastuzumab or an anti-PD-1 antibody.

However, the effect of use of a conjugate in which a STING agonist having a specific cyclic dinucleotide structure and an antibody are bound (Patent Document 17) in combination with an additional agent remains unknown.

### Citation List

### Patent Document

Patent Document 1: US 5821337
Patent Document 2: WO 2001/000244
Patent Document 3: WO 2006/121168
Patent Document 4: WO 2008/156712
Patent Document 5: WO 2015/112900
Patent Document 6: WO 2015/196051
Patent Document 7: WO 2010/077634
Patent Document 8: WO 2011/066389
Patent Document 9: WO 2013/079174
Patent Document 10: WO 2001/014424
Patent Document 11: WO 2000/037504
Patent Document 12: WO 2017/053748
Patent Document 13: WO 2016/028656
Patent Document 14: WO 2014/008218
Patent Document 15: WO 2021/202984
Patent Document 16: WO 2022/097117
Patent Document 17: WO 2020/050406
Patent Document 18: WO 2021/177438
Patent Document 19: WO 2023/172906

### Non-Patent Literature

Non-Patent Literature 1: Genes & Diseases 2023, 10, 1367-1401.
Non-Patent Literature 2: Cancers 2016, 8, 106.
Non-Patent Literature 3: Nat. Rev. Cancer 2012, 12, 252-264.
Non-Patent Literature 4: Cell 2015, 162, 937.
Non-Patent Literature 5: Nature 2008, 455, 674-678.
Non-Patent Literature 6: Mol. Cell 2013, 51, 226-235.
Non-Patent Literature 7: Science 2015, 347, aaa2630.
Non-Patent Literature 8: J. Virol. 2014, 88, 5328-5341.
Non-Patent Literature 9: Immunity 2014, 41, 830-842.

### Summary of Invention

### Technical Problem

An object of the present invention is to provide a novel drug combination therapy for diseases, particularly for cancer.

### Solution to Problem

The present inventors have conducted diligent studies to achieve the above object and found that combined administration of an anti-HER2 antibody-drug conjugate of Formula (I) below with an additional agent, such as an immune checkpoint inhibitor, safely exhibits excellent combination effects (in particular, remarkable anti-tumor effects), thereby completing the present invention.

That is, the present invention provides [A1] to [D28] below.

[A1] A pharmaceutical composition comprising a) an anti-HER2 antibody-drug conjugate, wherein
   a) the anti-HER2 antibody-drug conjugate, and
   b) one or more selected from an immune checkpoint inhibitor, a chemotherapeutic agent, a molecular-targeted drug, an anti-HER2 antibody, and an anti-HER2 antibody-containing compound, provided that the anti-HER2 antibody-drug conjugate described in a) and anti-HER2 antibody-deruxtecan are excluded,
      are used in combination, and
      the anti-HER2 antibody-drug conjugate is represented by Formula (I) below: 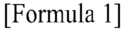 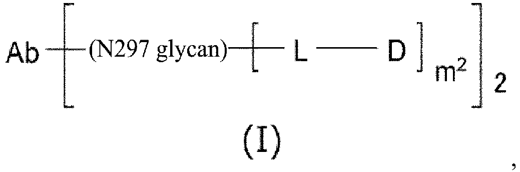
      wherein m² represents an integer of 1 or 2;
      L represents a linker connecting the N297 glycan and a drug D, and represents -Lb-La-Lp-Lc-*, wherein
      the asterisk represents binding to the drug D;
      Lb represents: 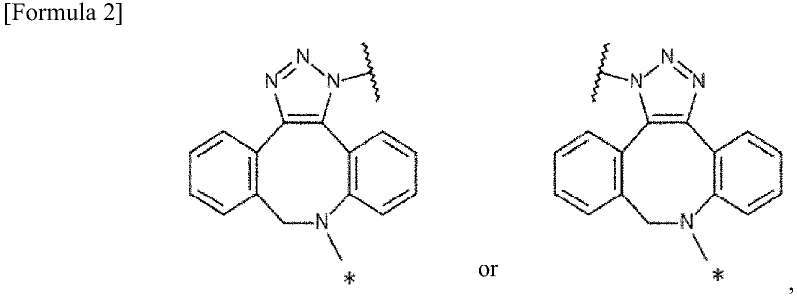
      wherein in the structural formula of Lb shown above, the asterisk represents binding to La, and the wavy line represents binding to the N297 glycan;
      La represents -C(=O)-CH₂CH₂-C(=O)-;
      Lp represents -GGFG- or -GGPI-; and
      Lc represents -NH-CH₂-;
      Ab is an anti-HER2 antibody comprising a heavy chain comprising CDRH1 consisting of the amino acid sequence represented by SEQ ID NO: 3, CDRH2 consisting of the amino acid sequence represented by SEQ ID NO: 4, and CDRH3 consisting of the amino acid sequence represented by SEQ ID NO: 5, and a light chain comprising CDRL1 consisting of the amino acid sequence represented by SEQ ID NO: 6, CDRL2 consisting of the amino acid sequence represented by SAS, and CDRL3 consisting of the amino acid sequence represented by SEQ ID NO: 7, or an antigen-binding fragment of the antibody;
      the N297 glycan is N297-(Fuc)MSG1 having a structure represented by a formula below: 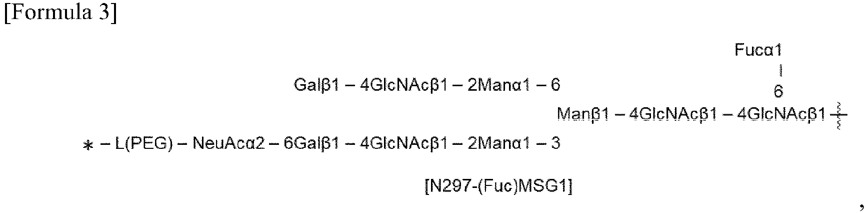
      wherein the wavy line represents binding to Asn297 of Ab;
      L(PEG) represents -(CH₂-CH₂-O)n⁵-CH₂-CH₂-NH-, and the amino group at the right end of the L(PEG) is amide-linked to the carboxyl group at the 2-position of the non-reducing-end sialic acid on the β-Man 1-3 branch chain of the N297 glycan;
      the asterisk represents binding to the nitrogen atom at the 1-position or 3-position on the 1,2,3-triazole ring of Lb in the linker L; and
      n⁵ represents an integer of 2 to 5; or
      N297-(Fuc)SG having a structure represented by the following formula: 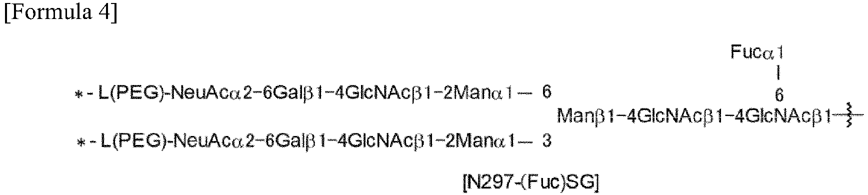
      wherein the wavy line represents binding to Asn297 of Ab;
      L(PEG) represents -(CH₂-CH₂-O)n⁵-CH₂-CH₂-NH-, and the amino group at the right end of the L(PEG) is amide-linked to the carboxyl group at the 2-position of the non-reducing-end sialic acid on each of the β-Man 1-3 and 1-6 branch chains of the N297 glycan;
      the asterisk represents binding to the nitrogen atom at the 1-position or 3-position on the 1,2,3-triazole ring of Lb in the linker L; and
      n⁵ represents an integer of 2 to 5; and
      D is 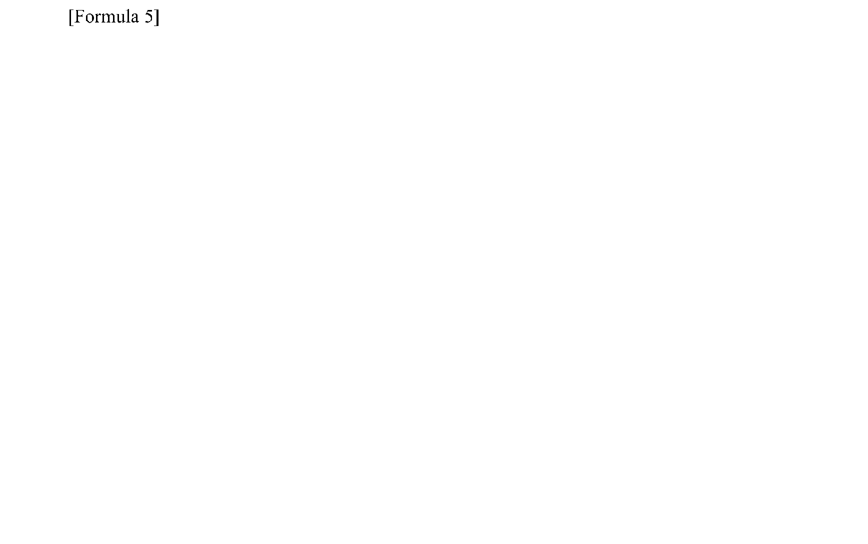 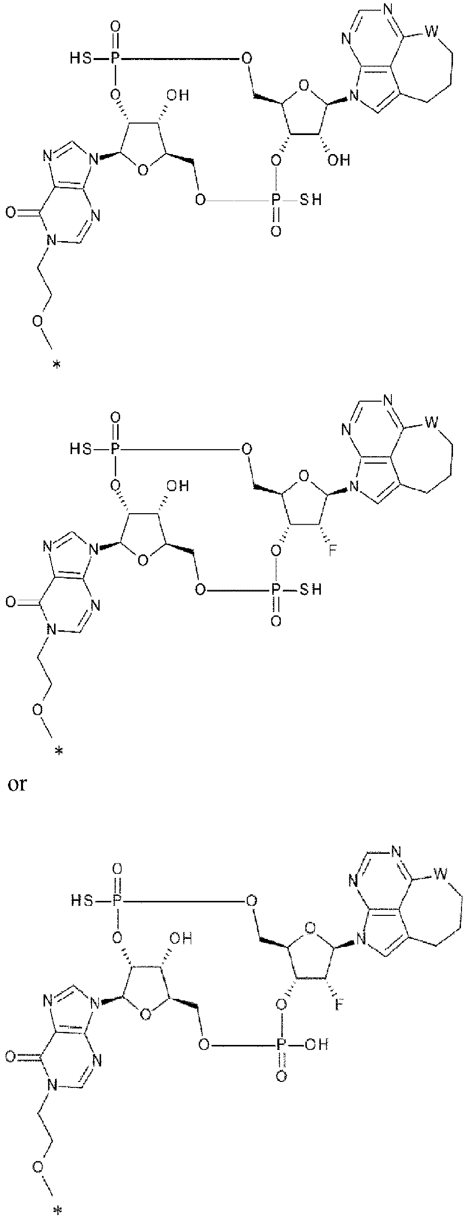 wherein the asterisk represents binding to L, and W represents -NH-.
[A1'] The pharmaceutical composition according to [A1], wherein Ab is an anti-HER2 antibody comprising an Fc region (wild-type or variant) of an antibody, or an antigen-binding fragment of the antibody.
[A2] The pharmaceutical composition according to [A1], wherein
   D is represented by 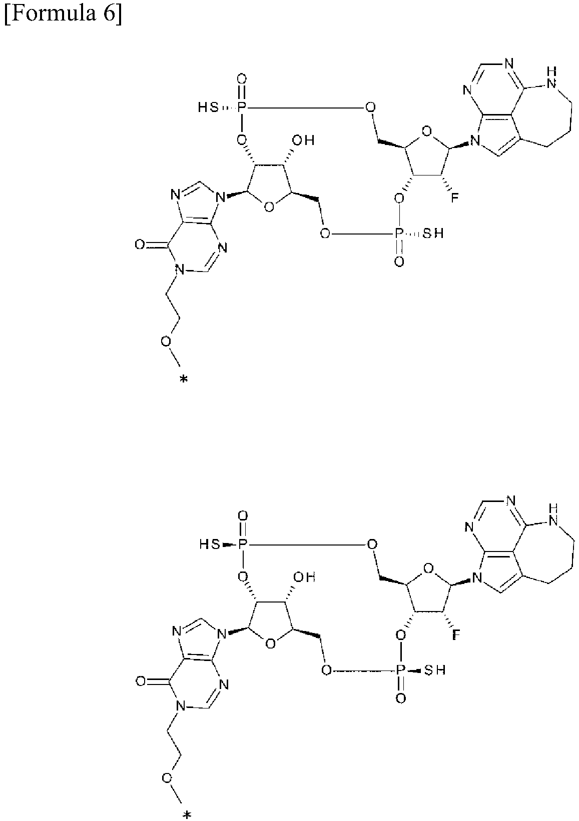 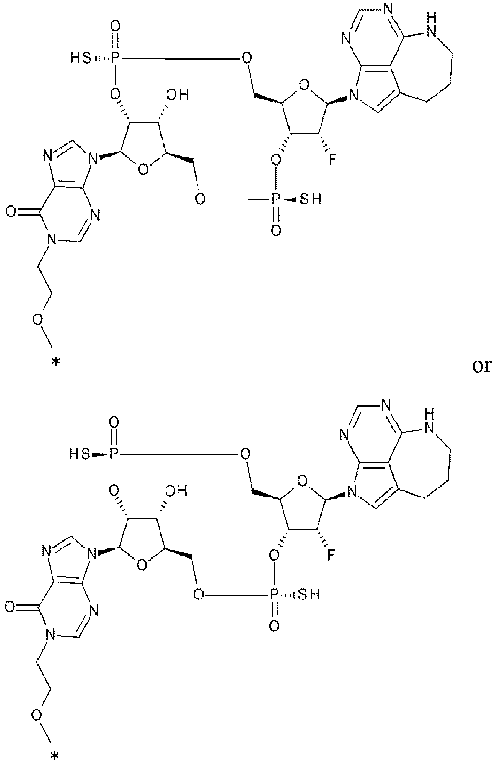
   wherein the asterisk represents binding to L.
[A3] The pharmaceutical composition according to [A1], wherein the anti-HER2 antibody-drug conjugate is represented by a formula below: 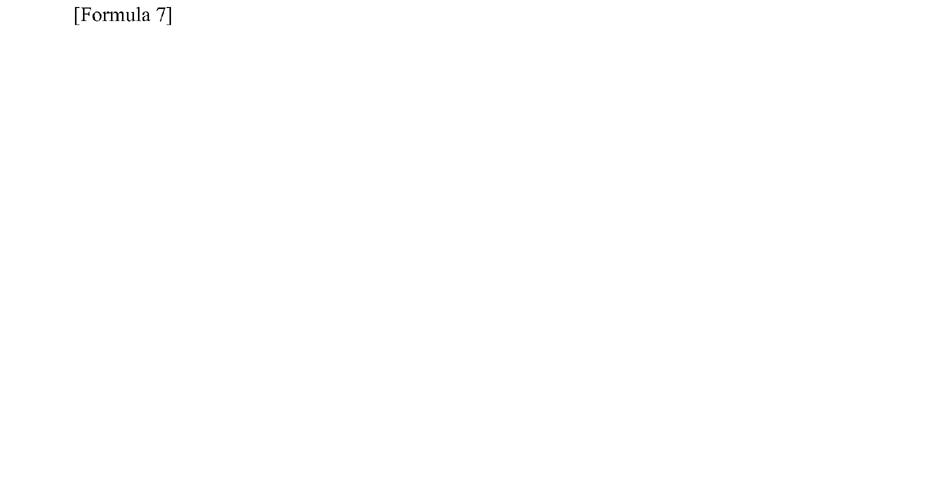 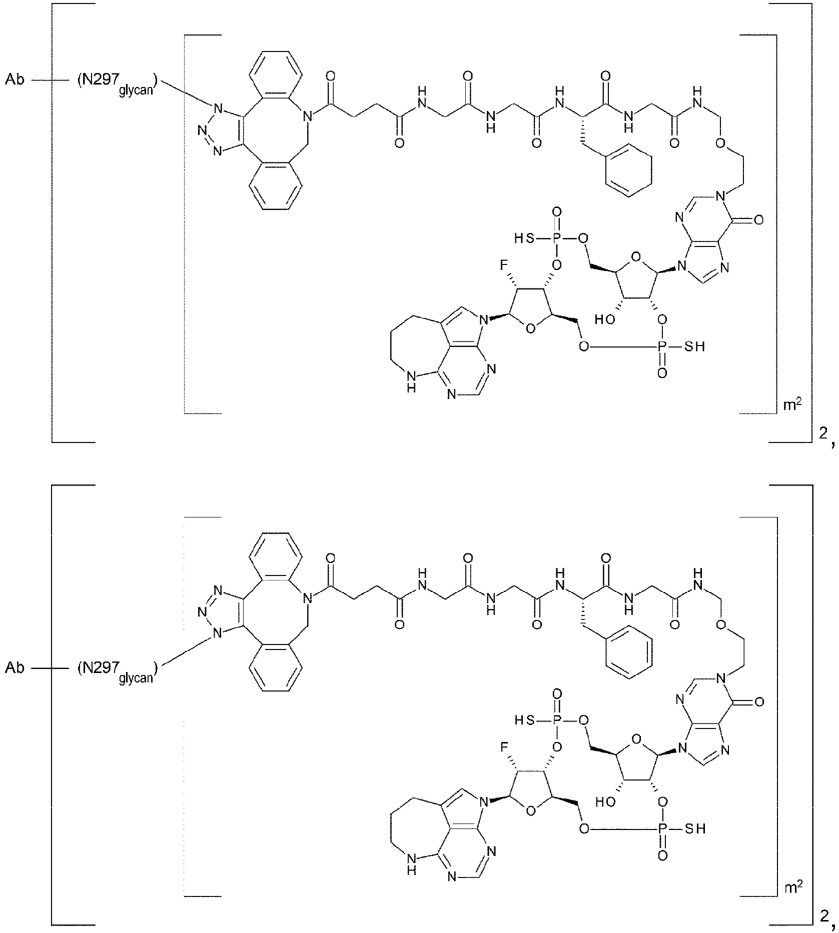 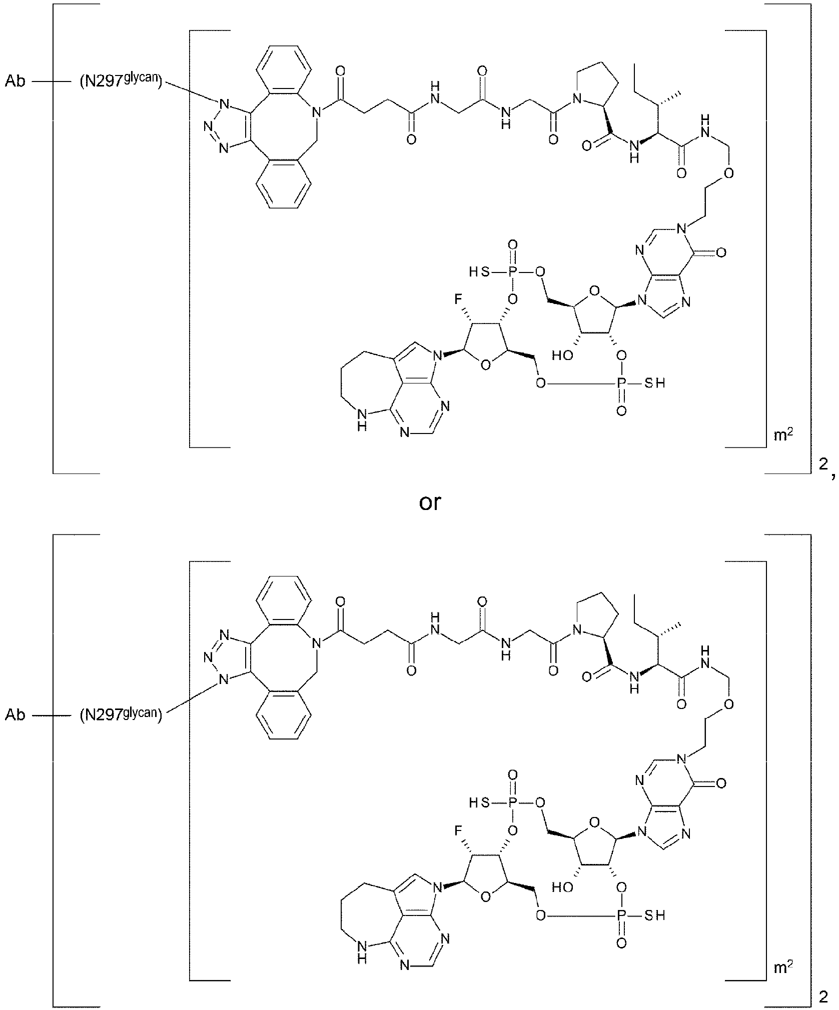 wherein Ab, the N297 glycan, and m² are as defined above.
[A4] The pharmaceutical composition according to any one of [A1] to [A3], wherein
   the anti-HER2 antibody-drug conjugate is represented by a formula below: 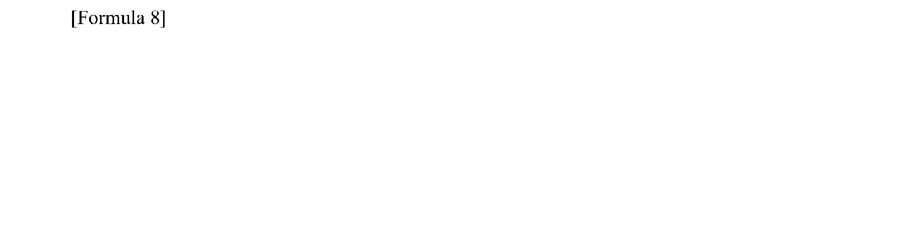 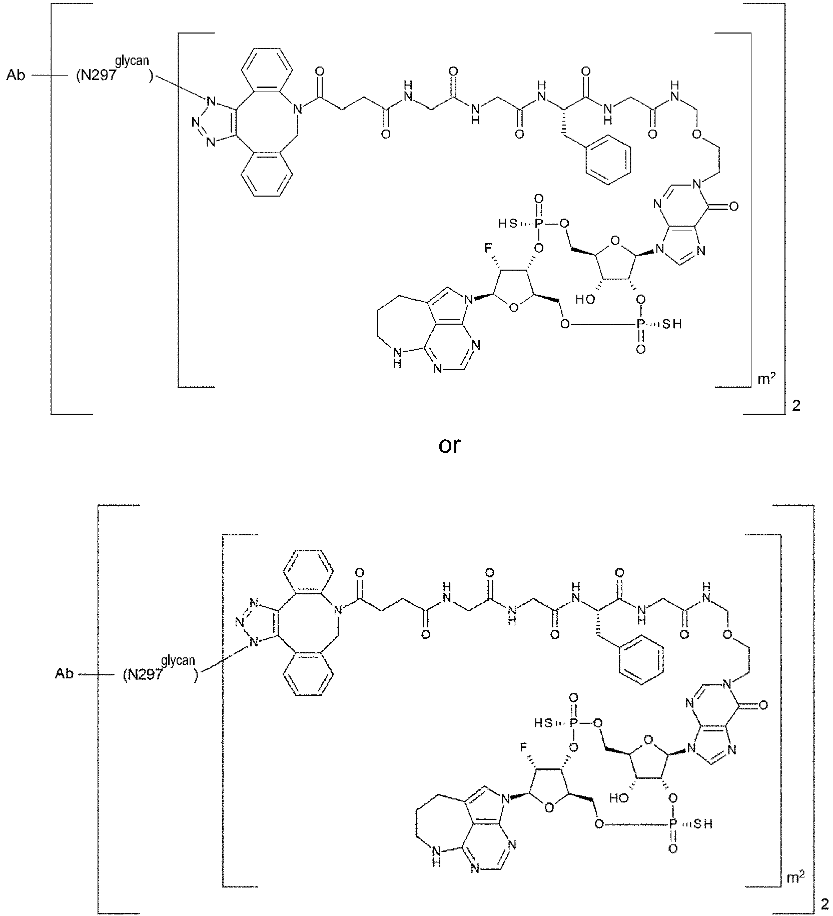
   wherein m² is 2;
   Ab comprises a heavy chain variable region consisting of an amino acid sequence having 80% or more, preferably 90% or more, and more preferably 95% or more identity with the amino acid sequence represented by the amino acid residues 1 to 119 of SEQ ID NO: 1, and a light chain variable region consisting of an amino acid sequence having 80% or more, preferably 90% or more, and more preferably 95% or more identity with the amino acid sequence represented by the amino acid residues 1 to 108 of SEQ ID NO: 2; and
   the N297 glycan is N297-(Fuc)SG having a structure represented by a formula below: 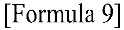 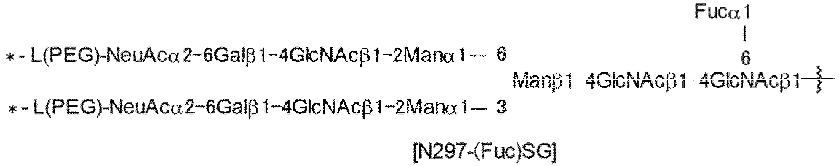
   wherein the wavy line represents binding to Asn297 of Ab,
   L(PEG) represents -(CH₂-CH₂-O)n⁵-CH₂-CH₂-NH-, and the amino group at the right end of the L(PEG) is amide-linked to the carboxyl group at the 2-position of the non-reducing-end sialic acid on each of the β-Man 1-3 and 1-6 branch chains of the N297 glycan,
   the asterisk represents binding to the nitrogen atom at the 1-position or 3-position on the 1,2,3-triazole ring, and
   n⁵ represents an integer of 3.
[A5] The pharmaceutical composition according to any one of [A1] to [A4], wherein Ab comprises a heavy chain variable region consisting of the amino acid sequence represented by the amino acid residues 1 to 119 of SEQ ID NO: 1, and a light chain variable region consisting of the amino acid sequence represented by the amino acid residues 1 to 108 of SEQ ID NO: 2.
[A6] The pharmaceutical composition according to any one of [A1] to [A5], wherein Ab comprises a heavy chain consisting of an amino acid sequence having 80% or more, preferably 90% or more, and more preferably 95% or more identity with the amino acid sequence represented by SEQ ID NO: 1, and a light chain consisting of an amino acid sequence having 80% or more, preferably 90% or more, and more preferably 95% or more identity with the amino acid sequence represented by SEQ ID NO: 2.
[A7] The pharmaceutical composition according to any one of [A1] to [A6], wherein Ab comprises a heavy chain consisting of the amino acid sequence represented by SEQ ID NO: 1, and a light chain consisting of the amino acid sequence represented by SEQ ID NO: 2.
[A8] The pharmaceutical composition according to any one of [A1] to [A5], wherein Ab comprises (i) a heavy chain consisting of an amino acid sequence in which one or two amino acids are deleted from the carboxyl terminus of the amino acid sequence of the heavy chain included in the antibody or the antigen-binding fragment of the antibody included in the pharmaceutical composition described in any one of [A1] to [A7]; and (ii) the light chain included in the antibody or the antigen-binding fragment of the antibody included in the pharmaceutical composition described in any one of [A1] to [A7].
[A9] The pharmaceutical composition according to any one of [A1] to [A8], wherein a) the anti-HER2 antibody-drug conjugate and b) an immune checkpoint inhibitor are used in combination; and the immune checkpoint inhibitor is an antibody selected from the group consisting of an anti-PD-1 antibody, an anti-PD-L1 antibody, an anti-CTLA-4 antibody, an anti-TIGIT antibody, and an anti-LAG-3 antibody; an antigen-binding fragment of the antibody; or a compound including either the antibody or the antigen-binding fragment.
[A10] The pharmaceutical composition according to [A9], wherein the anti-PD-1 antibody is nivolumab, pembrolizumab, spartalizumab, cemiplimab, or dostarlimab.
[A11] The pharmaceutical composition according to [A9], wherein the anti-PD-L1 antibody is atezolizumab, durvalumab, or avelumab.
[A12] The pharmaceutical composition according to [A9], wherein the anti-CTLA-4 antibody is ipilimumab or tremelimumab.
[A13] The pharmaceutical composition according to [A9], wherein the anti-TIGIT antibody is tiragolumab or vibostolimab.
[A14] The pharmaceutical composition according to [A9], wherein the anti-LAG-3 antibody is relatlimab.
[A15] The pharmaceutical composition according to any one of [A1] to [A8], wherein a) the anti-HER2 antibody-drug conjugate and b) a chemotherapeutic agent are used in combination, and the chemotherapeutic agent is an antimetabolite, a tubulin inhibitor, an alkylating agent, a platinum-based agent, or a topoisomerase inhibitor.
[A16] The pharmaceutical composition according to [A15], wherein the antimetabolite is capecitabine or a pharmaceutically acceptable salt of capecitabine.
[A17] The pharmaceutical composition according to [A15], wherein the tubulin inhibitor is paclitaxel or a pharmaceutically acceptable salt of paclitaxel.
[A18] The pharmaceutical composition according to [A15], wherein the topoisomerase inhibitor is irinotecan or a pharmaceutically acceptable salt of irinotecan.
[A19] The pharmaceutical composition according to any one of [A1] to [A8], wherein a) the anti-HER2 antibody-drug conjugate and b) an anti-HER2 antibody are used in combination, and the anti-HER2 antibody described in b) is trastuzumab, pertuzumab, or margetuximab.
[A20] The pharmaceutical composition according to any one of [A1] to [A8], wherein a) the anti-HER2 antibody-drug conjugate and b) an anti-HER2 antibody-containing compound are used in combination, and the anti-HER2 antibody-containing compound is ado-trastuzumab emtansine (T-DM1), disitamab vedotin, or vic-trastuzumab duocarmazine.
[A21] The pharmaceutical composition according to any one of [A1] to [A20], wherein
   a) the anti-HER2 antibody-drug conjugate; and
   b) the immune checkpoint inhibitor, the chemotherapeutic agent, the molecular-targeted drug, the anti-HER2 antibody, or the anti-HER2 antibody-containing compound
   are each included as an active ingredient in different formulations and are administered simultaneously or at different times (sequentially or separately).
[A22] The pharmaceutical composition according to any one of [A1] to [A21], for the treatment of cancer.
[A23] The pharmaceutical composition according to [A22], wherein the cancer is at least one selected from the group consisting of lung cancer, colorectal cancer, prostate cancer, pancreatic cancer, ovarian cancer, breast cancer, bladder cancer, gastric cancer, esophageal cancer, endometrial cancer, cervical cancer, head and neck cancer, bile duct cancer, myeloma, and sarcoma.
[A24] The pharmaceutical composition according to [A23], wherein the cancer is lung cancer.
[A25] The pharmaceutical composition according to [A23], wherein the cancer is colorectal cancer.
[A26] The pharmaceutical composition according to [A23], wherein the cancer is breast cancer.
[A27] The pharmaceutical composition according to [A23], wherein the cancer is gastric cancer.
[A28] The pharmaceutical composition according to [A23], wherein the cancer is bladder cancer.
[B1] An anti-HER2 antibody-drug conjugate for use in treating cancer in combination with one or more selected from an immune checkpoint inhibitor, a chemotherapeutic agent, a molecular-targeted drug, an anti-HER2 antibody, and an anti-HER2 antibody-containing compound, provided that the anti-HER2 antibody-drug conjugate represented by Formula (I) described below and anti-HER2 antibody-deruxtecan are excluded, wherein
   the anti-HER2 antibody-drug conjugate is represented by Formula (I) below: 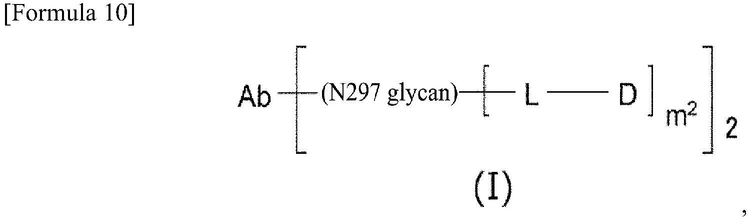
   wherein m² represents an integer of 1 or 2;
   L represents a linker connecting the N297 glycan and a drug D, and represents -Lb-La-Lp-Lc-*, wherein
   the asterisk represents binding to the drug D;
   Lb represents: 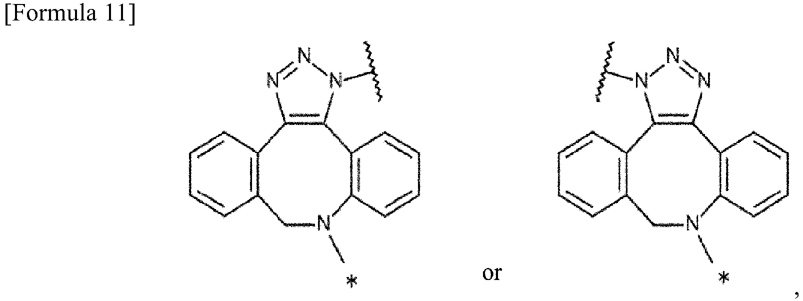
   wherein in the structural formula of Lb shown above, the asterisk represents binding to La, and the wavy line represents binding to the N297 glycan;
   La represents -C(=O)-CH₂CH₂-C(=O)-;
   Lp represents -GGFG- or -GGPI-; and
   Lc represents -NH-CH₂-;
   Ab is an anti-HER2 antibody comprising a heavy chain comprising CDRH1 consisting of the amino acid sequence represented by SEQ ID NO: 3, CDRH2 consisting of the amino acid sequence represented by SEQ ID NO: 4, and CDRH3 consisting of the amino acid sequence represented by SEQ ID NO: 5, and a light chain comprising CDRL1 consisting of the amino acid sequence represented by SEQ ID NO: 6, CDRL2 consisting of the amino acid sequence represented by SAS, and CDRL3 consisting of the amino acid sequence represented by SEQ ID NO: 7, or an antigen-binding fragment of the antibody;
   the N297 glycan is N297-(Fuc)MSG1 having a structure represented by a formula below: 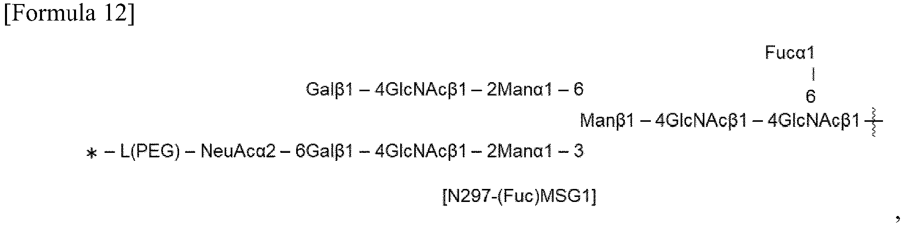
   wherein the wavy line represents binding to Asn297 of Ab;
   L(PEG) represents -(CH₂-CH₂-O)n⁵-CH₂-CH₂-NH-, and the amino group at the right end of the L(PEG) is amide-linked to the carboxyl group at the 2-position of the non-reducing-end sialic acid on the β-Man 1-3 branch chain of the N297 glycan;
   the asterisk represents binding to the nitrogen atom at the 1-position or 3-position on the 1,2,3-triazole ring of Lb in the linker L; and
   n⁵ represents an integer of 2 to 5; or
   N297-(Fuc)SG having a structure represented by the following formula: 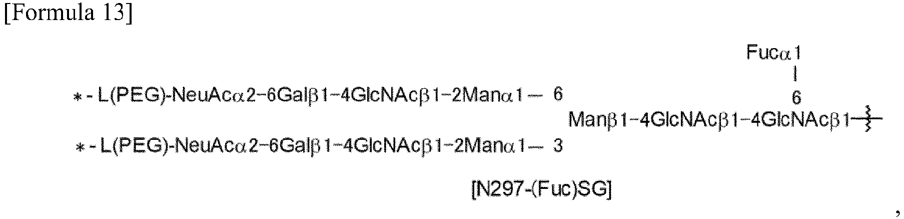
   wherein the wavy line represents binding to Asn297 of Ab;
   L(PEG) represents -(CH₂-CH₂-O)n⁵-CH₂-CH₂-NH-, and the amino group at the right end of the L(PEG) is amide-linked to the carboxyl group at the 2-position of the non-reducing-end sialic acid on each of the β-Man 1-3 and 1-6 branch chains of the N297 glycan;
   the asterisk represents binding to the nitrogen atom at the 1-position or 3-position on the 1,2,3-triazole ring of Lb in the linker L; and
   n⁵ represents an integer of 2 to 5; and
   D is 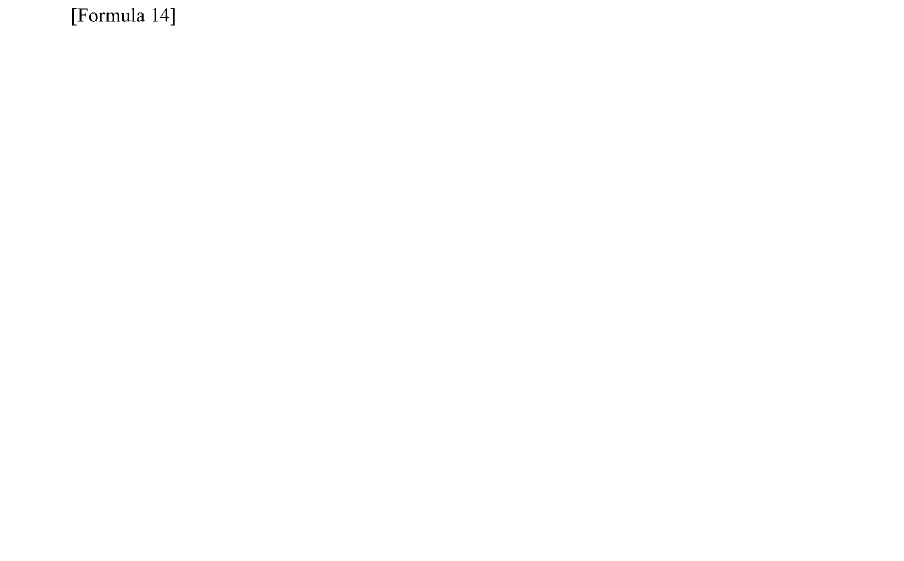 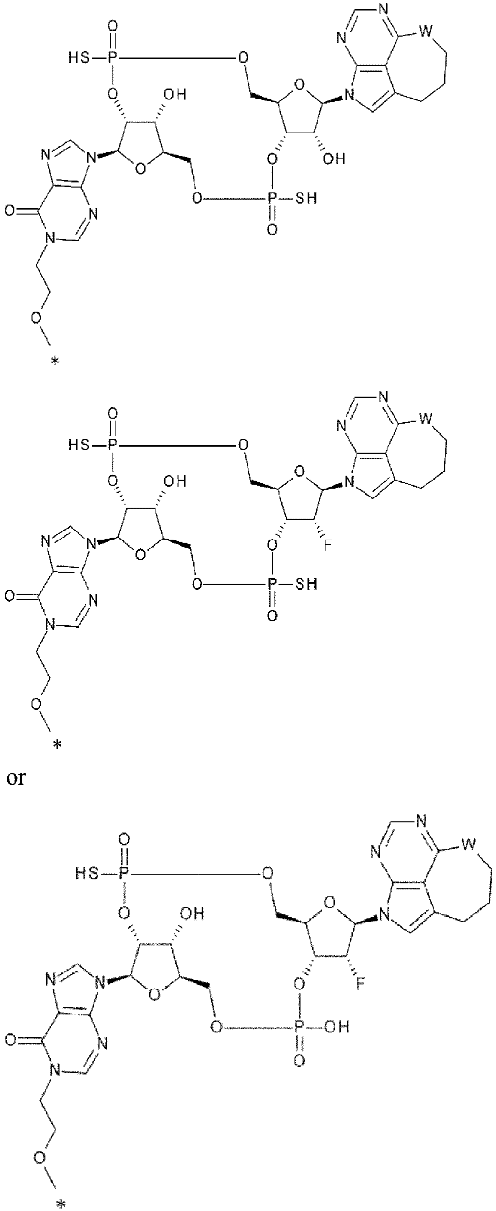
   wherein the asterisk represents binding to L, and W represents -NH-.
[B1'] The anti-HER2 antibody-drug conjugate according to [B1], wherein Ab is an anti-HER2 antibody comprising an Fc region (wild-type or variant) of an antibody, or an antigen-binding fragment of the antibody .
[B2] The anti-HER2 antibody-drug conjugate according to [B1], wherein
   D is represented by 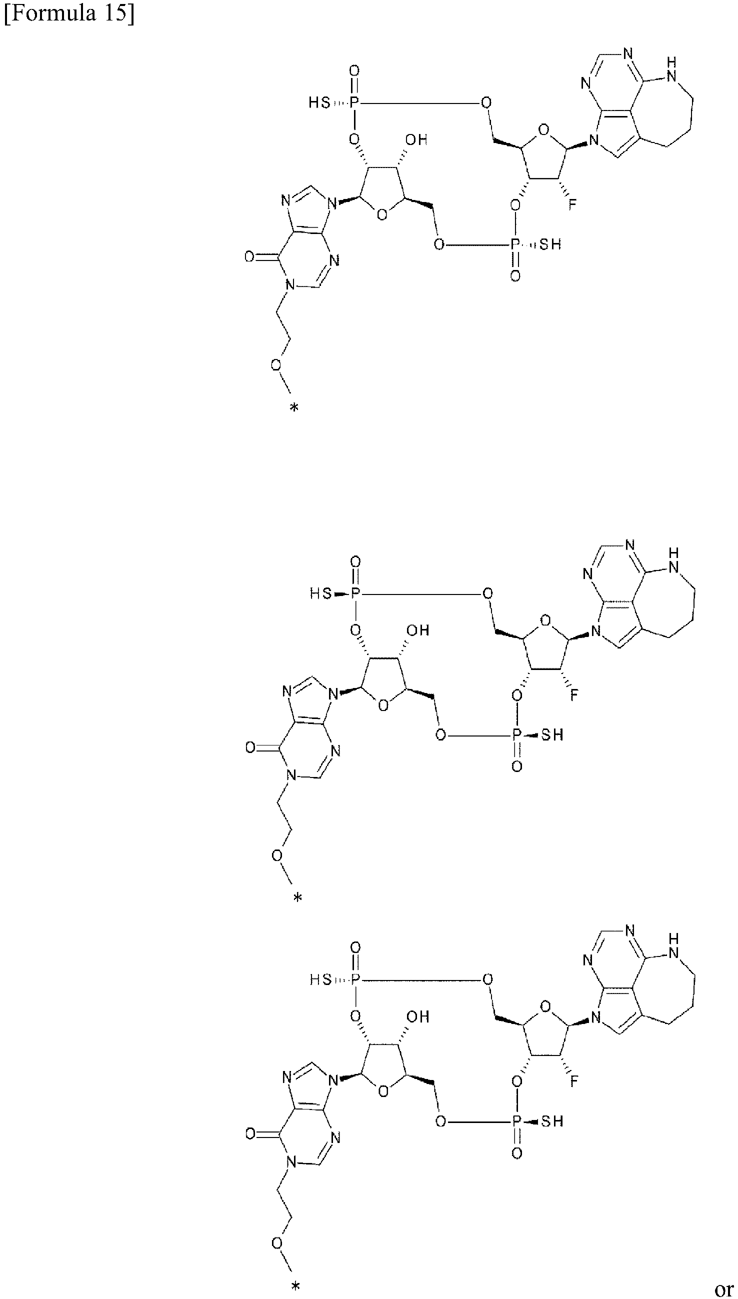 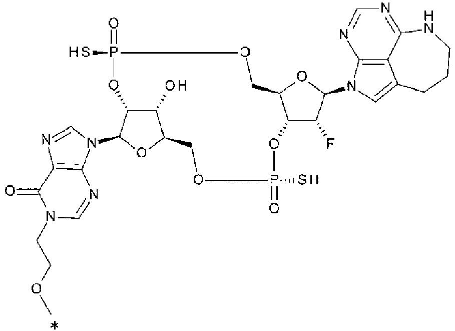
   wherein the asterisk represents binding to L.
[B3] The anti-HER2 antibody-drug conjugate according to [B1], wherein the anti-HER2 antibody-drug conjugate is represented by a formula below: 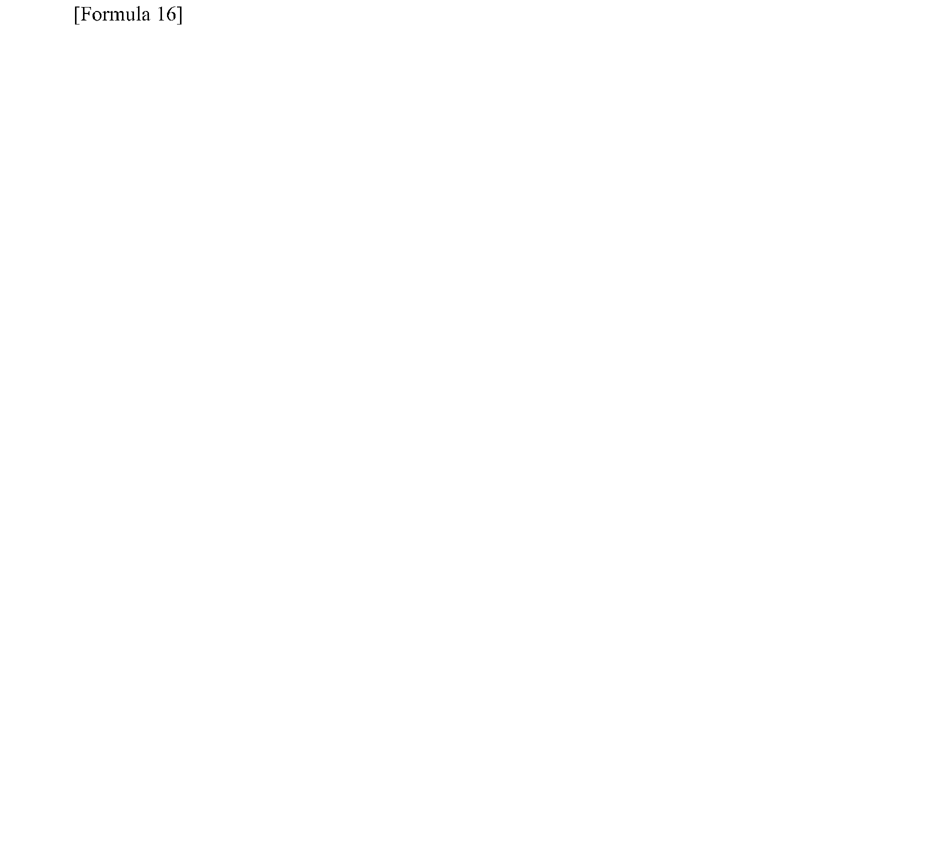 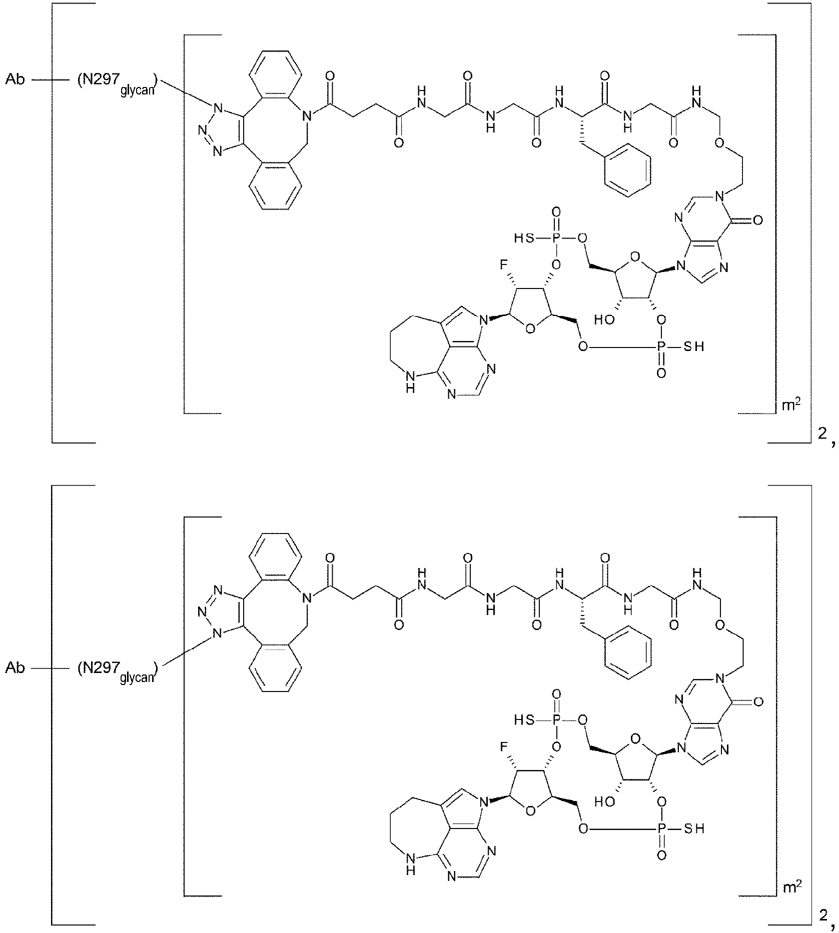 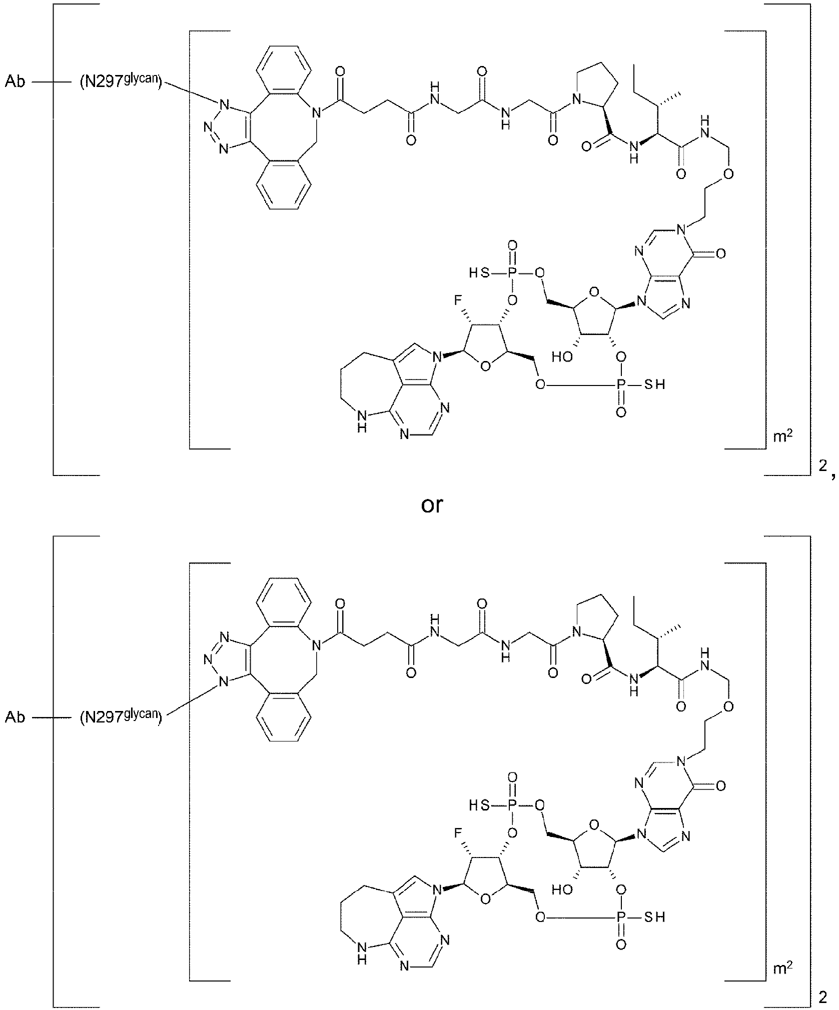 wherein Ab, the N297 glycan, and m² are as defined above.
[B4] The anti-HER2 antibody-drug conjugate according to any one of [B1] to [B3], wherein
   the anti-HER2 antibody-drug conjugate is represented by a formula below: 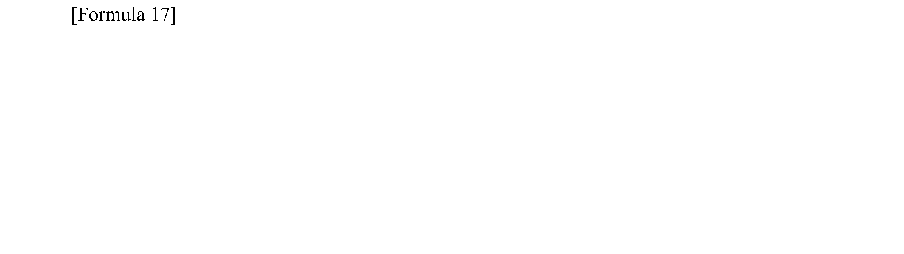 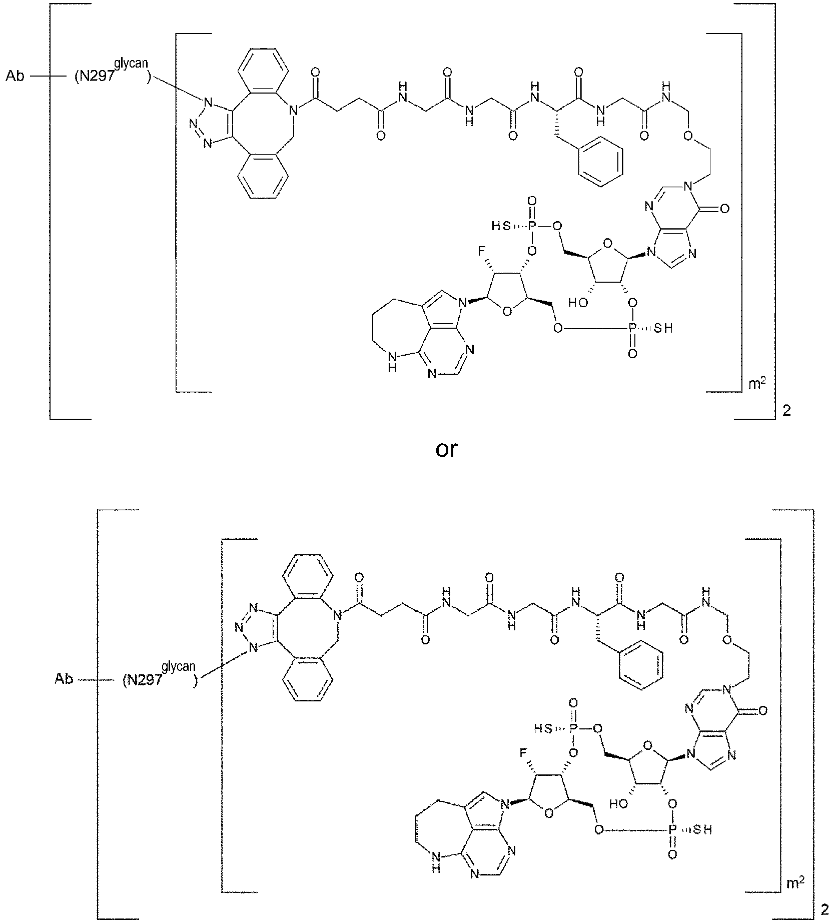
   wherein m² is 2;
   Ab comprises a heavy chain variable region consisting of an amino acid sequence having 80% or more, preferably 90% or more, and more preferably 95% or more identity with the amino acid sequence represented by the amino acid residues 1 to 119 of SEQ ID NO: 1, and a light chain variable region consisting of an amino acid sequence having 80% or more, preferably 90% or more, and more preferably 95% or more identity with the amino acid sequence represented by the amino acid residues 1 to 108 of SEQ ID NO: 2; and
   the N297 glycan is N297-(Fuc)SG having a structure represented by a formula below: 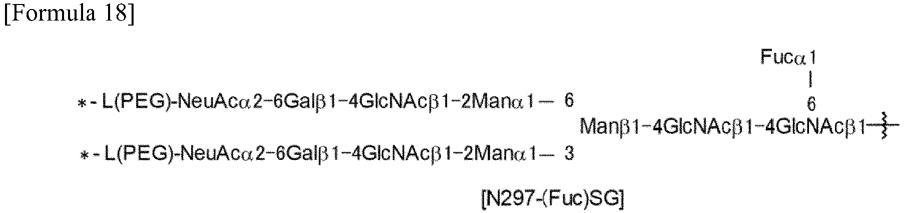
   wherein the wavy line represents binding to Asn297 of Ab,
   L(PEG) represents -(CH₂-CH₂-O)n⁵-CH₂-CH₂-NH-, and the amino group at the right end of the L(PEG) is amide-linked to the carboxyl group at the 2-position of the non-reducing-end sialic acid on each of the β-Man 1-3 and 1-6 branch chains of the N297 glycan,
   the asterisk represents binding to the nitrogen atom at the 1-position or 3-position on the 1,2,3-triazole ring, and
   n⁵ represents an integer of 3.
[B5] The anti-HER2 antibody-drug conjugate according to any one of [B1] to [B4], wherein Ab comprises a heavy chain variable region consisting of the amino acid sequence represented by the amino acid residues 1 to 119 of SEQ ID NO: 1, and a light chain variable region consisting of the amino acid sequence represented by the amino acid residues 1 to 108 of SEQ ID NO: 2.
[B6] The anti-HER2 antibody-drug conjugate according to any one of [B1] to [B5], wherein Ab comprises a heavy chain consisting of an amino acid sequence having 80% or more, preferably 90% or more, and more preferably 95% or more identity with the amino acid sequence represented by SEQ ID NO: 1, and a light chain consisting of an amino acid sequence having 80% or more, preferably 90% or more, and more preferably 95% or more identity with the amino acid sequence represented by SEQ ID NO: 2.
[B7] The anti-HER2 antibody-drug conjugate according to any one of [B1] to [B6], wherein Ab comprises a heavy chain consisting of the amino acid sequence represented by SEQ ID NO: 1, and a light chain consisting of the amino acid sequence represented by SEQ ID NO: 2.
[B8] The anti-HER2 antibody-drug conjugate according to any one of [B1] to [B5], wherein Ab comprises (i) a heavy chain consisting of an amino acid sequence in which one or two amino acids are deleted from the carboxyl terminus of the amino acid sequence of the heavy chain included in the antibody or the antigen-binding fragment of the antibody included in the anti-HER2 antibody-drug conjugate described in any one of [B1] to [B7]; and (ii) the light chain included in the antibody or the antigen-binding fragment of the antibody included in the anti-HER2 antibody-drug conjugate described in any one of [B1] to [B7].
[B9] The anti-HER2 antibody-drug conjugate according to any one of [B1] to [B8], for use in treating cancer in combination with an immune checkpoint inhibitor, wherein the immune checkpoint inhibitor is an antibody selected from the group consisting of an anti-PD-1 antibody, an anti-PD-L1 antibody, an anti-CTLA-4 antibody, an anti-TIGIT antibody, and an anti-LAG-3 antibody; an antigen-binding fragment of the antibody; or a compound including either the antibody or the antigen-binding fragment.
[B10] The anti-HER2 antibody-drug conjugate according to [B9], wherein the anti-PD-1 antibody is nivolumab, pembrolizumab, spartalizumab, cemiplimab, or dostarlimab.
[B11] The anti-HER2 antibody-drug conjugate according to [B9], wherein the anti-PD-L1 antibody is atezolizumab, durvalumab, or avelumab.
[B12] The anti-HER2 antibody-drug conjugate according to [B9], wherein the anti-CTLA-4 antibody is ipilimumab or tremelimumab.
[B13] The anti-HER2 antibody-drug conjugate according to [B9], wherein the anti-TIGIT antibody is tiragolumab or vibostolimab.
[B14] The anti-HER2 antibody-drug conjugate according to [B9], wherein the anti-LAG-3 antibody is relatlimab.
[B15] The anti-HER2 antibody-drug conjugate according to any one of [B1] to [B8], for use in treating cancer in combination with a chemotherapeutic agent, wherein the chemotherapeutic agent is an antimetabolite, a tubulin inhibitor, an alkylating agent, a platinum-based agent, or a topoisomerase inhibitor.
[B16] The anti-HER2 antibody-drug conjugate according to [B15], wherein the antimetabolite is capecitabine or a pharmaceutically acceptable salt of capecitabine.
[B17] The anti-HER2 antibody-drug conjugate according to [B15], wherein the tubulin inhibitor is paclitaxel or a pharmaceutically acceptable salt of paclitaxel.
[B18] The anti-HER2 antibody-drug conjugate according to [B15], wherein the topoisomerase inhibitor is irinotecan or a pharmaceutically acceptable salt of irinotecan.
[B19] The anti-HER2 antibody-drug conjugate according to any one of [B1] to [B8], for use in treating cancer in combination with an anti-HER2 antibody, wherein the anti-HER2 antibody used in combination with the anti-HER2 antibody-drug conjugate is trastuzumab, pertuzumab, or margetuximab.
[B20] The anti-HER2 antibody-drug conjugate according to any one of [B1] to [B8], for use in treating cancer in combination with an anti-HER2 antibody-containing compound, wherein the anti-HER2 antibody-containing compound is ado-trastuzumab emtansine (T-DM1), disitamab vedotin, or vic-trastuzumab duocarmazine.
[B21] The anti-HER2 antibody-drug conjugate according to any one of [B1] to [B20], wherein
   the anti-HER2 antibody-drug conjugate; and
   the immune checkpoint inhibitor, the chemotherapeutic agent, the molecular-targeted drug, the anti-HER2 antibody, or the anti-HER2 antibody-containing compound
   are each included as an active ingredient in different formulations and are administered simultaneously or at different times (sequentially or separately).
[B22] The anti-HER2 antibody-drug conjugate according to any one of [B1] to [B21], wherein the cancer is selected from the group consisting of lung cancer, colorectal cancer, prostate cancer, pancreatic cancer, ovarian cancer, breast cancer, bladder cancer, gastric cancer, esophageal cancer, endometrial cancer, cervical cancer, head and neck cancer, bile duct cancer, myeloma, and sarcoma.
[B23] The anti-HER2 antibody-drug conjugate according to [B22], wherein the cancer is lung cancer.
[B24] The anti-HER2 antibody-drug conjugate according to [B22], wherein the cancer is colorectal cancer.
[B25] The anti-HER2 antibody-drug conjugate according to [B22], wherein the cancer is breast cancer.
[B26] The anti-HER2 antibody-drug conjugate according to [B22], wherein the cancer is gastric cancer.
[B27] The anti-HER2 antibody-drug conjugate according to [B22], wherein the cancer is bladder cancer.
[C1] Use of an anti-HER2 antibody-drug conjugate for the manufacture of a medicament for treating cancer in combination with one or more selected from an immune checkpoint inhibitor, a chemotherapeutic agent, a molecular-targeted drug, an anti-HER2 antibody, and an anti-HER2 antibody-containing compound, provided that the anti-HER2 antibody-drug conjugate represented by Formula (I) described below and anti-HER2 antibody-deruxtecan are excluded, wherein
   the anti-HER2 antibody-drug conjugate is represented by Formula (I) below: 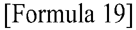 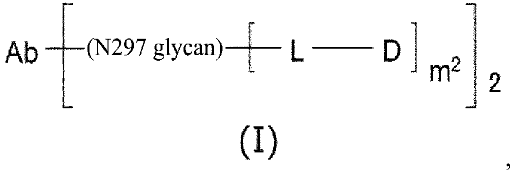
   wherein m² represents an integer of 1 or 2;
   L represents a linker connecting the N297 glycan and a drug D, and represents -Lb-La-Lp-Lc-*, wherein
   the asterisk represents binding to the drug D;
   Lb represents: 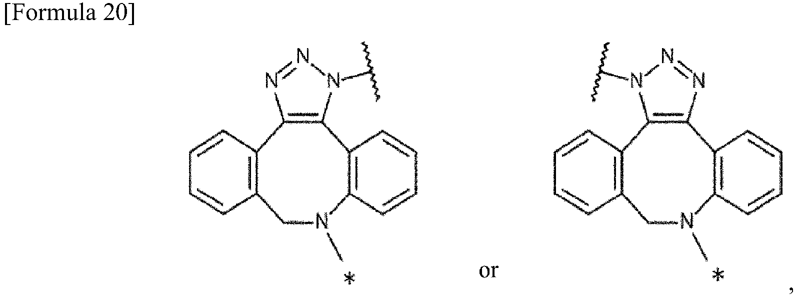
   wherein in the structural formula of Lb shown above, the asterisk represents binding to La, and the wavy line represents binding to the N297 glycan;
   La represents -C(=O)-CH₂CH₂-C(=O)-;
   Lp represents -GGFG- or -GGPI-; and
   Lc represents -NH-CH₂-;
   Ab is an anti-HER2 antibody comprising a heavy chain comprising CDRH1 consisting of the amino acid sequence represented by SEQ ID NO: 3, CDRH2 consisting of the amino acid sequence represented by SEQ ID NO: 4, and CDRH3 consisting of the amino acid sequence represented by SEQ ID NO: 5, and a light chain comprising CDRL1 consisting of the amino acid sequence represented by SEQ ID NO: 6, CDRL2 consisting of the amino acid sequence represented by SAS, and CDRL3 consisting of the amino acid sequence represented by SEQ ID NO: 7, or an antigen-binding fragment of the antibody;
   the N297 glycan is N297-(Fuc)MSG1 having a structure represented by a formula below: 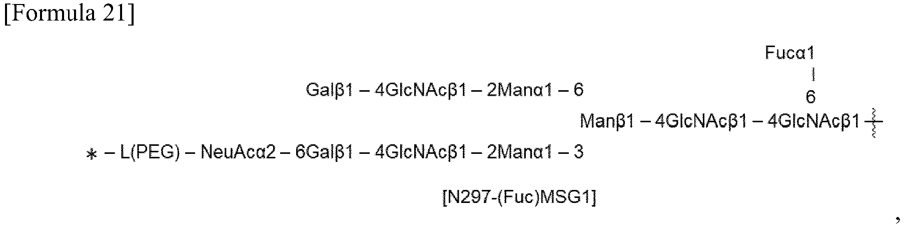
   wherein the wavy line represents binding to Asn297 of Ab;
   L(PEG) represents -(CH₂-CH₂-O)n⁵-CH₂-CH₂-NH-, and the amino group at the right end of the L(PEG) is amide-linked to the carboxyl group at the 2-position of the non-reducing-end sialic acid on the β-Man 1-3 branch chain of the N297 glycan;
   the asterisk represents binding to the nitrogen atom at the 1-position or 3-position on the 1,2,3-triazole ring of Lb in the linker L; and
   n⁵ represents an integer of 2 to 5; or
   N297-(Fuc)SG having a structure represented by the following formula: 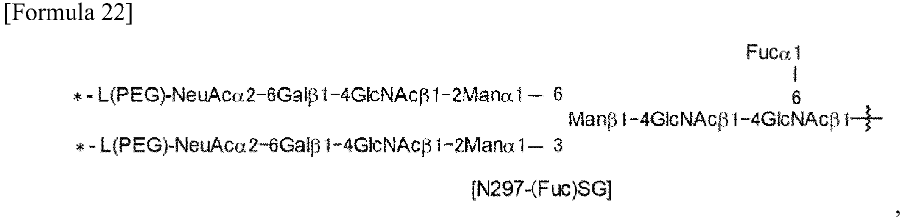
   wherein the wavy line represents binding to Asn297 of Ab;
   L(PEG) represents -(CH₂-CH₂-O)n⁵-CH₂-CH₂-NH-, and the amino group at the right end of the L(PEG) is amide-linked to the carboxyl group at the 2-position of the non-reducing-end sialic acid on each of the β-Man 1-3 and 1-6 branch chains of the N297 glycan;
   the asterisk represents binding to the nitrogen atom at the 1-position or 3-position on the 1,2,3-triazole ring of Lb in the linker L; and
   n⁵ represents an integer of 2 to 5; and
   D is 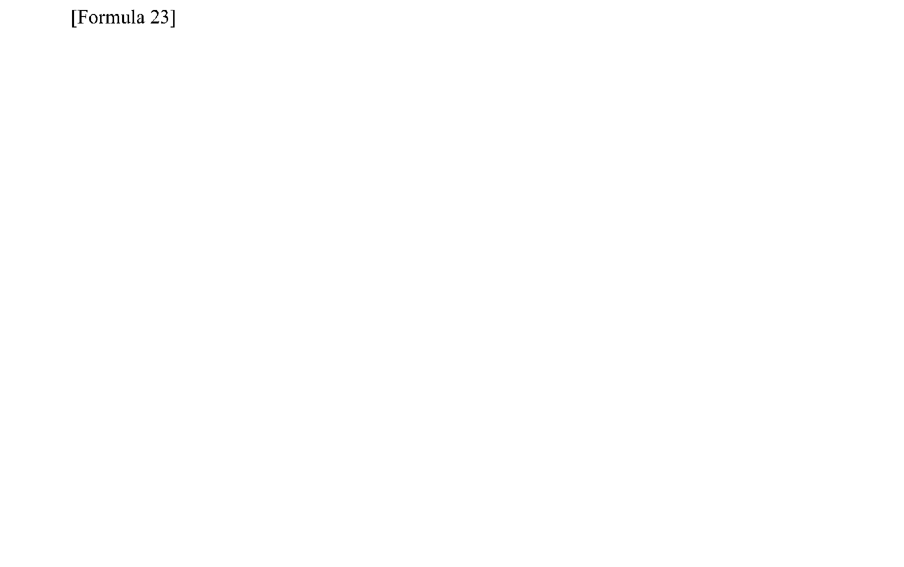 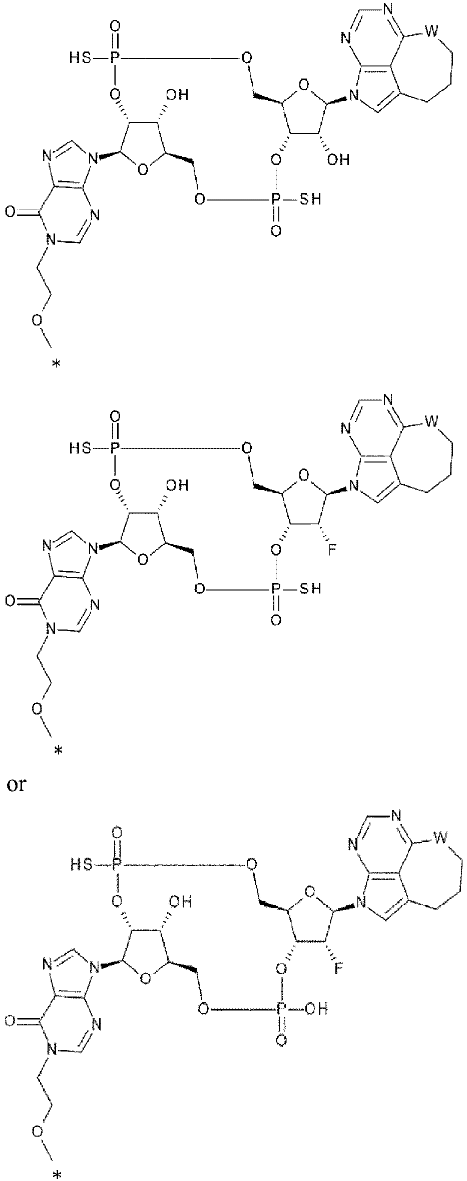
   wherein the asterisk represents binding to L, and W represents -NH-.
[C1'] The use according to [C1], wherein Ab is an anti-HER2 antibody comprising an Fc region (wild-type or variant) of an antibody, or an antigen-binding fragment of the antibody .
[C2] The use according to [C1], wherein
   D is represented by 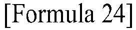 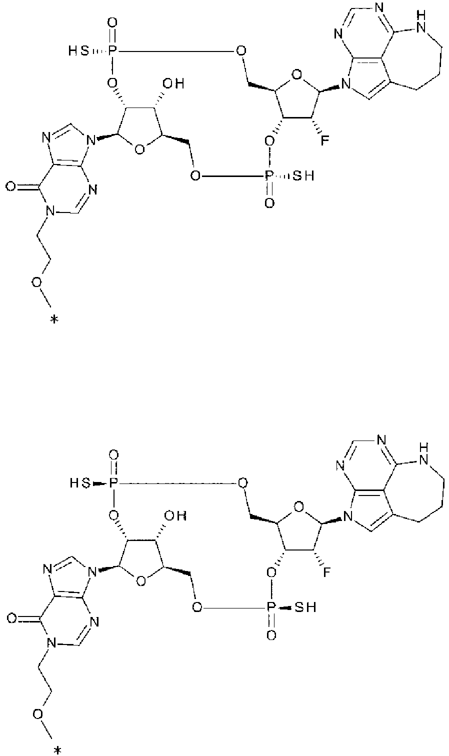 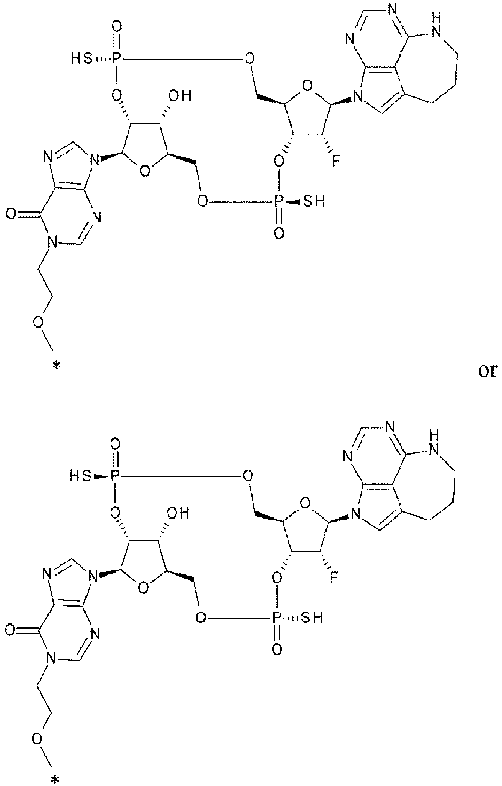
   wherein the asterisk represents binding to L.
[C3] The use according to [C1], wherein the anti-HER2 antibody-drug conjugate is represented by a formula below: 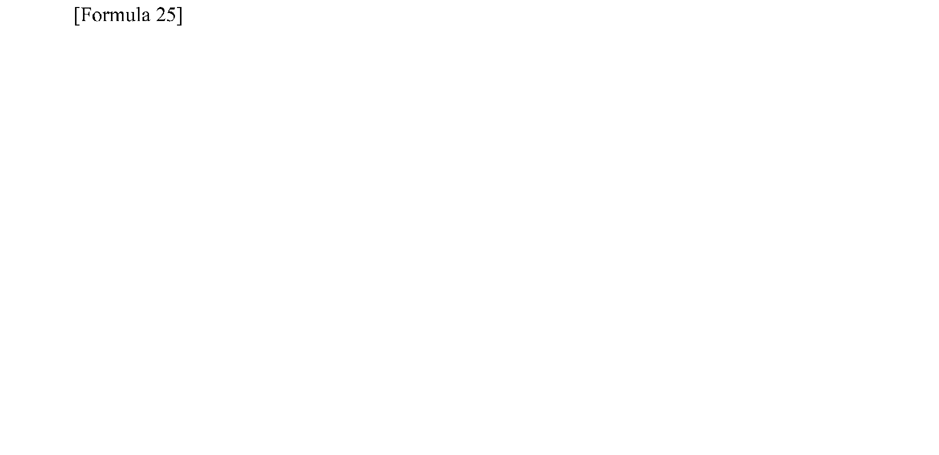 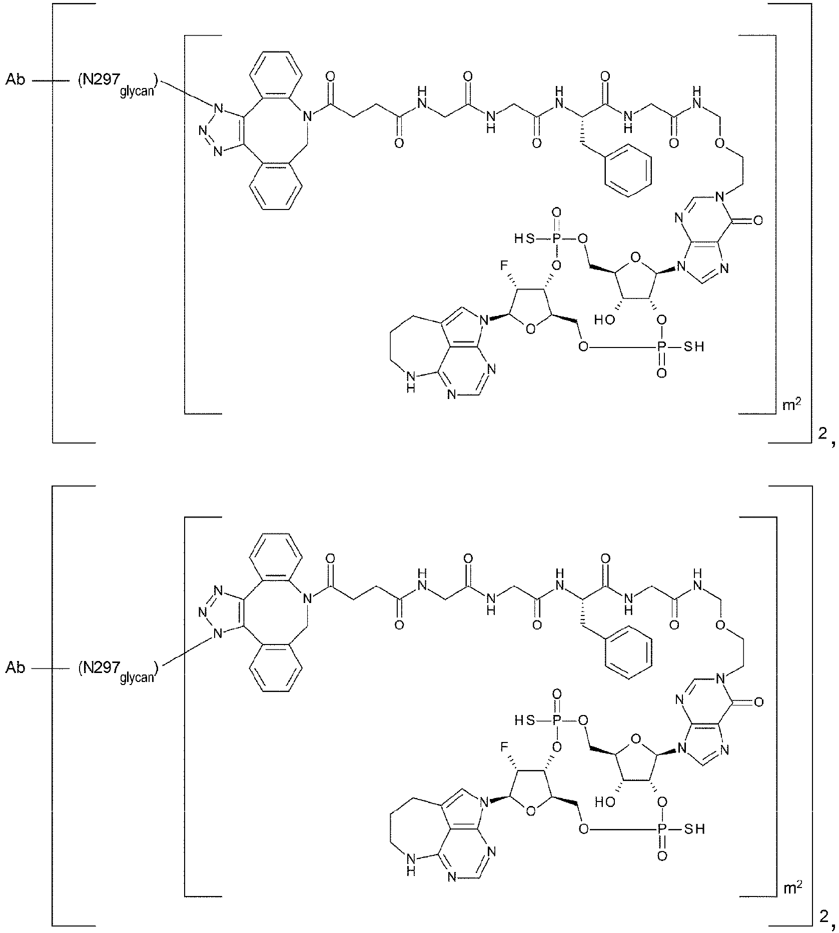 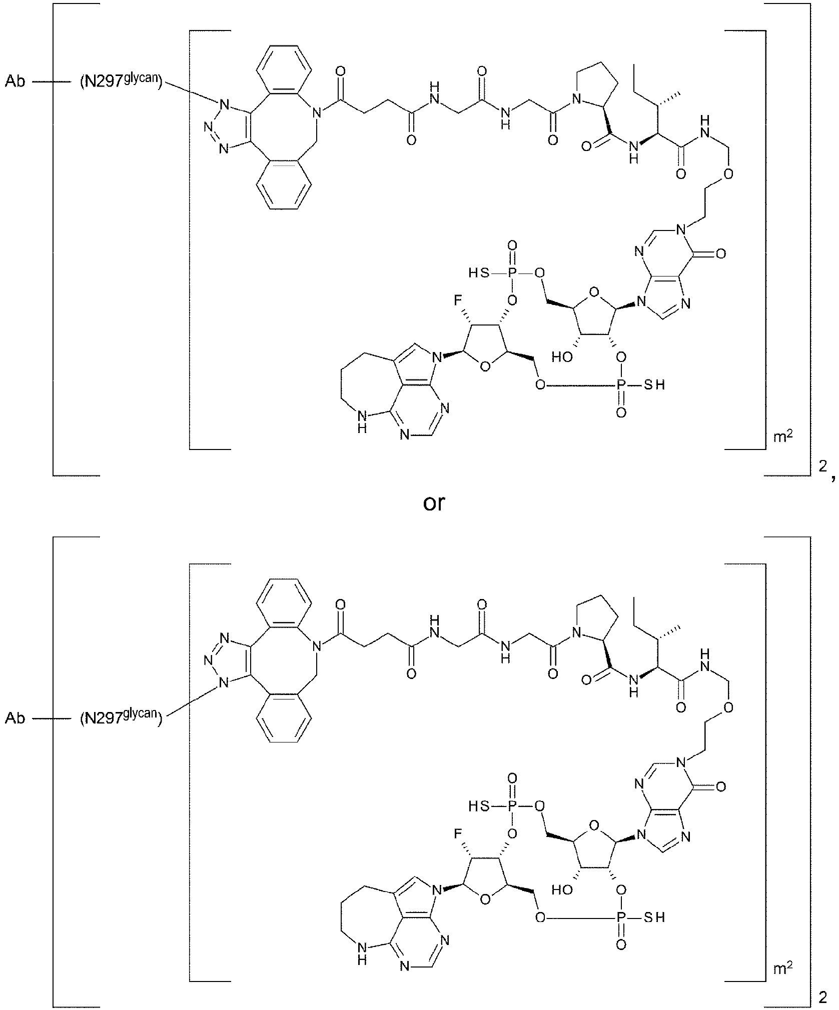 wherein Ab, the N297 glycan, and m² are as defined above.
[C4] The use according to any one of [C1] to [C3], wherein
   the anti-HER2 antibody-drug conjugate is represented by a formula below: 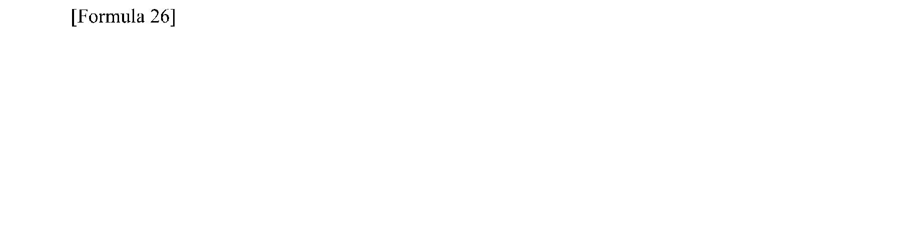 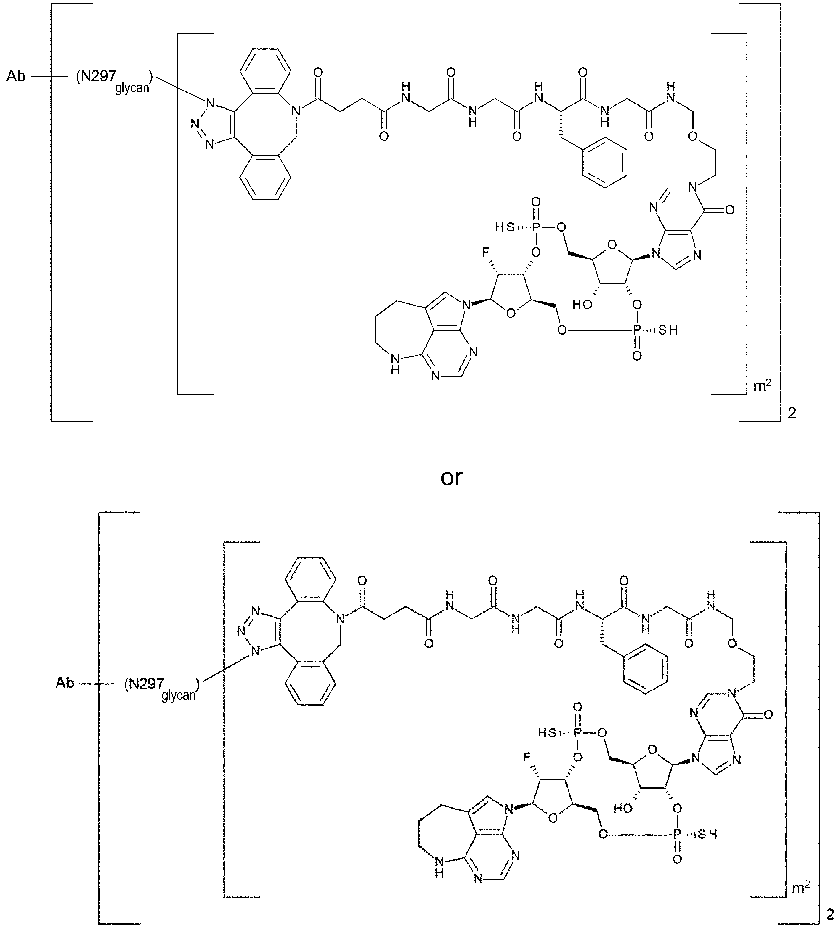
   wherein m² is 2;
   Ab comprises a heavy chain variable region consisting of an amino acid sequence having 80% or more, preferably 90% or more, and more preferably 95% or more identity with the amino acid sequence represented by the amino acid residues 1 to 119 of SEQ ID NO: 1, and a light chain variable region consisting of an amino acid sequence having 80% or more, preferably 90% or more, and more preferably 95% or more identity with the amino acid sequence represented by the amino acid residues 1 to 108 of SEQ ID NO: 2; and
   the N297 glycan is N297-(Fuc)SG having a structure represented by a formula below: 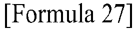 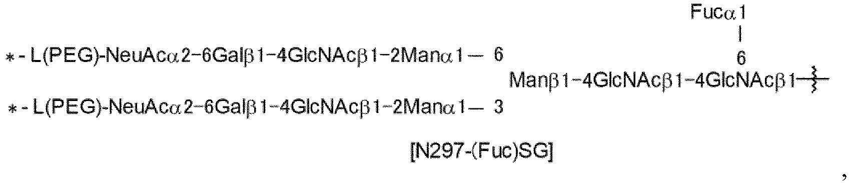
   wherein the wavy line represents binding to Asn297 of Ab,
   L(PEG) represents -(CH₂-CH₂-O)n⁵-CH₂-CH₂-NH-, and the amino group at the right end of the L(PEG) is amide-linked to the carboxyl group at the 2-position of the non-reducing-end sialic acid on each of the β-Man 1-3 and 1-6 branch chains of the N297 glycan,
   the asterisk represents binding to the nitrogen atom at the 1-position or 3-position on the 1,2,3-triazole ring, and
   n⁵ represents an integer of 3.
[C5] The use according to any one of [C1] to [C4], wherein Ab comprises a heavy chain variable region consisting of the amino acid sequence represented by the amino acid residues 1 to 119 of SEQ ID NO: 1, and a light chain variable region consisting of the amino acid sequence represented by the amino acid residues 1 to 108 of SEQ ID NO: 2.
[C6] The use according to any one of [C1] to [C5], wherein Ab comprises a heavy chain consisting of an amino acid sequence having 80% or more, preferably 90% or more, and more preferably 95% or more identity with the amino acid sequence represented by SEQ ID NO: 1, and a light chain consisting of an amino acid sequence having 80% or more, preferably 90% or more, and more preferably 95% or more identity with the amino acid sequence represented by SEQ ID NO: 2.
[C7] The use according to any one of [C1] to [C6], wherein Ab comprises a heavy chain consisting of the amino acid sequence represented by SEQ ID NO: 1, and a light chain consisting of the amino acid sequence represented by SEQ ID NO: 2.
[C8] The use according to any one of [C1] to [C5], wherein Ab comprises (i) a heavy chain consisting of an amino acid sequence in which one or two amino acids are deleted from the carboxyl terminus of the amino acid sequence of the heavy chain included in the antibody or the antigen-binding fragment of the antibody included in the anti-HER2 antibody-drug conjugate described in any one of [C1] to [C7]; and (ii) the light chain included in the antibody or the antigen-binding fragment of the antibody included in the anti-HER2 antibody-drug conjugate described in any one of [C1] to [C7].
[C9] The use according to any one of [C1] to [C8], of the anti-HER2 antibody-drug conjugate in combination with an immune checkpoint inhibitor for the manufacture of a medicament for treating cancer, wherein the immune checkpoint inhibitor is an antibody selected from the group consisting of an anti-PD-1 antibody, an anti-PD-L1 antibody, an anti-CTLA-4 antibody, an anti-TIGIT antibody, and an anti-LAG-3 antibody; an antigen-binding fragment of the antibody; or a compound including either the antibody or the antigen-binding fragment.
[C10] The use according to [C9], wherein the anti-PD-1 antibody is nivolumab, pembrolizumab, spartalizumab, cemiplimab, or dostarlimab.
[C11] The use according to [C9], wherein the anti-PD-L1 antibody is atezolizumab, durvalumab, or avelumab.
[C12] The use according to [C9], wherein the anti-CTLA-4 antibody is ipilimumab or tremelimumab.
[C13] The use according to [C9], wherein the anti-TIGIT antibody is tiragolumab or vibostolimab.
[C14] The use according to [C9], wherein the anti-LAG-3 antibody is relatlimab.
[C15] The use according to any one of [C1] to [C8], of the anti-HER2 antibody-drug conjugate in combination with a chemotherapeutic agent for the manufacture of a medicament for treating cancer, wherein the chemotherapeutic agent is an antimetabolite, a tubulin inhibitor, an alkylating agent, a platinum-based agent, or a topoisomerase inhibitor.
[C16] The use according to [C15], wherein the antimetabolite is capecitabine or a pharmaceutically acceptable salt of capecitabine.
[C17] The use according to [C15], wherein the tubulin inhibitor is paclitaxel or a pharmaceutically acceptable salt of paclitaxel.
[C18] The use according to [C15], wherein the topoisomerase inhibitor is irinotecan or a pharmaceutically acceptable salt of irinotecan.
[C19] The use according to any one of [C1] to [C8], of the anti-HER2 antibody-drug conjugate in combination with an anti-HER2 antibody for the manufacture of a medicament for treating cancer, wherein the anti-HER2 antibody used in combination with the anti-HER2 antibody-drug conjugate is trastuzumab, pertuzumab, or margetuximab.
[C20] The use according to any one of [C1] to [C8], of the anti-HER2 antibody-drug conjugate in combination with an anti-HER2 antibody-containing compound for the manufacture of a medicament for treating cancer, wherein the anti-HER2 antibody-containing compound is ado-trastuzumab emtansine (T-DM1), disitamab vedotin, or vic-trastuzumab duocarmazine.
[C21] The use according to any one of [C1] to [C20], wherein
   the anti-HER2 antibody-drug conjugate; and
   the immune checkpoint inhibitor, the chemotherapeutic agent, the molecular-targeted drug, the anti-HER2 antibody, or the anti-HER2 antibody-containing compound
   are each included as an active ingredient in different formulations and are administered simultaneously or at different times (sequentially or separately).
[C22] The use according to any one of [C1] to [C21], wherein the cancer is selected from the group consisting of lung cancer, colorectal cancer, prostate cancer, pancreatic cancer, ovarian cancer, breast cancer, bladder cancer, gastric cancer, esophageal cancer, endometrial cancer, cervical cancer, head and neck cancer, bile duct cancer, myeloma, and sarcoma.
[C23] The use according to [C22], wherein the cancer is lung cancer.
[C24] The use according to [C22], wherein the cancer is colorectal cancer.
[C25] The use according to [C22], wherein the cancer is breast cancer.
[C26] The use according to [C22], wherein the cancer is gastric cancer.
[C27] The use according to [C22], wherein the cancer is bladder cancer.
[D1] A method of treating a disease, the method comprising administering in combination to a subject in need of treatment of the disease:
   a) an anti-HER2 antibody-drug conjugate; and
   b) one or more selected from an immune checkpoint inhibitor, a chemotherapeutic agent, a molecular-targeted drug, an anti-HER2 antibody, and an anti-HER2 antibody-containing compound, provided that the anti-HER2 antibody-drug conjugate described in a) and anti-HER2 antibody-deruxtecan are excluded, wherein
      the anti-HER2 antibody-drug conjugate is represented by Formula (I) below: 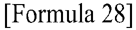 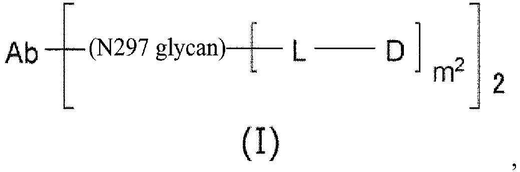
      wherein m² represents an integer of 1 or 2;
      L represents a linker connecting the N297 glycan and a drug D, and represents -Lb-La-Lp-Lc-*, wherein
      the asterisk represents binding to the drug D;
      Lb represents: 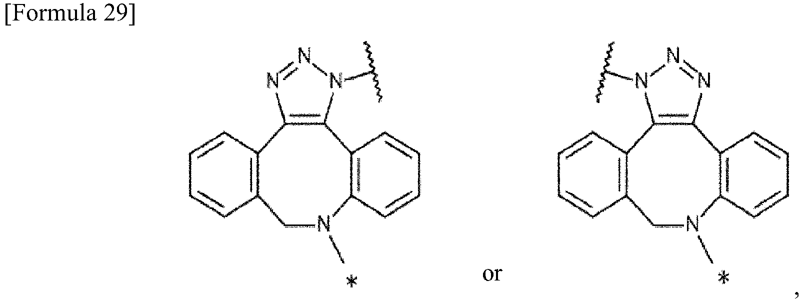
      wherein in the structural formula of Lb shown above, the asterisk represents binding to La, and the wavy line represents binding to the N297 glycan;
      La represents -C(=O)-CH₂CH₂-C(=O)-;
      Lp represents -GGFG- or -GGPI-; and
      Lc represents -NH-CH₂-;
      Ab is an anti-HER2 antibody comprising a heavy chain comprising CDRH1 consisting of the amino acid sequence represented by SEQ ID NO: 3, CDRH2 consisting of the amino acid sequence represented by SEQ ID NO: 4, and CDRH3 consisting of the amino acid sequence represented by SEQ ID NO: 5, and a light chain comprising CDRL1 consisting of the amino acid sequence represented by SEQ ID NO: 6, CDRL2 consisting of the amino acid sequence represented by SAS, and CDRL3 consisting of the amino acid sequence represented by SEQ ID NO: 7, or an antigen-binding fragment of the antibody;
      the N297 glycan is N297-(Fuc)MSG1 having a structure represented by a formula below: 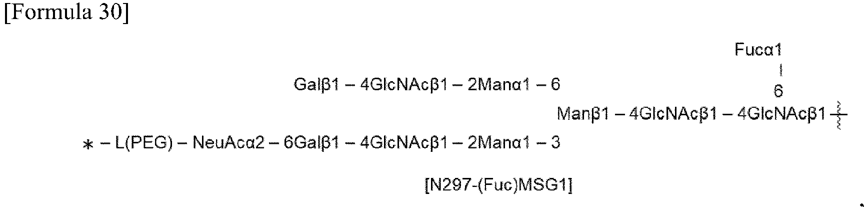
      wherein the wavy line represents binding to Asn297 of Ab;
      L(PEG) represents -(CH₂-CH₂-O)n⁵-CH₂-CH₂-NH-, and the amino group at the right end of the L(PEG) is amide-linked to the carboxyl group at the 2-position of the non-reducing-end sialic acid on the β-Man 1-3 branch chain of the N297 glycan;
      the asterisk represents binding to the nitrogen atom at the 1-position or 3-position on the 1,2,3-triazole ring of Lb in the linker L; and
      n⁵ represents an integer of 2 to 5; or
      N297-(Fuc)SG having a structure represented by the following formula: 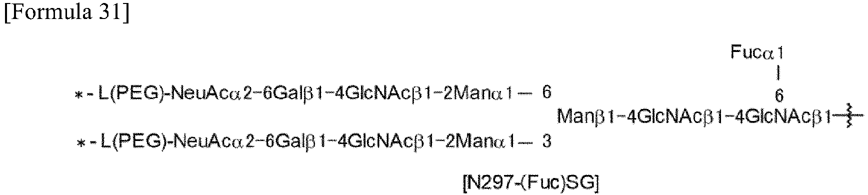
      wherein the wavy line represents binding to Asn297 of Ab;
      L(PEG) represents -(CH₂-CH₂-O)n⁵-CH₂-CH₂-NH-, and the amino group at the right end of the L(PEG) is amide-linked to the carboxyl group at the 2-position of the non-reducing-end sialic acid on each of the β-Man 1-3 and 1-6 branch chains of the N297 glycan;
      the asterisk represents binding to the nitrogen atom at the 1-position or 3-position on the 1,2,3-triazole ring of Lb in the linker L; and
      n⁵ represents an integer of 2 to 5; and
      D is 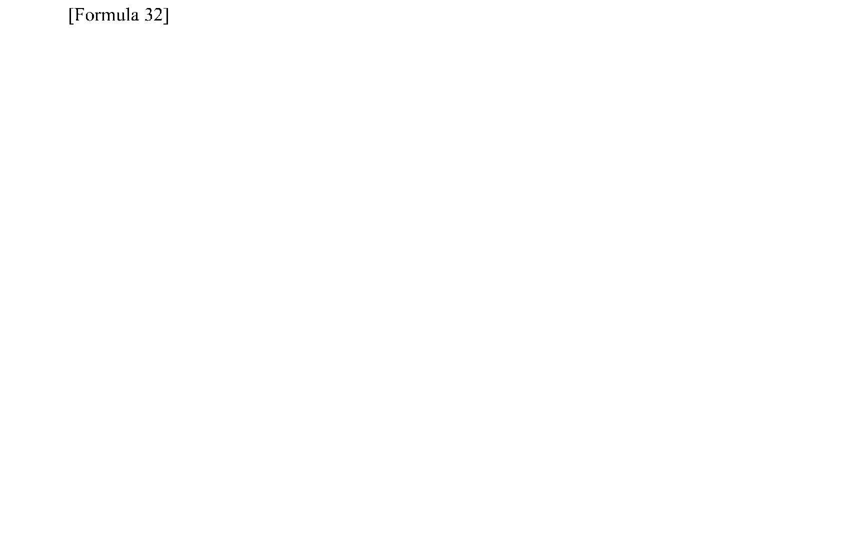 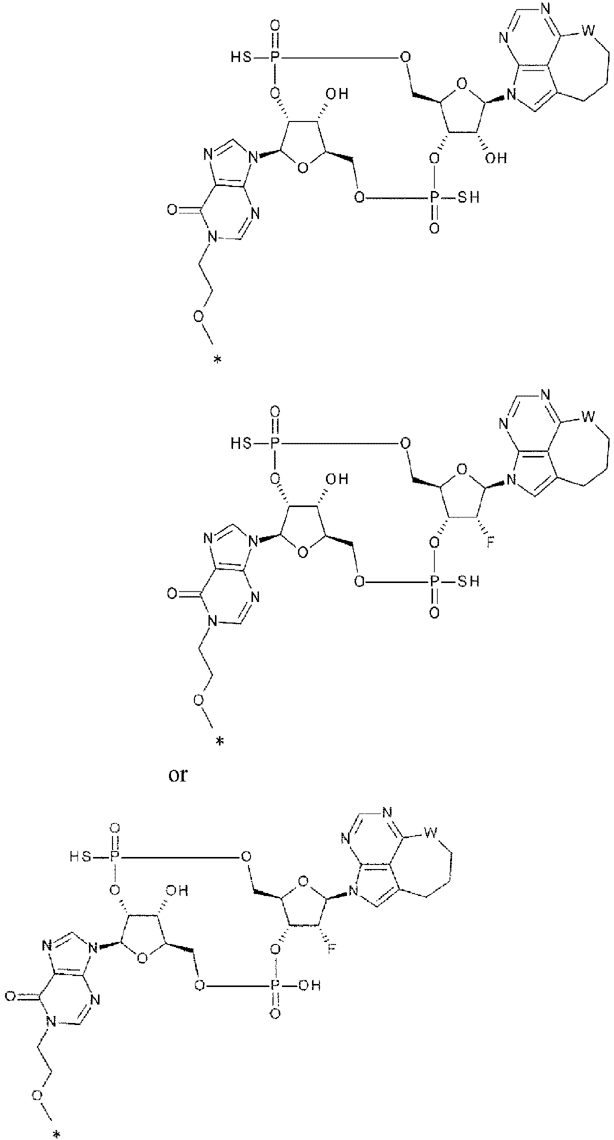
      wherein the asterisk represents binding to L, and W represents -NH-.
[D1'] The method according to [D1], wherein Ab is an anti-HER2 antibody comprising an Fc region (wild-type or variant) of an antibody, or an antigen-binding fragment of the antibody .
[D2] The method according to [D1], wherein
   D is represented by 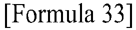 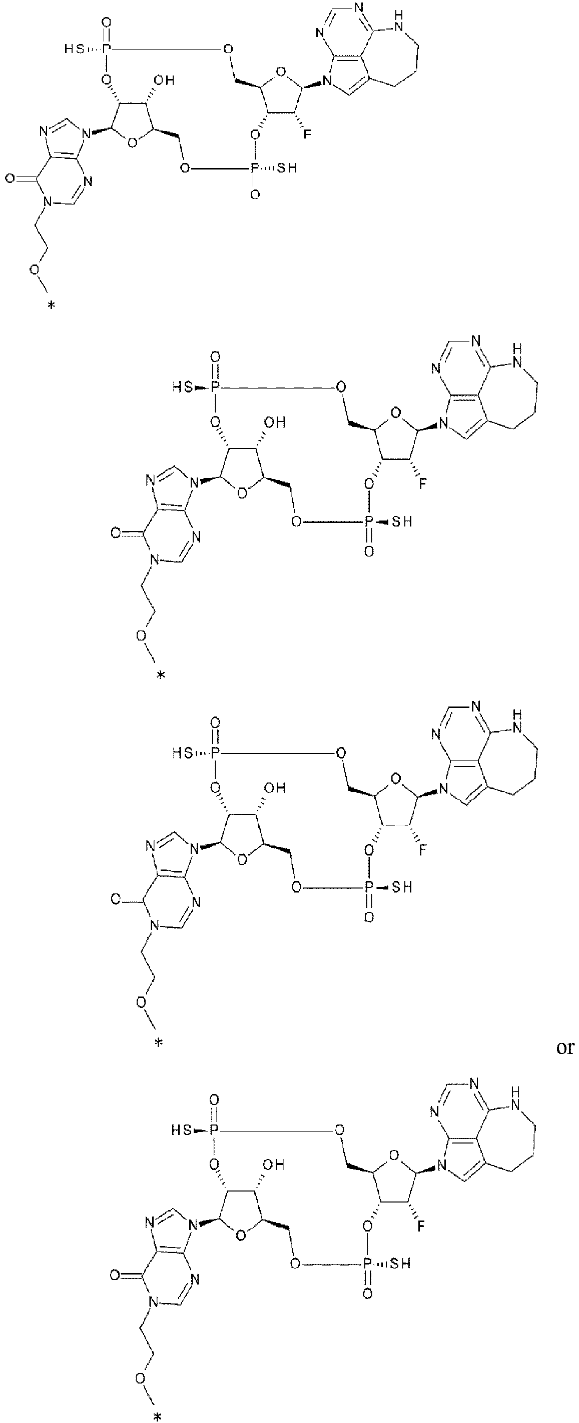
   wherein the asterisk represents binding to L.
[D3] The method according to [D1], wherein the anti-HER2 antibody-drug conjugate is represented by a formula below: 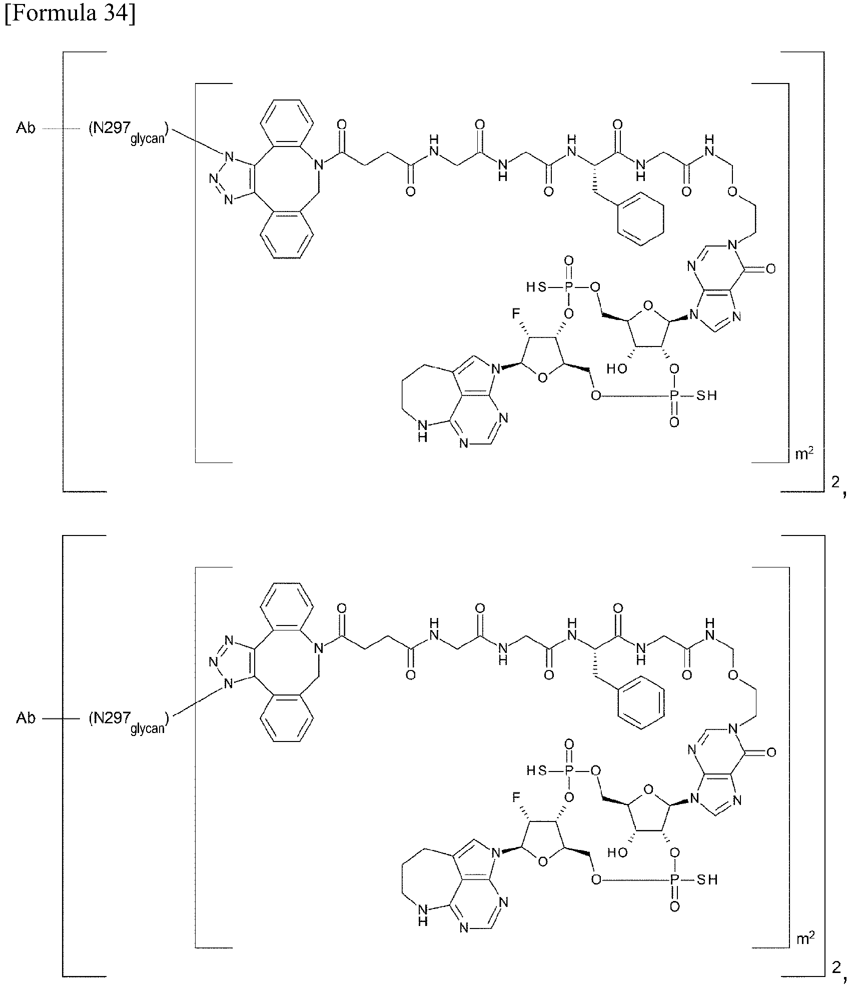 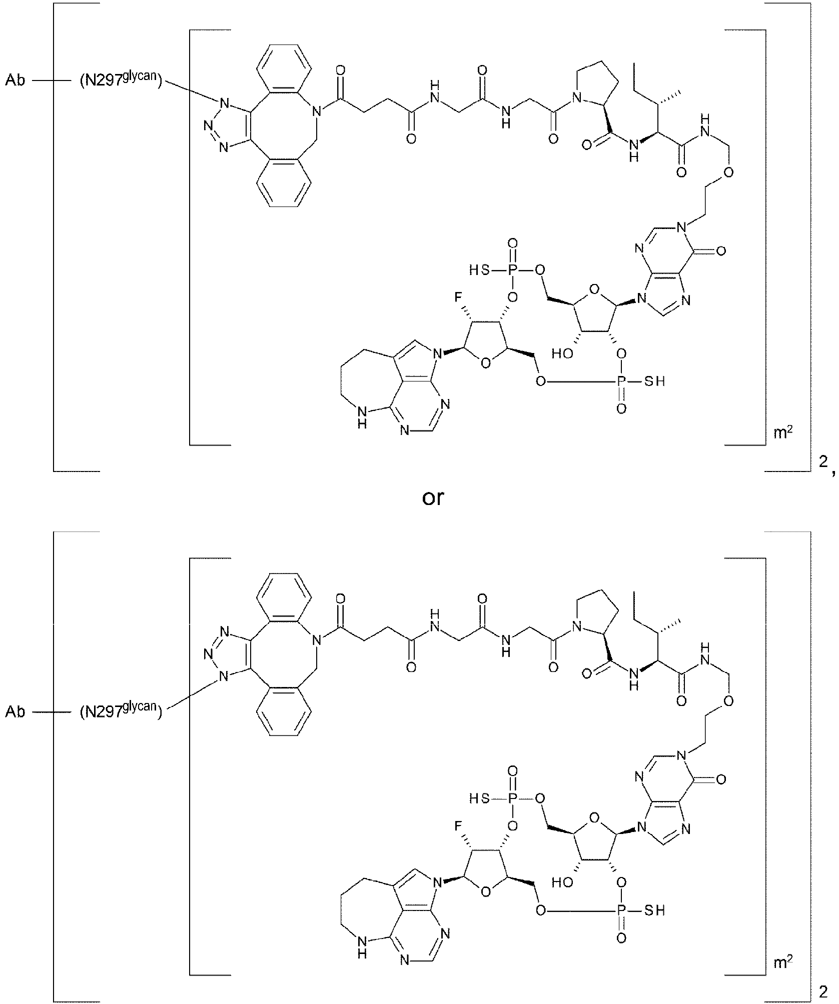 wherein Ab, the N297 glycan, and m² are as defined above.
[D4] The method according to any one of [D1] to [D3], wherein
   the anti-HER2 antibody-drug conjugate is represented by a formula below: 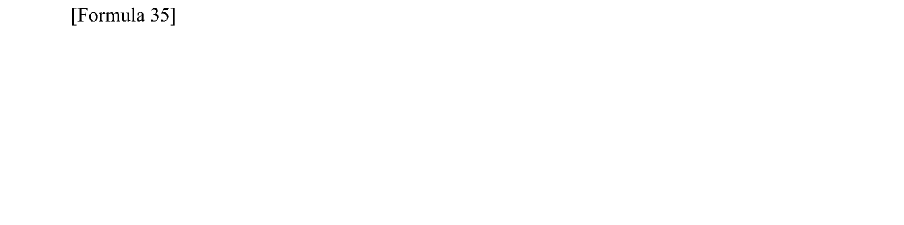 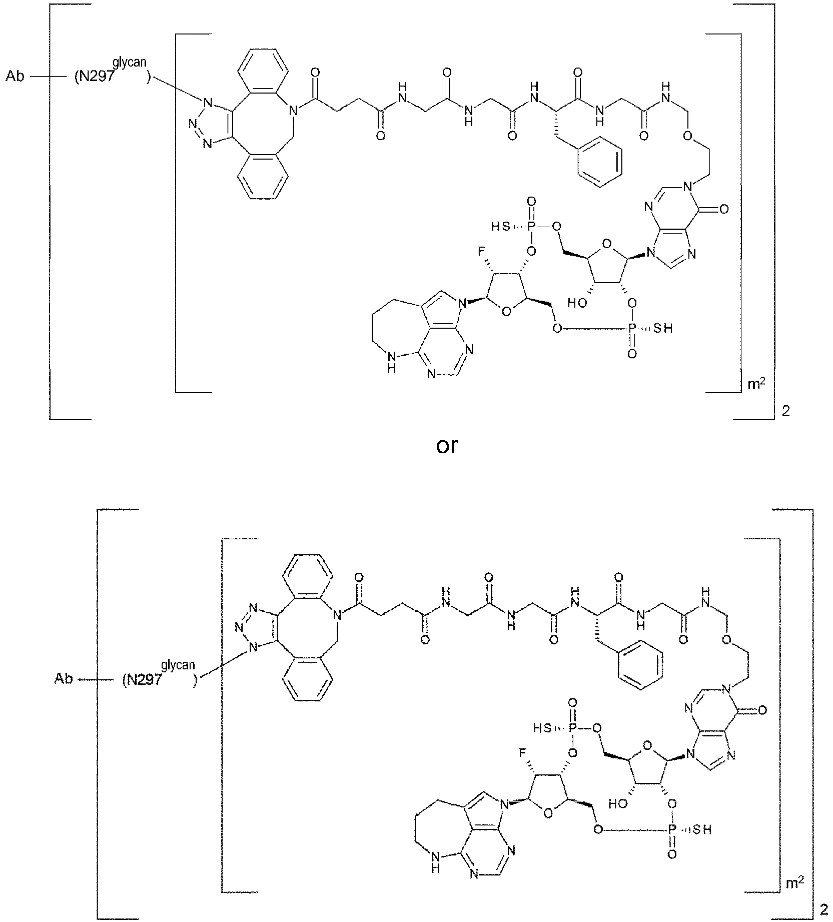
   wherein m² is 2;
   Ab comprises a heavy chain variable region consisting of an amino acid sequence having 80% or more, preferably 90% or more, and more preferably 95% or more identity with the amino acid sequence represented by the amino acid residues 1 to 119 of SEQ ID NO: 1, and a light chain variable region consisting of an amino acid sequence having 80% or more, preferably 90% or more, and more preferably 95% or more identity with the amino acid sequence represented by the amino acid residues 1 to 108 of SEQ ID NO: 2; and
   the N297 glycan is N297-(Fuc)SG having a structure represented by a formula below: 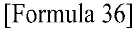 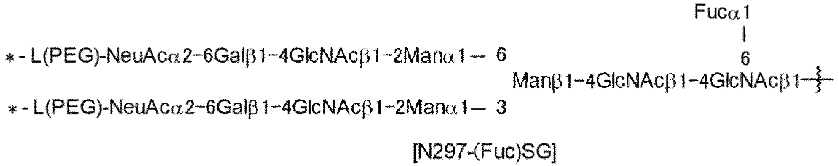
   wherein the wavy line represents binding to Asn297 of Ab,
   L(PEG) represents -(CH₂-CH₂-O)n⁵-CH₂-CH₂-NH-, and the amino group at the right end of the L(PEG) is amide-linked to the carboxyl group at the 2-position of the non-reducing-end sialic acid on each of the β-Man 1-3 and 1-6 branch chains of the N297 glycan,
   the asterisk represents binding to the nitrogen atom at the 1-position or 3-position on the 1,2,3-triazole ring, and
   n⁵ represents an integer of 3.
[D5] The method according to any one of [D1] to [D4], wherein Ab comprises a heavy chain variable region consisting of the amino acid sequence represented by the amino acid residues1 to 119 of SEQ ID NO: 1, and a light chain variable region consisting of the amino acid sequence represented by the amino acid residues 1 to 108 of SEQ ID NO: 2.
[D6] The method according to any one of [D1] to [D5], wherein Ab comprises a heavy chain consisting of an amino acid sequence having 80% or more, preferably 90% or more, and more preferably 95% or more identity with the amino acid sequence represented by SEQ ID NO: 1, and a light chain consisting of an amino acid sequence having 80% or more, preferably 90% or more, and more preferably 95% or more identity with the amino acid sequence represented by SEQ ID NO: 2.
[D7] The method according to any one of [D1] to [D6], wherein Ab comprises a heavy chain consisting of the amino acid sequence represented by SEQ ID NO: 1, and a light chain consisting of the amino acid sequence represented by SEQ ID NO: 2.
[D8] The method according to any one of [D1] to [D5], wherein Ab comprises (i) a heavy chain consisting of an amino acid sequence in which one or two amino acids are deleted from the carboxyl terminus of the amino acid sequence of the heavy chain included in the antibody or the antigen-binding fragment of the antibody included in the anti-HER2 antibody-drug conjugate described in any one of [D1] to [D7]; and (ii) the light chain included in the antibody or the antigen-binding fragment of the antibody included in the anti-HER2 antibody-drug conjugate described in any one of [D1] to [D7].
[D9] The method of treating a disease according to any one of [D1] to [D8], comprising administering in combination to a subject in need of treatment of the disease: a) the anti-HER2 antibody-drug conjugate; and b) an immune checkpoint inhibitor, wherein the immune checkpoint inhibitor is an antibody selected from the group consisting of an anti-PD-1 antibody, an anti-PD-L1 antibody, an anti-CTLA-4 antibody, an anti-TIGIT antibody, and an anti-LAG-3 antibody; an antigen-binding fragment of the antibody; or a compound including either the antibody or the antigen-binding fragment.
[D10] The method according to [D9], wherein the anti-PD-1 antibody is nivolumab, pembrolizumab, spartalizumab, cemiplimab, or dostarlimab.
[D11] The method according to [D9], wherein the anti-PD-L1 antibody is atezolizumab, durvalumab, or avelumab.
[D12] The method according to [D9], wherein the anti-CTLA-4 antibody is ipilimumab or tremelimumab.
[D13] The method according to [D9], wherein the anti-TIGIT antibody is tiragolumab or vibostolimab.
[D14] The method according to [D9], wherein the anti-LAG-3 antibody is relatlimab.
[D15] The method of treating a disease according to any one of [D1] to [D8] comprising administering in combination to a subject in need of treatment of the disease: a) the anti-HER2 antibody-drug conjugate; and b) a chemotherapeutic agent, wherein the chemotherapeutic agent is an antimetabolite, a tubulin inhibitor, an alkylating agent, a platinum-based agent, or a topoisomerase inhibitor.
[D16] The method according to [D15], wherein the antimetabolite is capecitabine or a pharmaceutically acceptable salt of capecitabine.
[D17] The method according to [D15], wherein the tubulin inhibitor is paclitaxel or a pharmaceutically acceptable salt of paclitaxel.
[D18] The method according to [D15], wherein the topoisomerase inhibitor is irinotecan or a pharmaceutically acceptable salt of irinotecan.
[D19] The method of treating a disease according to any one of [D1] to [D8] comprising administering in combination to a subject in need of treatment of the disease: a) the anti-HER2 antibody-drug conjugate; and b) an anti-HER2 antibody, wherein the anti-HER2 antibody described in b) is trastuzumab, pertuzumab, or margetuximab.
[D20] The method of treating a disease according to any one of [D1] to [D8] comprising administering in combination to a subject in need of treatment of the disease: a) the anti-HER2 antibody-drug conjugate; and b) an anti-HER2 antibody-containing compound, wherein the anti-HER2 antibody-containing compound is ado-trastuzumab emtansine (T-DM1), disitamab vedotin, or vic-trastuzumab duocarmazine.
[D21] The method according to any one of [D1] to [D20], wherein
   the anti-HER2 antibody-drug conjugate; and
   the immune checkpoint inhibitor, the chemotherapeutic agent, the molecular-targeted drug, the anti-HER2 antibody, or the anti-HER2 antibody-containing compound
   are each included as an active ingredient in different formulations and are administered simultaneously or at different times (sequentially or separately).
[D22] The method according to any one of [D1] to [D21], wherein the disease is cancer.
[D23] The method according to [D22], wherein the cancer is selected from the group consisting of lung cancer, colorectal cancer, prostate cancer, pancreatic cancer, ovarian cancer, breast cancer, bladder cancer, gastric cancer, esophageal cancer, endometrial cancer, cervical cancer, head and neck cancer, bile duct cancer, myeloma, and sarcoma.
[D24] The method according to [D22], wherein the cancer is lung cancer.
[D25] The method according to [D22], wherein the cancer is colorectal cancer.
[D26] The method according to [D22], wherein the cancer is breast cancer.
[D27] The method according to [D22], wherein the cancer is gastric cancer.
[D28] The method according to [D22], wherein the cancer is bladder cancer.

### Advantageous Effects of Invention

The present invention provides a safe novel drug combination therapy that exhibits a remarkable anti-tumor effect in the treatment of a disease, in particular, cancer.

### Brief Description of Drawings

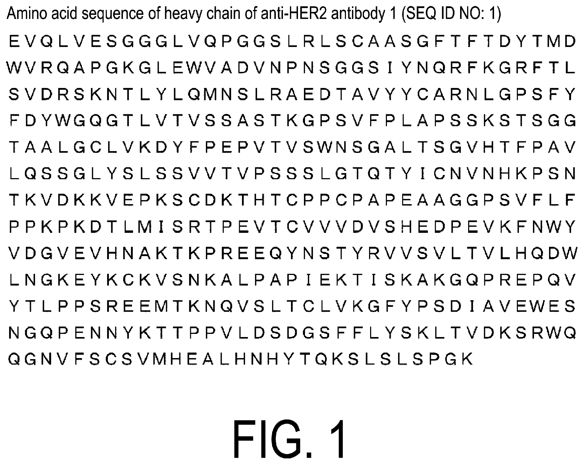

FIG. 1 shows the amino acid sequence (SEQ ID NO: 1) of the heavy chain of anti-HER2 antibody 1.

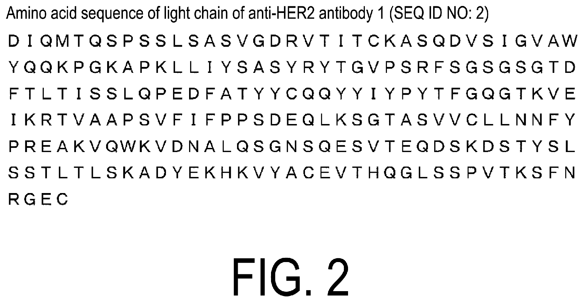

FIG. 2 shows the amino acid sequence (SEQ ID NO: 2) of the light chain of anti-HER2 antibody 1.

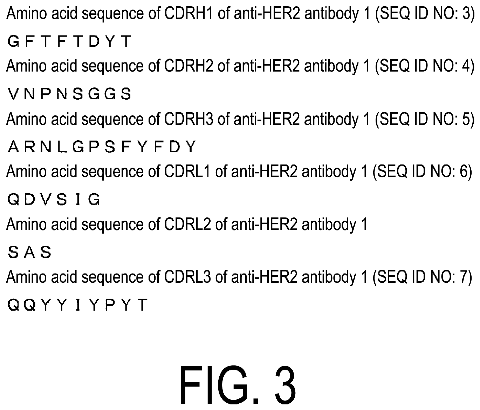

FIG. 3 shows the amino acid sequences of CDRH1 (SEQ ID NO: 3), CDRH2 (SEQ ID NO: 4), CDRH3 (SEQ ID NO: 5), CDRL1 (SEQ ID NO: 6), CDRL2, and CDRL3 (SEQ ID NO: 7) of anti-HER2 antibody 1.

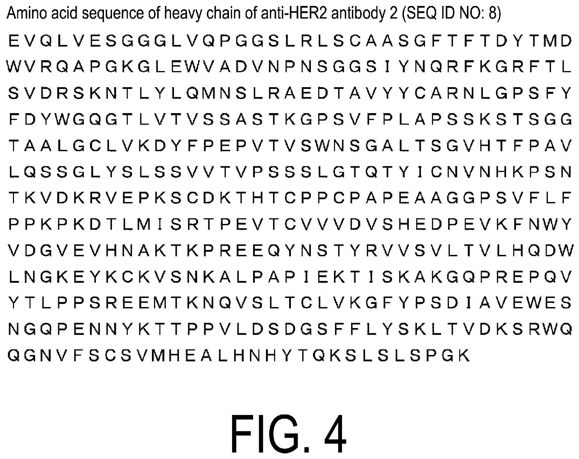

FIG. 4 shows the amino acid sequence (SEQ ID NO: 8) of the heavy chain of anti-HER2 antibody 2.

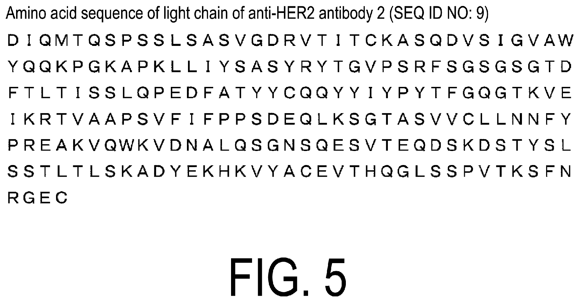

FIG. 5 shows the amino acid sequence (SEQ ID NO: 9) of the light chain of anti-HER2 antibody 2.

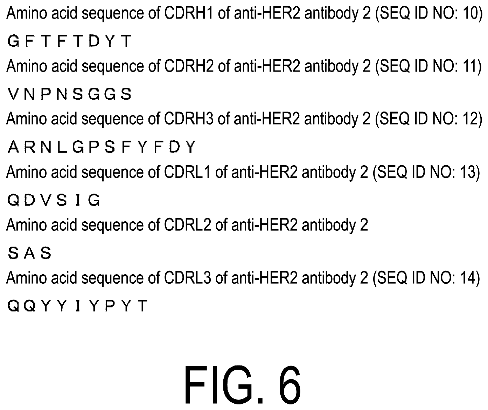

FIG. 6 shows the amino acid sequences of CDRH1 (SEQ ID NO: 10), CDRH2 (SEQ ID NO: 11), CDRH3 (SEQ ID NO: 12), CDRL1 (SEQ ID NO: 13), CDRL2, and CDRL3 (SEQ ID NO: 14) of anti-HER2 antibody 2.

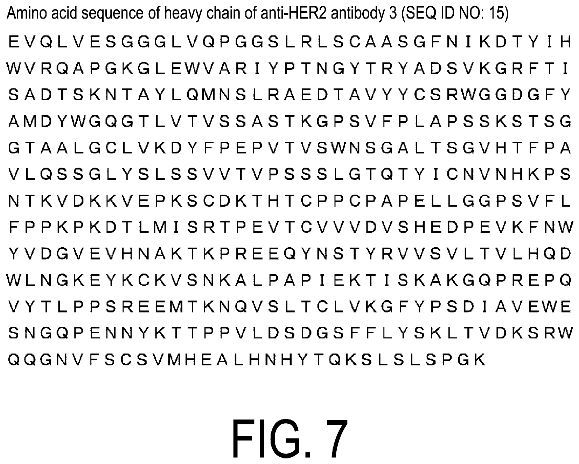

FIG. 7 shows the amino acid sequence (SEQ ID NO: 15) of the heavy chain of anti-HER2 antibody 3.

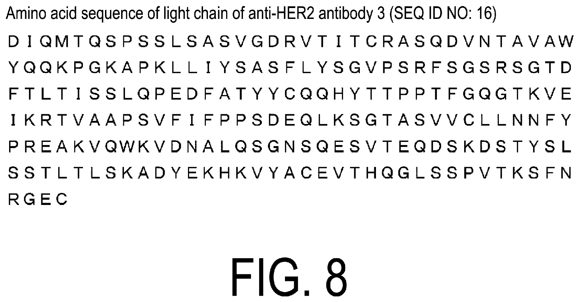

FIG. 8 shows the amino acid sequence (SEQ ID NO: 16) of the light chain of anti-HER2 antibody 3.

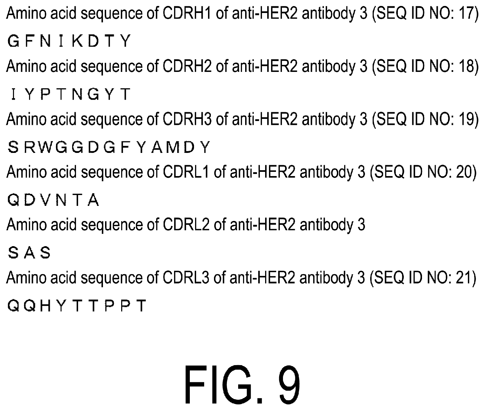

FIG. 9 shows the amino acid sequences of CDRH1 (SEQ ID NO: 17), CDRH2 (SEQ ID NO: 18), CDRH3 (SEQ ID NO: 19), CDRL1 (SEQ ID NO: 20), CDRL2, and CDRL3 (SEQ ID NO: 21) of anti-HER2 antibody 3.

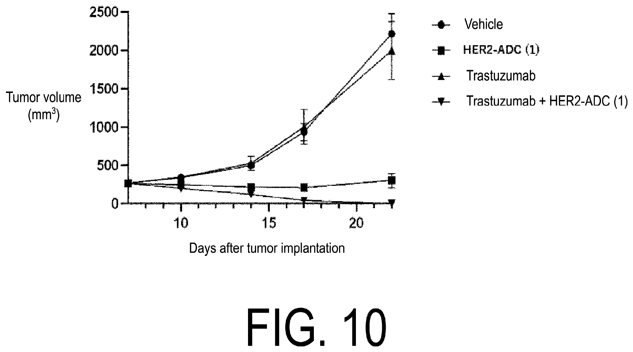

FIG. 10 is a graph showing a anti-tumor combination effect of intravenous administrations of HER2-ADC (1) and trastuzumab in mice implanted subcutaneously with CT26.WT-HER2 cells obtained by introducing the human HER2 gene into a mouse colorectal cancer cell line CT26.WT. In the graph, the line with black circles represents a vehicle group, the line with black squares represents a HER2-ADC (1) administration group, the line with black triangles represents a trastuzumab administration group, and the line with black inverted triangles represents a combination administration group of HER2-ADC (1) and trastuzumab. The vertical axis represents tumor volume (mm³), and the horizontal axis represents days after tumor implantation.

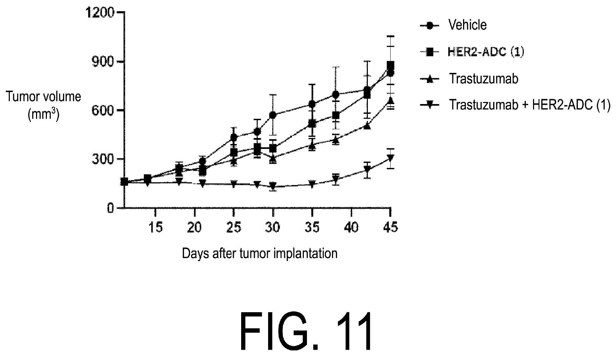

FIG. 11 is a graph showing a anti-tumor combination effect of intravenous administrations of HER2-ADC (1) and trastuzumab in mice implanted subcutaneously with a human breast cancer cell line JIMT-1. In the graph, the line with black circles represents a vehicle group, the line with black squares represents a HER2-ADC (1) administration group, the line with black triangles represents a trastuzumab administration group, and the line with black inverted triangles represents a combination administration group of HER2-ADC (1) and trastuzumab. The vertical axis represents tumor volume (mm³), and the horizontal axis represents days after tumor implantation.

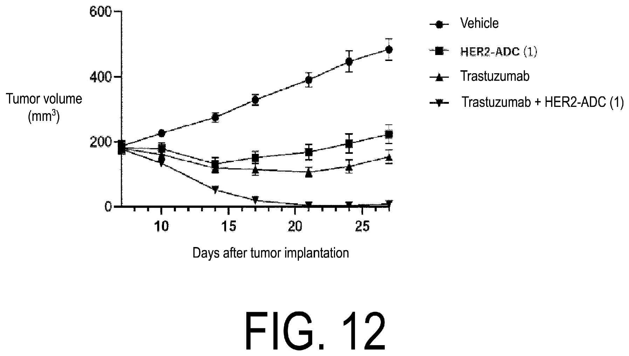

FIG. 12 is a graph showing a anti-tumor combination effect of intravenous administrations of HER2-ADC (1) and trastuzumab in mice implanted subcutaneously with a human gastric cancer cell line NCI-N87. In the graph, the line with black circles represents a vehicle group, the line with black squares represents a HER2-ADC (1) administration group, the line with black triangles represents a trastuzumab administration group, and the line with black inverted triangles represents a combination administration group of HER2-ADC (1) and trastuzumab. The vertical axis represents tumor volume (mm³), and the horizontal axis represents days after tumor implantation.

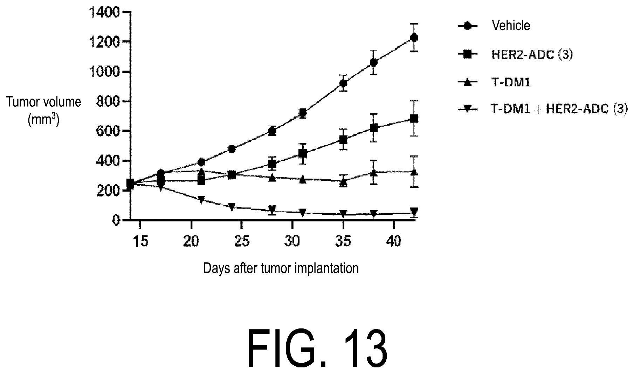

FIG. 13 is a graph showing a anti-tumor combination effect of intravenous administrations of HER2-ADC (3) and T-DM1 in mice implanted subcutaneously with a human breast cancer cell line KPL-4. In the graph, the line with black circles represents a vehicle group, the line with black squares represents a HER2-ADC (3) administration group, the line with black triangles represents a T-DM1 administration group, and the line with black inverted triangles represents a combination administration group of HER2-ADC (3) and T-DM1. The vertical axis represents tumor volume (mm³), and the horizontal axis represents days after tumor implantation.

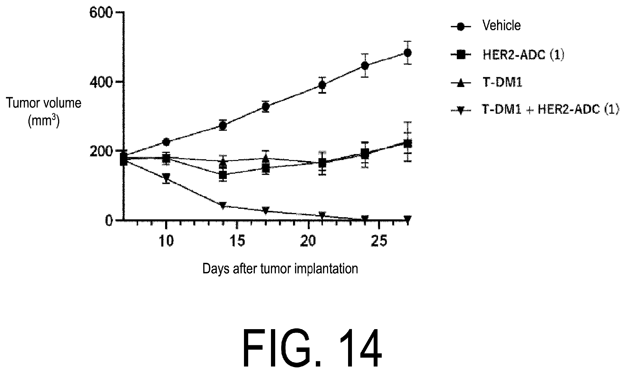

FIG. 14 is a graph showing a anti-tumor combination effect of intravenous administrations of HER2-ADC (1) and T-DM1 in mice implanted subcutaneously with the human gastric cancer cell line NCI-N87. In the graph, the line with black circles represents a vehicle group, the line with black squares represents a HER2-ADC (1) administration group, the line with black triangles represents a T-DM1 administration group, and the line with black inverted triangles represents a combination administration group of HER2-ADC (1) and T-DM1. The vertical axis represents tumor volume (mm³), and the horizontal axis represents days after tumor implantation.

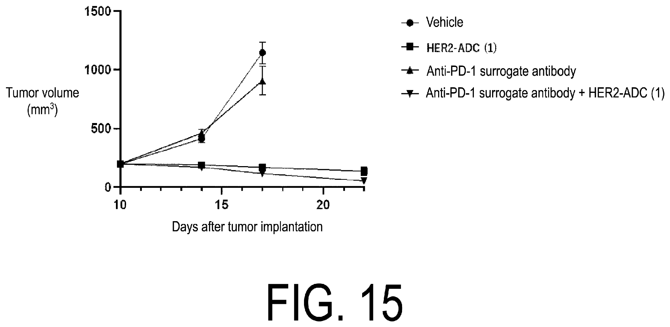

FIG. 15 is a graph showing a anti-tumor combination effect of intravenous administrations of HER2-ADC (1) and an anti-PD-1 surrogate antibody in mice implanted subcutaneously with LL/2-HER2 cells obtained by introducing the human HER2 gene into a mouse lung cancer cell line LL/2. In the graph, the line with black circles represents a vehicle group, the line with black squares represents a HER2-ADC (1) administration group, the line with black triangles represents an anti-PD-1 surrogate antibody administration group, and the line with black inverted triangles represents a combination administration group of HER2-ADC (1) and the anti-PD-1 surrogate antibody. The vertical axis represents tumor volume (mm³), and the horizontal axis represents days after tumor implantation.

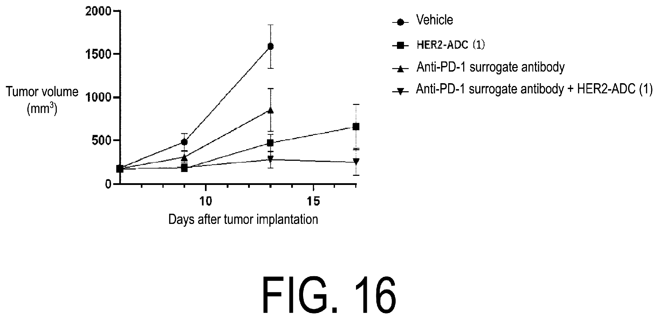

FIG. 16 is a graph showing a anti-tumor combination effect of intravenous administrations of HER2-ADC (1) and an anti-PD-1 surrogate antibody in mice implanted subcutaneously with CT26.WT-chimera HER2 cells prepared by introducing a human-mouse chimera HER2 gene, in which the epitope site of an anti-HER2 antibody is replaced with a human type, into the mouse colorectal cancer cell line CT26.WT. In the graph, the line with black circles represents a vehicle group, the line with black squares represents a HER2-ADC (1) administration group, the line with black triangles represents an anti-PD-1 surrogate antibody administration group, and the line with black inverted triangles represents a combination administration group of HER2-ADC (1) and the anti-PD-1 surrogate antibody. The vertical axis represents tumor volume (mm³), and the horizontal axis represents days after tumor implantation.

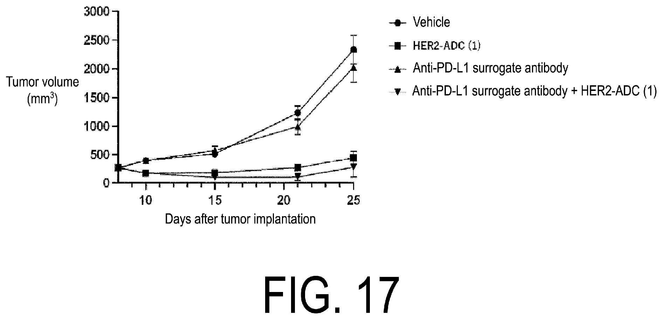

FIG. 17 is a graph showing a anti-tumor combination effect of intravenous administrations of HER2-ADC (1) and an anti-PD-L1 surrogate antibody in mice implanted subcutaneously with CT26.WT-HER2 cells obtained by introducing the human HER2 gene into the mouse colorectal cancer cell line CT26.WT. In the graph, the line with black circles represents a vehicle group, the line with black squares represents a HER2-ADC (1) administration group, the line with black triangles represents an anti-PD-L1 surrogate antibody administration group, and the line with black inverted triangles represents a combination administration group of HER2-ADC (1) and the anti-PD-L1 surrogate antibody. The vertical axis represents tumor volume (mm³), and the horizontal axis represents days after tumor implantation.

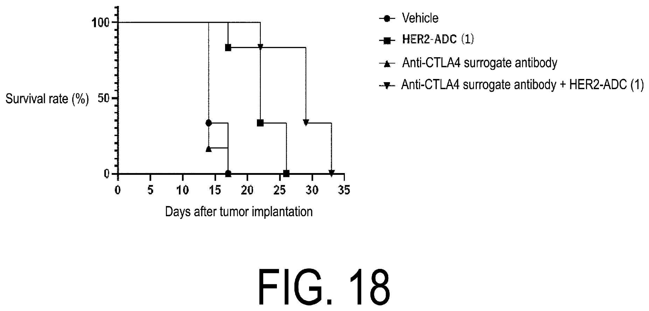

FIG. 18 is a graph showing a life-extending effect of intravenous administrations of HER2-ADC (1) and an anti-CTLA-4 surrogate antibody in mice implanted subcutaneously with CT26.WT-chimera HER2 cells prepared by introducing a human-mouse chimera HER2 gene, in which the epitope site of an anti-HER2 antibody is replaced with a human type, into the mouse colorectal cancer cell line CT26.WT. In the graph, the line with black circles represents a vehicle group, the line with black squares represents a HER2-ADC (1) administration group, the line with black triangles represents an anti-CTLA-4 surrogate antibody administration group, and the line with black inverted triangles represents a combination administration group of HER2-ADC (1) and the anti-CTLA-4 surrogate antibody. The vertical axis represents a survival rate (%), and the horizontal axis represents days after tumor implantation.

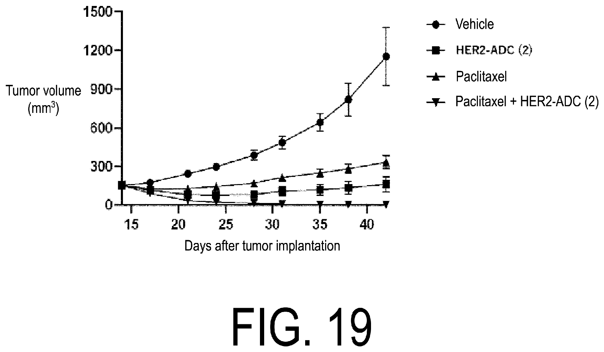

FIG. 19 is a graph showing a combined anti-tumor effect of intravenous administrations of HER2-ADC (2) and paclitaxel in mice implanted subcutaneously with the human breast cancer cell line KPL-4. In the graph, the line with black circles represents a vehicle group, the line with black squares represents a HER2-ADC (2) administration group, the line with black triangles represents a paclitaxel administration group, and the line with black inverted triangles represents a combination administration group of HER2-ADC (2) and paclitaxel. The vertical axis represents tumor volume (mm³), and the horizontal axis represents days after tumor implantation.

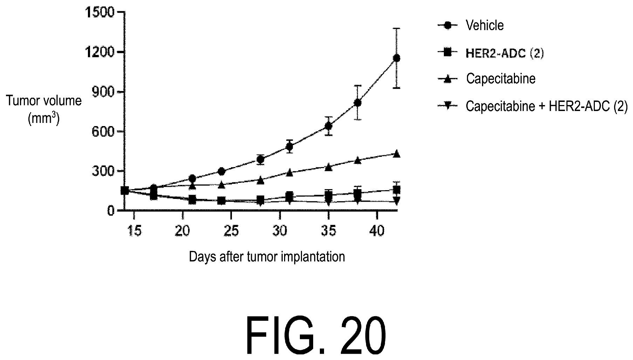

FIG. 20 is a graph showing a combined anti-tumor effect of intravenous administration of HER2-ADC (2) and oral administration of capecitabine in mice implanted subcutaneously with the human breast cancer cell line KPL-4. In the graph, the line with black circles represents a vehicle group, the line with black squares represents a HER2-ADC (2) administration group, the line with black triangles represents a capecitabine administration group, and the line with black inverted triangles represents a combination administration group of HER2-ADC (2) and capecitabine. The vertical axis represents tumor volume (mm³), and the horizontal axis represents days after tumor implantation.

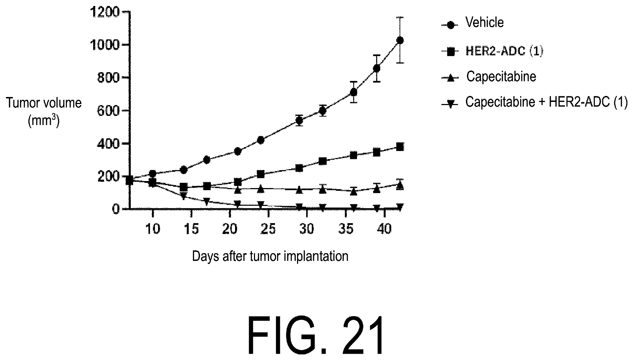

FIG. 21 is a graph showing a combined anti-tumor effect of intravenous administration of HER2-ADC (1) and oral administration of capecitabine in mice implanted subcutaneously with the human gastric cancer cell line NCI-N87. In the graph, the line with black circles represents a vehicle group, the line with black squares represents a HER2-ADC (1) administration group, the line with black triangles represents a capecitabine administration group, and the line with black inverted triangles represents a combination administration group of HER2-ADC (1) and capecitabine. The vertical axis represents tumor volume (mm³), and the horizontal axis represents days after tumor implantation.

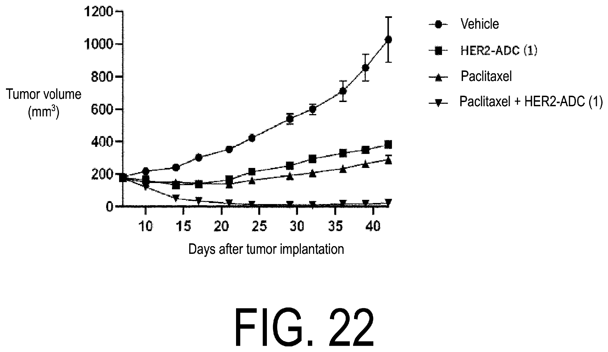

FIG. 22 is a graph showing a combined anti-tumor effect of intravenous administrations of HER2-ADC (1) and paclitaxel in mice implanted subcutaneously with the human gastric cancer cell line NCI-N87. In the graph, the line with black circles represents a vehicle group, the line with black squares represents a HER2-ADC (1) administration group, the line with black triangles represents a paclitaxel administration group, and the line with black inverted triangles represents a combination administration group of HER2-ADC (1) and paclitaxel. The vertical axis represents tumor volume (mm³), and the horizontal axis represents days after tumor implantation.

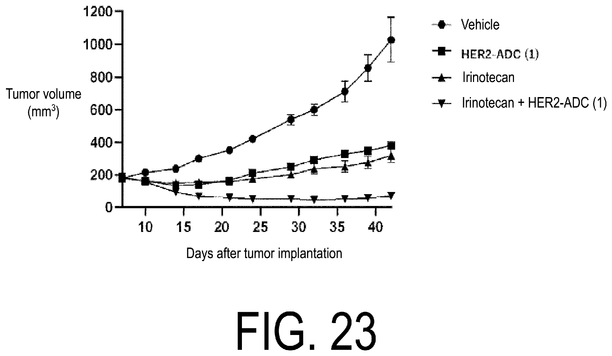

FIG. 23 is a graph showing a combined anti-tumor effect of intravenous administrations of HER2-ADC (1) and irinotecan in mice implanted subcutaneously with the human gastric cancer cell line NCI-N87. In the graph, the line with black circles represents a vehicle group, the line with black squares represents a HER2-ADC (1) administration group, the line with black triangles represents an irinotecan administration group, and the line with black inverted triangles represents a combination administration group of HER2-ADC (1) and irinotecan. The vertical axis represents tumor volume (mm³), and the horizontal axis represents days after tumor implantation.

### Description of Embodiments

Hereinafter, preferred embodiments for carrying out the present invention will be described. The embodiments described below are merely one example of representative embodiments of the present invention; the scope of the present invention is not narrowly construed by these embodiments.

### 1. Anti-HER2 Antibody-Drug Conjugate

The anti-HER2 antibody-drug conjugate used in the present invention is represented by Formula (I):

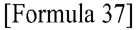

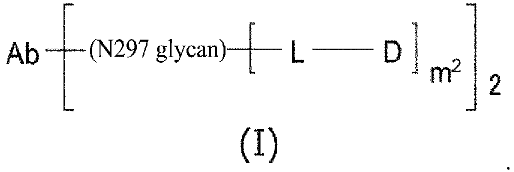

Ab represents an anti-HER2 antibody or an antigen-binding fragment of the antibody; the N297 glycan represents an N-linked glycan linked to the side chain of Asn at position 297 of the IgG heavy chain included in Ab; L represents a linker connecting the N297 glycan and D; D represents a drug that is a cyclic dinucleotide derivative; and m² represents an integer of 1 or 2.

### 1-1. Anti-HER2 Antibody

The anti-HER2 antibody in the antibody-drug conjugate used in the present invention may be derived from any species but is preferably an antibody derived from a human, a rat, a mouse, or a rabbit. If the antibody is derived from a species other than a human, it is preferably chimerized or humanized using a well-known technique. The anti-HER2 antibody in the antibody-drug conjugates used in the present invention may be either a polyclonal antibody or a monoclonal antibody but is preferably a monoclonal antibody.

The heavy and light chains of an antibody molecule are each known to have three complementarity determining regions (CDRs). CDRs, also called hypervariable regions, are sites within the variable regions of the heavy and light chains of an antibody and have particularly high variability of the primary structure, where the sites are at three separate locations on the primary structure of each of the heavy and light polypeptide chains. In the present specification, for CDRs of the antibody, CDRs of the heavy chain are denoted as CDRH1, CDRH2, and CDRH3 from the amino-terminal side of the amino acid sequence of the heavy chain, and CDRs of the light chain are denoted as CDRL1, CDRL2, and CDRL3 from the amino-terminal side of the amino acid sequence of the light chain. These sites are in close proximity to each other in the three-dimensional structure and determine the specificity for the antigen to which they bind.

In the present invention, the positions and lengths of the CDRs were determined according to the IMGT definition (Developmental and Comparative Immunology 27 (2003) 55-77).

Multiple allotypes are known to be present in the constant region of an antibody. Examples of the allotypes for the IgG1 heavy chain include G1m17, G1m3, G1m1, and G1m2. The constant region of the antibody used in the present invention is not particularly limited, but G1m17 or G1m3 is preferably used.

In the present invention, the term "antigen-binding fragment of the anti-HER2 antibody" means a fragment of the antibody that has antigen-binding activity; the fragment has an N-linked glycan well conserved at the asparagine residue at position 297 (hereinafter referred to as "Asn297 or N297") in the Fc region of the heavy chain of IgG (the glycan is referred to as the "Asn297 glycan" or "N297 glycan" in the present specification and claims). However, the antigen-binding fragment is not limited to these molecules as long as it has antigen-binding activity. In addition, these antigen-binding fragments include not only fragments obtained by treating the full-length molecule of the antibody protein with an appropriate enzyme but also proteins produced in appropriate host cells by using a gene which is modified by genetic engineering.

The "anti-HER2 antibody" in the antibody-drug conjugate used in the present invention is an antibody that specifically binds to human epidermal growth factor receptor type 2 (ErbB-2; HER2) and preferably has an activity of internalizing into HER2-expressing cells by binding to HER2.

Examples of the anti-HER2 antibody include trastuzumab (US 5821337) and pertuzumab (WO 2001/00245), and preferably include pertuzumab.

Replacement of a part of the amino acid residues of the constant region makes it possible to modulate the effector function (see WO 88/07089, WO 94/28027, and WO 94/29351). Examples of the IgG1 variant include the IgG1 LALA mutation (IgG1-L234A, L235A). The L234A and L235A indicate the replacement of leucines at positions 234 and 235, specified by the EU index (Proceedings of the National Academy of Sciences of the United States of America, Vol. 63, No. 1 (May 15, 1969), pp. 78-85), with alanines.

Examples of the anti-HER2 antibody include antibodies in which the heavy chain constant region is a heavy chain constant region of the human IgG1 and leucines at positions 234 and 235 indicated by the EU index are replaced with alanines, and preferably include antibodies comprising a heavy chain consisting of the amino acid sequence set forth in SEQ ID NO: 1 and a light chain consisting of the amino acid sequence set forth in SEQ ID NO: 2.

Furthermore, it is known that in the production in mammalian cultured cells, a lysine residue at the carboxyl terminus of the heavy chain of the antibody is deleted (Journal of Chromatography A, 705: 129-134 (1995)), and it is also known that two amino acid residues, glycine and lysine, at the carboxyl terminus of the heavy chain are deleted and a proline residue newly located at the carboxyl terminus is amidated (Analytical Biochemistry, 360: 75 to 83 (2007)). Thus, the antibody according to the present invention also includes deletion mutants in which one or two amino acids are deleted at the heavy chain carboxyl terminus, and amidated deletion mutants (e.g., antibodies in which a proline residue at the heavy chain carboxyl terminus site is amidated).

In the present invention, examples of the heavy chain and the light chain included in the anti-HER2 antibody, that is, the antibody or the antigen-binding fragment of the antibody that binds to HER2, include, but are not limited to (i) to (xvi) below:
(i)
   (i-H) a heavy chain comprising CDRH1 consisting of the amino acid sequence represented by SEQ ID NO: 3, CDRH2 consisting of the amino acid sequence represented by SEQ ID NO: 4, and CDRH3 consisting of the amino acid sequence represented by SEQ ID NO: 5, and
   (i-L) a light chain comprising CDRL1 consisting of the amino acid sequence represented by SEQ ID NO: 6, CDRL2 consisting of the amino acid sequence represented by SAS, and CDRL3 consisting of the amino acid sequence represented by SEQ ID NO: 7;
(ii)
   a combination of the heavy chain and the light chain described in (i),
   (ii-H) a heavy chain comprising a heavy chain variable region including an amino acid sequence having 80% or more, preferably 90% or more, and more preferably 95% or more identity with the amino acid sequence represented by the amino acid residues 1 to 119 of SEQ ID NO: 1, and
   (ii-L) a light chain comprising a light chain variable region including an amino acid sequence having 80% or more, preferably 90% or more, and more preferably 95% or more identity with the amino acid sequence represented by the amino acid residues 1 to 108 of SEQ ID NO: 2;
(iii)
   a combination of the heavy chain and the light chain described in (i) or (ii),
   (iii-H) a heavy chain comprising a heavy chain variable region consisting of the amino acid sequence represented by the amino acid residues 1 to 119 of SEQ ID NO: 1, and
   (iii-L) a light chain comprising a light chain variable region consisting of the amino acid sequence represented by the amino acid residues 1 to 108 of SEQ ID NO: 2;
(iv)
   a combination of the heavy chain and the light chain described in (i), (ii), or (iii),
   (iv-H) a heavy chain consisting of an amino acid sequence having 80% or more, preferably 90% or more, and more preferably 95% or more identity with the amino acid sequence represented by SEQ ID NO: 1, and
   (iv-L) a light chain consisting of an amino acid sequence having 80% or more, preferably 90% or more, and more preferably 95% or more identity with the amino acid sequence represented by SEQ ID NO: 2;
(v)
   a combination of the heavy chain and the light chain described in (i), (ii), (iii), or (iv),
   (v-H) a heavy chain consisting of the amino acid sequence represented by SEQ ID NO: 1, and
   (v-L) a light chain consisting of the amino acid sequence represented by SEQ ID NO: 2;
(vi)
   (vi-H) a heavy chain comprising CDRH1 consisting of the amino acid sequence represented by SEQ ID NO: 10, CDRH2 consisting of the amino acid sequence represented by SEQ ID NO: 11, and CDRH3 consisting of the amino acid sequence represented by SEQ ID NO: 12, and
   (vi-L) a light chain comprising CDRL1 consisting of the amino acid sequence represented by SEQ ID NO: 13, CDRL2 consisting of the amino acid sequence represented by SAS, and CDRL3 consisting of the amino acid sequence represented by SEQ ID NO: 14;
(vii)
   a combination of the heavy chain and the light chain described in (vi),
   (vii-H) a heavy chain comprising a heavy chain variable region consisting of an amino acid sequence having 80% or more, preferably 90% or more, and more preferably 95% or more identity with the amino acid sequence represented by the amino acid residues 1 to 119 of SEQ ID NO: 8, and
   (vii-L) a light chain comprising a light chain variable region consisting of an amino acid sequence having 80% or more, preferably 90% or more, and more preferably 95% or more identity with the amino acid sequence represented by the amino acid residues 1 to 108 of SEQ ID NO: 9;
(viii)
   a combination of the heavy chain and the light chain described in (vi) or (vii),
   (viii-H) a heavy chain comprising a heavy chain variable region consisting of the amino acid sequence represented by the amino acid residues 1 to 119 of SEQ ID NO: 8, and
   (viii-L) a light chain comprising a light chain variable region consisting of the amino acid sequence represented by the amino acid residues 1 to 108 of SEQ ID NO: 9;
(ix)
   a combination of the heavy chain and the light chain described in (vi), (vii), or (viii),
   (ix-H) a heavy chain consisting of an amino acid sequence having 80% or more, preferably 90% or more, and more preferably 95% or more identity with the amino acid sequence represented by SEQ ID NO: 8, and
   (ix-L) a light chain consisting of an amino acid sequence having 80% or more, preferably 90% or more, and more preferably 95% or more identity with the amino acid sequence represented by SEQ ID NO: 9;
(x)
   a combination of the heavy chain and the light chain described in (vi), (vii), (viii), or (ix),
   (x-H) a heavy chain consisting of the amino acid sequence represented by SEQ ID NO: 8, and
   (x-L) a light chain consisting of the amino acid sequence represented by SEQ ID NO: 9;
(xi)
   (xi-H) a heavy chain comprising CDRH1 consisting of the amino acid sequence represented by SEQ ID NO: 17, CDRH2 consisting of the amino acid sequence represented by SEQ ID NO: 18, and CDRH3 consisting of the amino acid sequence represented by SEQ ID NO: 19, and
   (xi-L) a light chain comprising CDRL1 consisting of the amino acid sequence represented by SEQ ID NO: 20, CDRL2 consisting of the amino acid sequence represented by SAS, and CDRL3 consisting of the amino acid sequence represented by SEQ ID NO: 21;
(xii)
   a combination of the heavy chain and the light chain described in (xi),
   (xii-H) a heavy chain comprising a heavy chain variable region consisting of an amino acid sequence having 80% or more, preferably 90% or more, and more preferably 95% or more identity with the amino acid sequence represented by the amino acid residues 1 to 120 of SEQ ID NO: 15, and
   (xii-L) a light chain comprising a light chain variable region consisting of an amino acid sequence having 80% or more, preferably 90% or more, and more preferably 95% or more identity with the amino acid sequence represented by the amino acid residues 1 to 108 of SEQ ID NO: 16;
(xiii)
   a combination of the heavy chain and the light chain described in (xi) or (xii),
   (xiii-H) a heavy chain comprising a heavy chain variable region consisting of the amino acid sequence represented by the amino acid residues 1 to 120 of SEQ ID NO: 15, and
   (xiii-L) a light chain comprising a light chain variable region consisting of the amino acid sequence represented by the amino acid residues 1 to 108 of SEQ ID NO: 16;
(xiv)
   a combination of the heavy chain and the light chain described in (xi), (xii), or (xiii), including:
   (xiv-H) a heavy chain consisting of an amino acid sequence having 80% or more, preferably 90% or more, and more preferably 95% or more identity with the amino acid sequence represented by SEQ ID NO: 15, and
   (xiv-L) a light chain consisting of an amino acid sequence having 80% or more, preferably 90% or more, and more preferably 95% or more identity with the amino acid sequence of SEQ ID NO: 16;
(xv)
   a combination of the heavy chain and the light chain described in (xi), (xii), (xiii), or (xiv),
   (xv-H) a heavy chain consisting of the amino acid sequence represented by SEQ ID NO: 15, and
   (xv-L) a light chain consisting of the amino acid sequence represented by SEQ ID NO: 16; and
(xvi)
   (xvi-a) a heavy chain consisting of an amino acid sequence in which one or two amino acids are deleted from the carboxyl terminus of the amino acid sequence of the heavy chain (heavy chain a) described in any one of (i-H) to (v-H), and a light chain (light chain a) combined with the heavy chain a in (i) to (v);
   (xvi-b) a heavy chain consisting of an amino acid sequence in which one or two amino acids are deleted from the carboxyl terminus of the amino acid sequence of the heavy chain (heavy chain b) described in any one of (vi-H) to (x-H), and a light chain (light chain b) combined with the heavy chain b in (vi) to (x); and
   (xvi-c) a heavy chain consisting of an amino acid sequence in which one or two amino acids are deleted from the carboxyl terminus of the amino acid sequence of the heavy chain (heavy chain c) described in any one of (xi-H) to (xv-H), and a light chain (light chain c) combined with the heavy chain c in (xi) to (xv).

Examples preferably include (i) to (v), (xv), (xvi-a), and (xvi-c); more preferably (i) to (v) and (xvi-a); and even more preferably (v) and (xvi-a). The anti-HER2 antibody or an antigen-binding fragment of the antibody of the present invention may preferably contain an Fc region (wild-type or variant) of an antibody, more preferably an Fc region derived from IgG (same as above), and even more preferably an Fc region (same as above) derived from human IgG (preferably human IgG1). The variant Fc region may be a naturally occurring Fc region or an artificially created Fc region.

The numerical value of "80%", "90%", or "95%" in (ii), (iv), (vii), (ix), (xii), or (xiv) above is not limited to these, and may be, for example, any of 80%, 81%, 82%, 83%, 84%, 85%, 86%, 87%, 88%, 89%, 90%, 91%, 92%, 93%, 94%, 95%, 96%, 97%, 98%, or 99%, and may preferably be the numerical value of 90% or more, and more preferably the numerical value of 95% or more.

### 1-2. N297 Glycan of Antibody

IgG has an N-linked glycan well conserved at the asparagine residue at position 297 (hereinafter referred to as "Asn297" or "N297") in the Fc region of the heavy chain (hereinafter the glycan is referred to as the "Asn297 glycan" or "N297 glycan"); this glycan is known to contribute to the activity, kinetics, and the like of the antibody molecule (Eon-Duval, A. et al, Biotechnol. Prog. 2012, 28, 608-622; Sanglier-Cianferani, S., Anal. Chem. 2013, 85, 715-736).

The amino acid sequences in the constant regions of IgG are well conserved, and each amino acid is specified by an EU index in the report of Edelman et al. (Proc. Natl. Acad. Sci. U.S.A., 63, 78-85, (1969)). For example, Asn297, to which an N-linked glycan is added, in the Fc region corresponds to position 297 in the EU index, and even when the actual amino acid position varies due to molecular fragmentation or domain deletion, the amino acid is unambiguously specified by indicating the EU index.

The N297 glycan used in the antibody-drug conjugate of the present invention is N297-(Fuc)SG or N297-(Fuc)MSG1, and preferably N297-(Fuc)SG.

N297-(Fuc)SG is represented by a structural formula or sequence formula below:

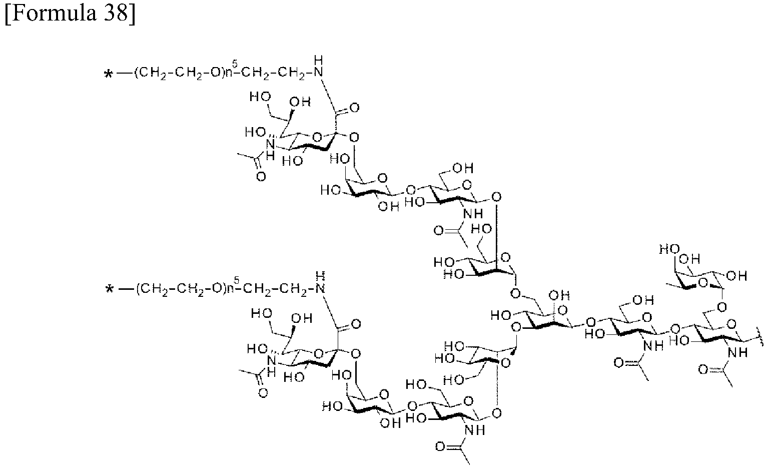

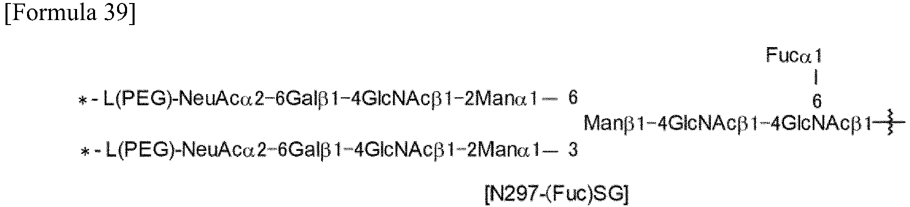

wherein the wavy line represents binding to Asn297 of the antibody;
L(PEG) represents -(CH₂-CH₂-O)n⁵-CH₂-CH₂-NH-, and the amino group at the right end of the L(PEG) is amide-linked to the carboxyl group at the 2-position of the non-reducing-end sialic acid on each of the β-Man 1-3 and 1-6 branch chains of the N297 glycan;
the asterisk represents binding to the nitrogen atom at the 1-position or 3-position on the 1,2,3-triazole ring of Lb in the linker L; and
n⁵ is an integer of 2 to 5 and is preferably 3.

N297-(Fuc)MSG1 is represented by a structural formula or sequence formula below:

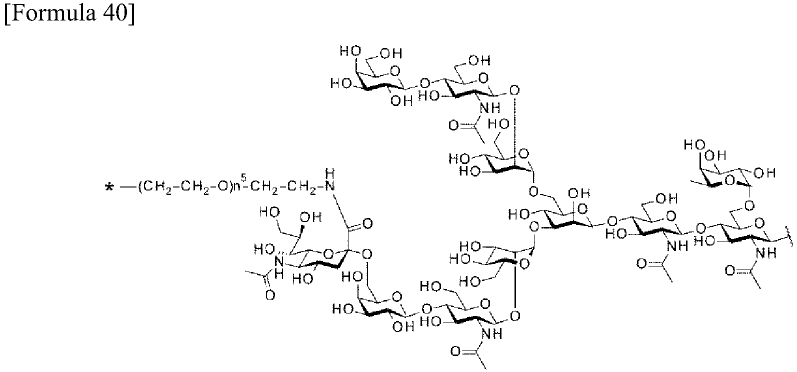

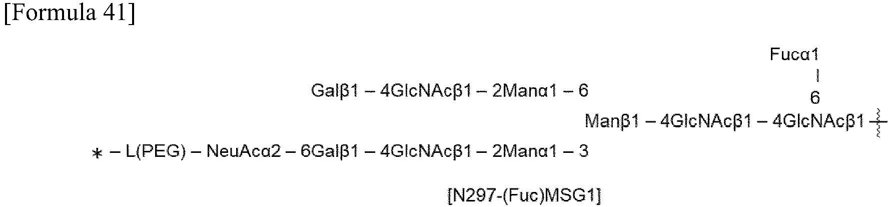

wherein the wavy line represents binding to Asn297 of the antibody;
L(PEG) represents -(CH₂-CH₂-O)n⁵-CH₂-CH₂-NH- and the amino group at the right end of the L(PEG) is amide-linked to the carboxyl group at the 2-position of the non-reducing-end sialic acid on the β-Man 1-3 branch chain of the N297 glycan;
the asterisk represents binding to the nitrogen atom at the 1-position or 3-position on the 1,2,3-triazole ring of Lb in the linker L; and
n⁵ is an integer of 2 to 5 and is preferably 3.

In the case where the N297 glycan of the antibody in the antibody-drug conjugate of the present invention is N297-(Fuc)SG, the antibody-drug conjugate is a molecule to which four linkers L and four drugs D are bound (m² above is 2).

In the case where the N297 glycan of the antibody in the antibody-drug conjugate of the present invention is N297-(Fuc)MSG1, the antibody-drug conjugate is a molecule to which two linkers L and two drugs D are bound (m² above is 1).

In the case where the N297 glycan in the antibody-drug conjugate of the present invention is N297-(Fuc)SG or N297-(Fuc)MSG1, a highly homogeneous ADC can be obtained.

### 1-3. Drug

The drug (D in Formula (I) above, also referred to as drug D) of the anti-HER2 antibody-drug conjugate used in the present invention is a cyclic dinucleotide derivative represented by: 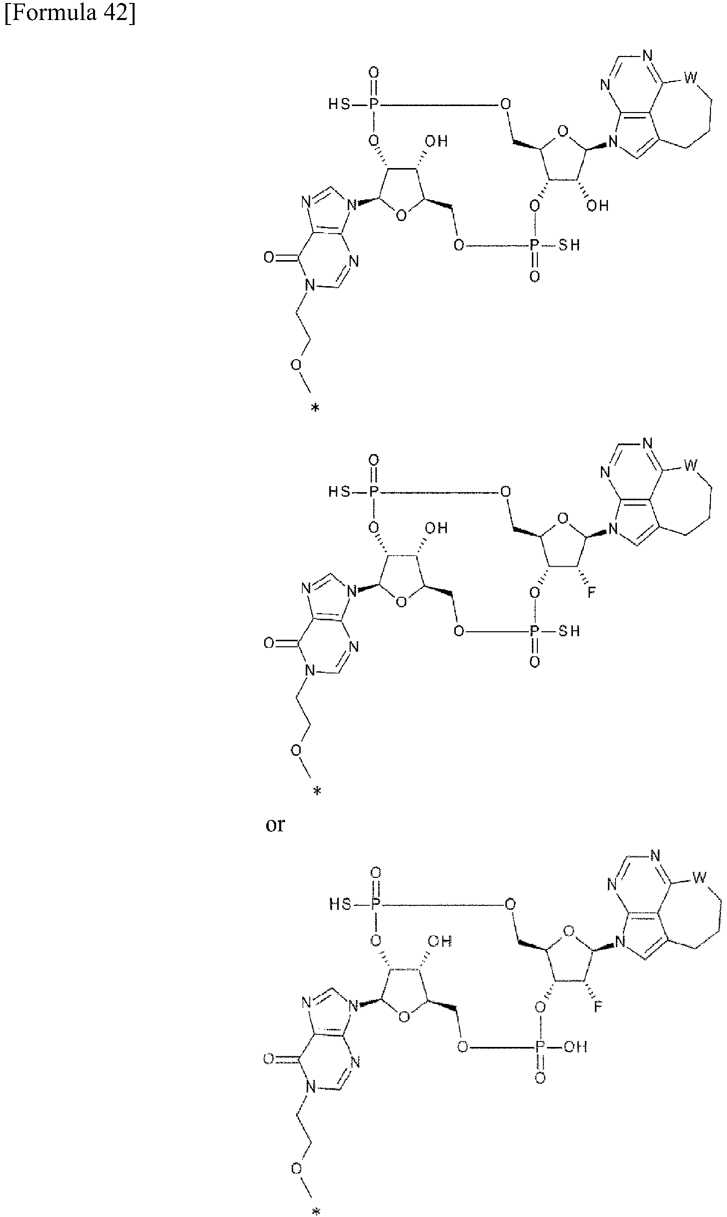 wherein the asterisk represents binding to L, and W represents -NH-.

The drug D is preferably 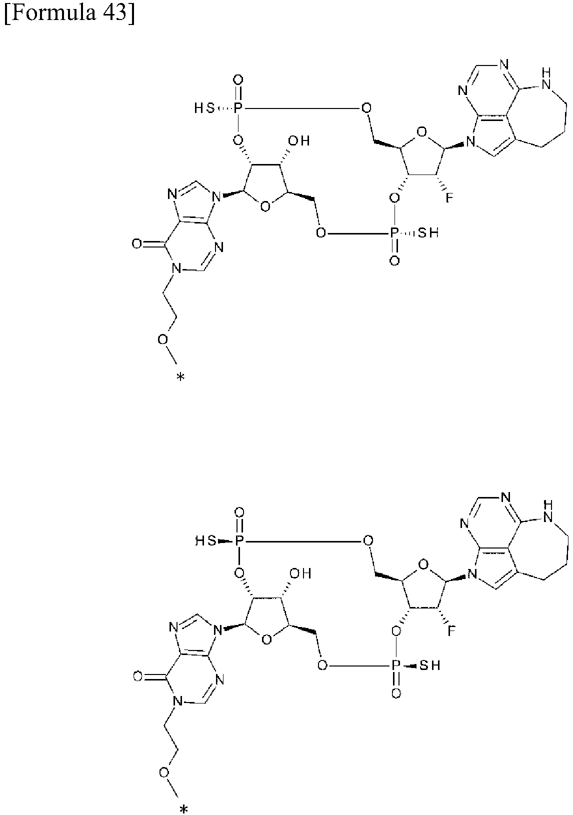 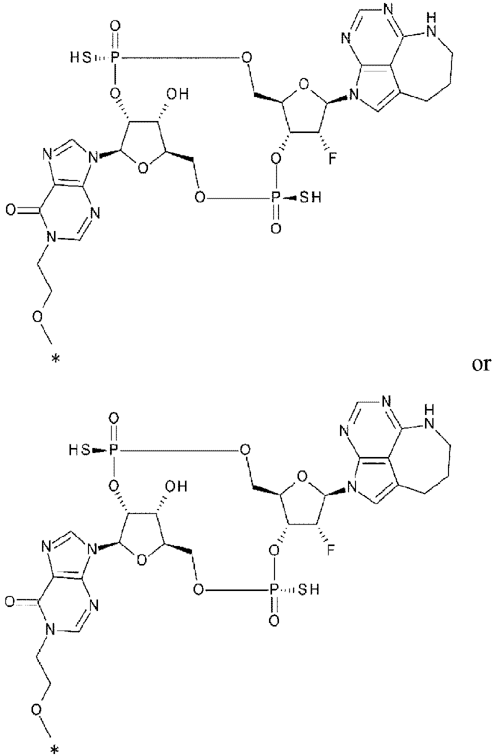 and more preferably 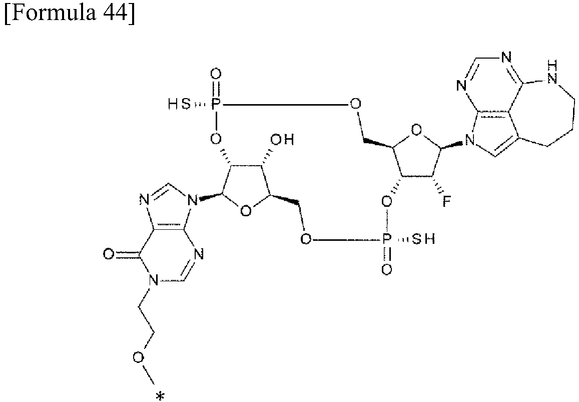 wherein the asterisk represents binding to L.

### 1-4. Linker

The linker connecting the N297 glycan and the drug D is represented by -Lb-La-Lp-Lc-*;
the asterisk represents binding to the drug D;
Lp is -GGFG- or -GGPI-, and preferably -GGFG-;
La represents -C(=O)-CH₂CH₂-C(=O)-;
Lb represents a formula below: 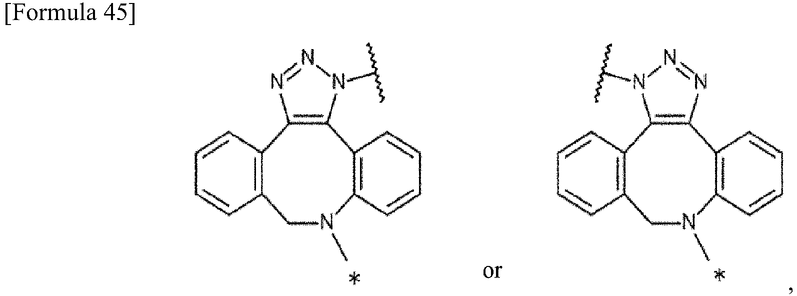
wherein in the structural formula of Lb shown above, the asterisk represents binding to La, and the wavy line represents binding to the N297 glycan; and
Lc represents -NH-CH₂-.

### 1-5. Drug Linker

In the present invention, the drug linker means a compound in which the drug D in the antibody-drug conjugate is bound to the linker moiety; a drug linker of Formula x1, Formula x2, Formula y1, or Formula y2 is preferably used, and a drug linker of Formula x1 or Formula x2 is more preferably used: 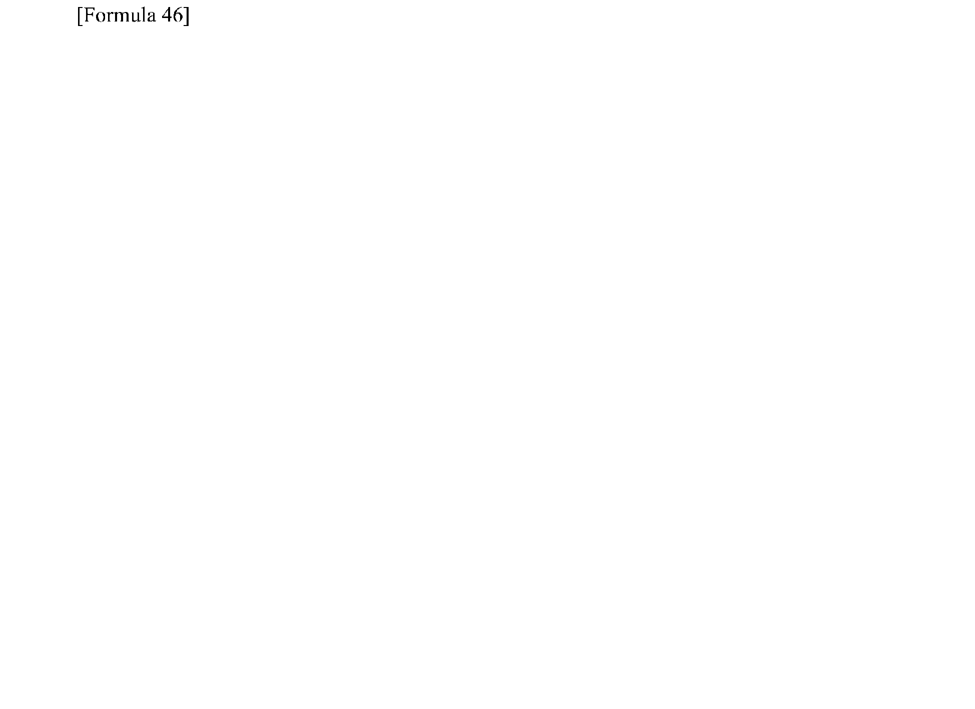 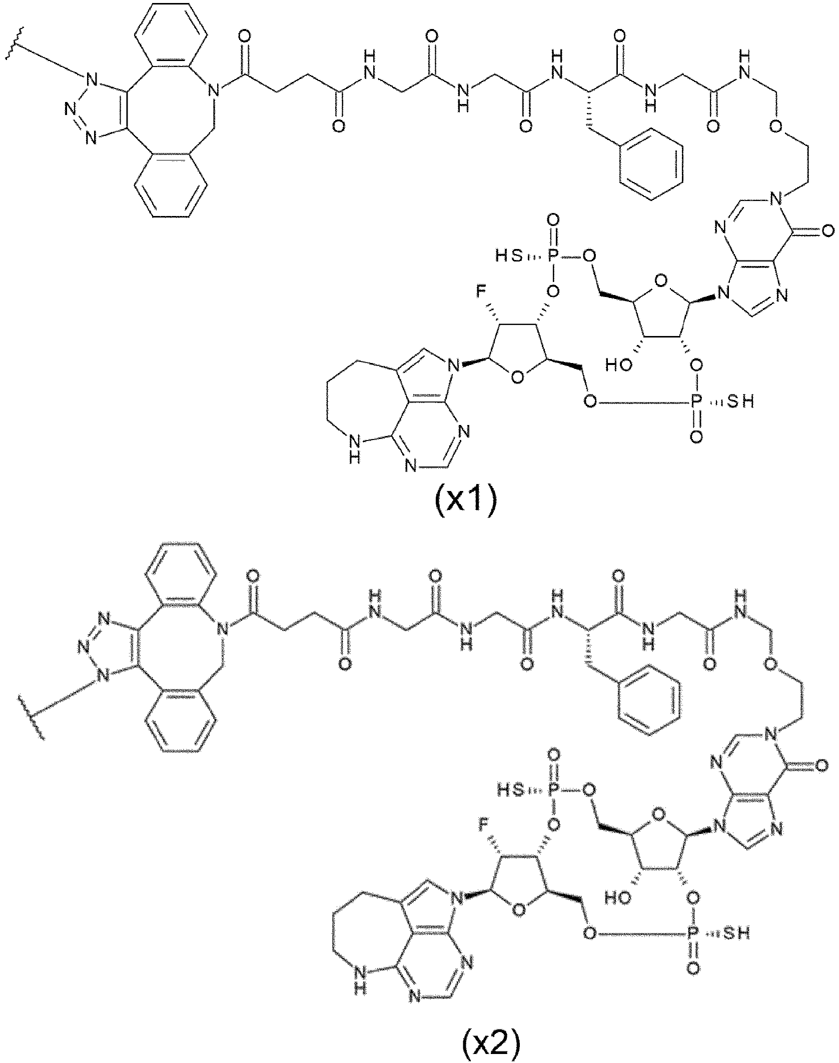 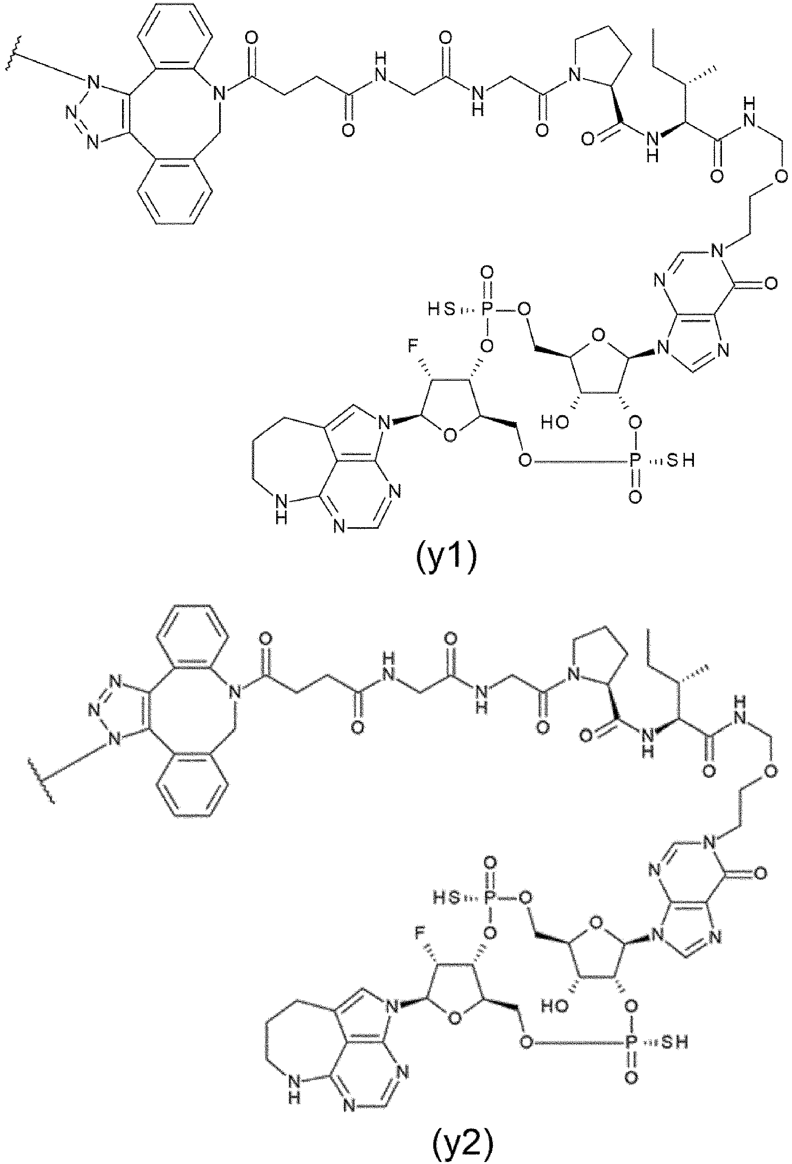 wherein in Formulas x1, x2, y1, and y2, the wavy line represents binding to the N297 glycan.

### 2. Production of Anti-HER2 Antibody-Drug Conjugate

### 2-1. Production of Antibody

The anti-HER2 antibody described in 1-1. can be obtained by methods known in the art (e.g., US 5821337 or WO 2001/00245).

In recent years, a method has been reported, in which heterogeneous glycans of an antibody are remodeled (modified) by enzymatic reaction to homogeneously introduce glycans having a functional group (ACS Chem. Biol. 2012, 7, 110-122; ACS Med. Chem. Lett. 2016, 7, 1005-1008). Attempts have also been made to synthesize a homogeneous ADC by site-specifically introducing a drug using this glycan remodeling technique (Bioconjugate Chem. 2015, 26, 2233-2242; Angew. Chem. Int. Ed. 2016, 55, 2361-2367; US 2016361436).

The antibody having the N297 glycan used in the present invention can be produced by using a glycan remodeling technique for the antibody described in 1-1., according to a method described, for example, in WO 2018/003983, WO 2020/050406, WO 2021/177438, WO 2022/050300, WO 2023/167238, or PLos ONE 2018, 13, e0193534.

### 2-2. Production of Drug and Linker

The drug and the linker used in the present invention can be produced with reference to the description of WO 2020/050406, WO 2021/177438, WO 2022/163846, and the like.

### 2-3. Production of Drug-Linker Intermediate (Conjugation Precursor)

The drug-linker intermediate (conjugation precursor) used in the production of the anti-HER2 antibody-drug conjugate used in the present invention is represented by a formula below:

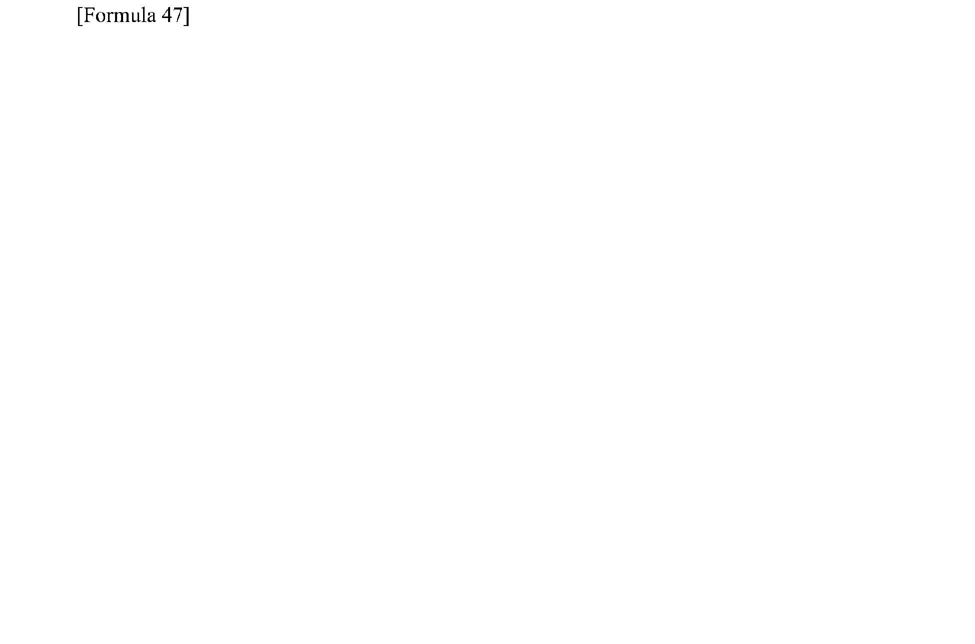

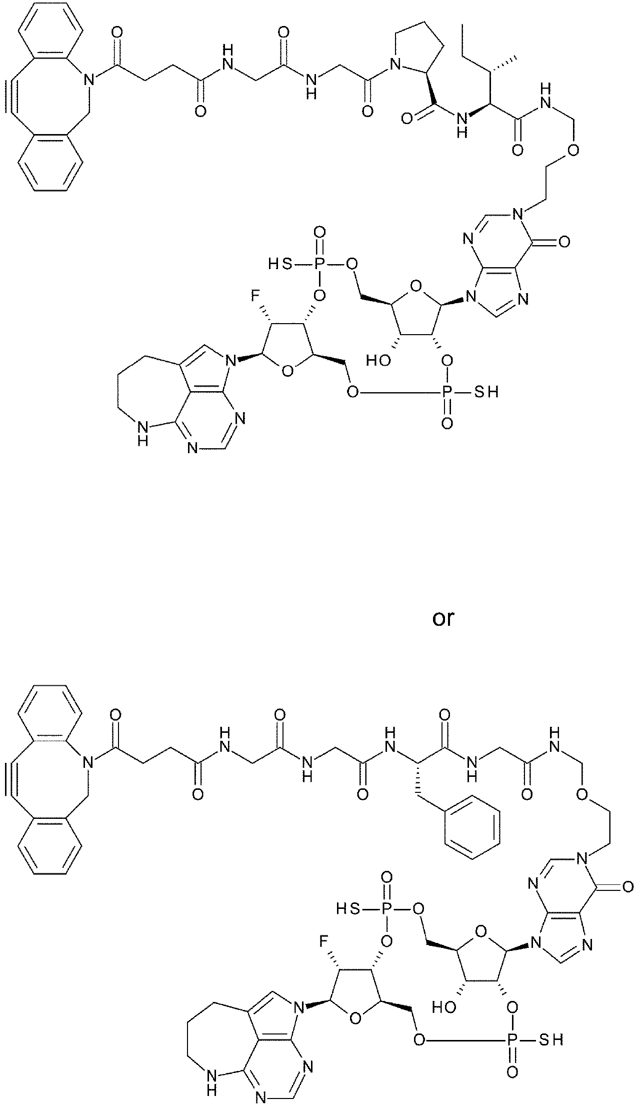

Preferably, a drug-linker intermediate (conjugation precursor) represented by a formula below is used.

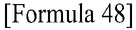

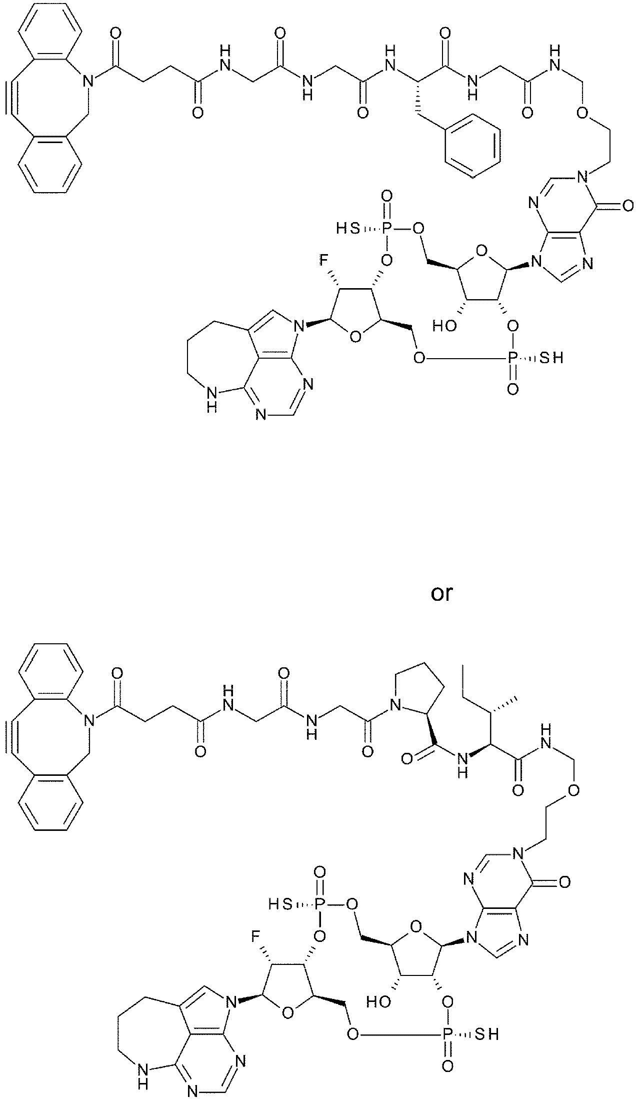

More preferably, a drug-linker intermediate (conjugation precursor) represented by a formula below is used.

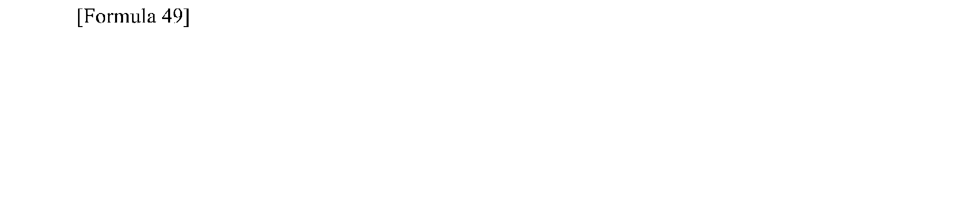

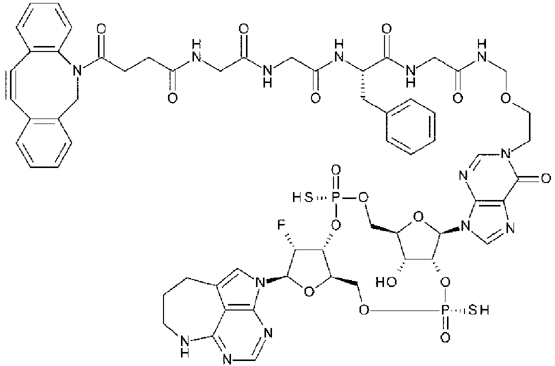

The drug-linker intermediate (conjugation precursor) described above can be produced with reference to the description of WO 2020/050406, WO 2021/177438, WO 2022/163846, and the like.

### 2-4. Production of Anti-HER2 Antibody-Drug Conjugate

The anti-HER2 antibody-drug conjugate used in the present invention can be produced by binding the drug-linker intermediate (conjugation precursor) described above and the antibody having the N297 glycan by cycloaddition reaction. Examples of the cycloaddition reaction include Diels-Alder reaction and 1,3-dipolar cycloaddition reaction, and 1,3-dipolar cycloaddition reaction is preferred. Examples of the 1,3-dipolar cycloaddition reaction include a cycloaddition reaction between azide and terminal alkyne, and strain-promoted azide-alkyne cycloaddition (SPAAC: J. Am. Chem. Soc. 2004, 126, 15046-15047) reaction; and SPAAC reaction is preferred.

The anti-HER2 antibody-drug conjugate described above can be produced with reference to the description of WO 2020/050406, WO 2021/177438, WO 2022/163846, and the like.

### 3. Immune Checkpoint Inhibitor

In the present invention, the term "immune checkpoint inhibitor" refers to an agent that inhibits the immunosuppressive system and activates tumor immunity.

The immune checkpoint inhibitor used in the present invention is not particularly limited, but examples preferably include anti-PD-1 antibodies, anti-PD-L1 antibodies, anti-CTLA-4 antibodies, anti-TIGIT antibodies, and anti-LAG-3 antibodies, and more preferably include anti-PD-1 antibodies, anti-PD-L1 antibodies, and anti-CTLA-4 antibodies.

In the present invention, the term "anti-PD-1 antibody" refers to an antibody that specifically binds to programmed cell death-1 (PD-1; CD279; PDCD1), and preferably refers to an antibody having an effect of reducing, inhibiting, and/or interfering with the signal transduction resulting from the interaction between PD-1 and its binding partner, PD-L1 and/or PD-L2.

The anti-PD-1 antibody used in the present invention is not particularly limited as long as its clinical efficacy and safety are confirmed, but preferred examples include nivolumab (WO 2006/121168 and the like), pembrolizumab (WO 2008/156712 and the like), dostarlimab, spartalizumab (WO 2015/112900 and the like), and cemiplimab (WO 2015/196051 and the like). In addition, for the purpose of confirming the effect of the combination with the anti-HER2 antibody-drug conjugate used in the present invention in pre-clinical studies, commercially available anti-PD-1 antibodies for research (e.g., clone RMP1-14, anti-mPD-1 mIgG1e3) or the like can also be used. These anti-PD-1 antibodies are commonly used as surrogate antibodies to inhibit PD-1 signaling in mice.

In the present invention, the term "anti-PD-L1 antibody" refers to an antibody that specifically binds to programmed cell death ligand 1 (PD-L1; CD274; B7-H1), and preferably refers to an antibody having an effect of reducing, inhibiting, and/or interfering with the signal transduction resulting from the interaction between PD-L1 and its binding partner, PD-1 and/or B7.1 (CD80). The anti-PD-L1 antibody used in the present invention is not particularly limited as long as its clinical efficacy and safety are confirmed, but preferred examples include atezolizumab (WO 2010/077634 and the like), durvalumab (WO 2011/066389 and the like), and avelumab (WO 2013/079174 and the like). In addition, for the purpose of confirming the effect of the combination with the anti-HER2 antibody-drug conjugate used in the present invention in pre-clinical studies, commercially available anti-PD-L1 antibodies for research (e.g., clone 10F.9G2) or the like can also be used. These anti-PD-L1 antibodies are commonly used as surrogate antibodies to inhibit PD-L1 signaling in mice.

In the present invention, the term "anti-CTLA-4 antibody" refers to an antibody that specifically binds to cytotoxic T-lymphocyte-associated protein 4 (CTLA-4; CD152), and preferably refers to an antibody having an effect of reducing, inhibiting, and/or interfering with the signal transduction resulting from the interaction between CTLA-4 and its binding partner, B7.1 (CD80) and/or B7.2 (CD86). The anti-CTLA-4 antibody used in the present invention is not particularly limited as long as its clinical efficacy and safety are confirmed, but preferred examples include ipilimumab (WO 2001/014424 and the like) and tremelimumab (WO 2000/037504 and the like). In addition, for the purpose of confirming the effect of the combination with the anti-HER2 antibody-drug conjugate used in the present invention in pre-clinical studies, commercially available anti-CTLA-4 antibodies for research (e.g., clone 9D9, 9H10) or the like can also be used. These anti-CTLA-4 antibodies are commonly used as surrogate antibodies to inhibit CTLA-4 signaling in mice.

In the present invention, the term "anti-TIGIT antibody" refers to an antibody that specifically binds to T cell immunoreceptor with Ig and ITIM domains (TIGIT), and preferably refers to an antibody having an effect of reducing, inhibiting, and/or interfering with the signal transduction resulting from the interaction between TIGIT and its binding partner, such as CD155. The anti-TIGIT antibody used in the present invention is not particularly limited as long as its clinical efficacy and safety are confirmed, but preferred examples include tiragolumab (WO 2017/053748) and vibostolimab (WO 2016/028656). In addition, for the purpose of confirming the effect of the combination with the antibody-drug conjugate used in the present invention in pre-clinical studies, commercially available anti-TIGIT antibodies for research (e.g., clone 1G9) or the like can also be used.

In the present invention, the term "anti-LAG-3 antibody" refers to an antibody that specifically binds to lymphocyte-activation gene 3 (LAG-3), and preferably refers to an antibody having an effect of reducing, inhibiting, and/or interfering with the signal transduction resulting from the interaction between LAG-3 and its binding partner, major histocompatibility complex (MHC) class II. The anti-LAG-3 antibody used in the present invention is not particularly limited as long as its clinical efficacy and safety are confirmed, but preferred examples include relatlimab. In addition, for the purpose of confirming the effect of the combination with the antibody-drug conjugate used in the present invention in pre-clinical studies, commercially available anti-LAG-3 antibodies (e.g., clone C9B7W) for research or the like can also be used.

In the present invention, the immune checkpoint inhibitor used in combination with the anti-HER2 antibody-drug conjugate may be an antigen-binding fragment of any of the above antibodies, or may be a compound containing the antibody or the antigen-binding fragment of the antibody. Here, the antigen-binding fragment means a fragment of an antibody having antigen-binding activity, and includes Fab, F(ab')2, Fv, scFv, diabody, linear antibodies, and multispecific antibodies formed from antibody fragments. However, the antigen-binding fragment is not limited to these molecules as long as it has antigen-binding activity.

### 4. Chemotherapeutic Agent

In the present invention, the term "chemotherapeutic agent" means a chemically synthesized agent among compounds having anticancer and anti-tumor activities. The chemotherapeutic agent used in the present invention is not particularly limited, but preferred examples include topoisomerase inhibitors, tubulin inhibitors, antimetabolites, platinum-based agents , DNA demethylating agents, anticancer antibiotics, and alkylating agents. The chemotherapeutic agent may be a pharmaceutically acceptable salt of any of these agents or the like.

Examples of the topoisomerase inhibitor include irinotecan, topotecan, etoposide, and sacituzumab govitecan; and preferably include irinotecan.

Examples of the tubulin inhibitor include paclitaxel, docetaxel, vincristine, vinblastine, vindesine, eribulin, vinorelbine, and albumin-suspended paclitaxel (nab-paclitaxel); and preferably include paclitaxel.

Examples of the antimetabolite include fluorouracil (also referred to as 5-FU), mercaptopurine, decitabine, gemcitabine (also referred to as GEM), azacitidine (also referred to as 5-Aza), methotrexate, tegafur, UFT, S-1, carmofur, doxifluridine, and capecitabine; and preferably include capecitabine.

Examples of the platinum-based agent include oxaliplatin, carboplatin, cisplatin, and nedaplatin; and preferably include cisplatin.

Examples of the DNA demethylating agent include azacitidine and decitabine.

Examples of the anticancer antibiotic include doxorubicin, bleomycin, and liposomal doxorubicin.

Examples of the alkylating agent include ifosfamide, cyclophosphamide, and dacarbazine; and preferably include cyclophosphamide.

However, the chemotherapeutic agent used in combination with the anti-HER2 antibody-drug conjugate of the present invention is not limited to these.

### 5. Anti-HER2 Antibody

In the present invention, examples of the anti-HER2 antibody used in combination with the anti-HER2 antibody-drug conjugate include trastuzumab, pertuzumab, and margetuximab; and preferably include trastuzumab. The anti-HER2 antibodies also include their biosimilars or biobetters.

### 6. Anti-HER2 Antibody-Containing Compound

In the present invention, examples of the anti-HER2 antibody-containing compound used in combination with the anti-HER2 antibody-drug conjugate include ado-trastuzumab emtansine (T-DM1), disitamab vedotin, and vic-trastuzumab duocarmazine; and preferably include T-DM1, provided that the anti-HER2 antibody-drug conjugate represented by Formula (I) and anti-HER2 antibody-deruxtecan are excluded. The anti-HER2 antibody-containing compounds also include their biosimilars or biobetters.

Examples of the anti-HER2 antibody in the anti-HER2 antibody-deruxtecan include trastuzumab, as well as fragments, variants, derivatives, and constructs of trastuzumab.

### 7. Molecular-Targeted Drug

In the present invention, the term "molecular-targeted drug" refers to an agent designed for the purpose of particularly attacking a gene or protein present in cancer cells or around cancer cells and involved in the growth of cancer cells, and its main targets are cell surface antigens, signal transduction molecules, ligands, and the like. The molecular-targeted drug used in the present invention is not particularly limited, but preferred examples include tyrosine kinase inhibitors, such as EGFR inhibitors, VEGF inhibitors, and FGFR inhibitors; and multikinase inhibitors. Examples of the EGFR inhibitor include gefitinib, erlotinib, afatinib, osimertinib, cetuximab, panitumumab, necitumumab, matuzumab, nimotuzumab, and zalutumumab. Preferred examples of the VEGF inhibitor include bevacizumab, ramucirumab, aflibercept beta, and axitinib. Preferred examples of the FGFR inhibitor include pemigatinib and futibatinib. Preferred examples of the multikinase inhibitor include sorafenib, sunitinib, pazopanib, regorafenib, and lenvatinib. In addition, examples of the tyrosine kinase inhibitor targeting HER2 include tucatinib, and examples of the tyrosine kinase inhibitor targeting HER2 and the like include neratinib and lapatinib.

### 8. Pharmaceutical Composition, Therapeutic Method, and the Like

Hereinafter, a pharmaceutical composition used in a combination therapy of the anti-HER2 antibody-drug conjugate according to the present invention with an immune checkpoint inhibitor, a chemotherapeutic agent, a molecular-targeted drug, an anti-HER2 antibody, or an anti-HER2 antibody-containing compound; and the combination therapy (therapeutic method) will be described.

In the present invention, "used in combination" means that a subject receiving administration takes a plurality of agents into the body in a certain period. In addition, a plurality of agents need not be present in the body at the same time and need only be taken into the body for a certain period (e.g., one month, preferably one week, more preferably several days, and even more preferably one day), and an additional active ingredient may be eliminated from the body at any time of administration.

In the present invention, the anti-HER2 antibody-drug conjugate and the immune checkpoint inhibitor, the chemotherapeutic agent, the molecular-targeted drug, the anti-HER2 antibody, or the anti-HER2 antibody-containing compound may each be included as an active ingredient in different formulations; in that case, the timing of administration and the number of administrations are not particularly limited, and, for example, they are administered simultaneously or at different times (sequentially or separately). When administered at different times, they may be administered continuously or non-continuously. When they are administered at different times, the administration interval and the administration order can be changed as appropriate. Furthermore, in the present invention, the anti-HER2 antibody-drug conjugate and the immune checkpoint inhibitor, the chemotherapeutic agent, the molecular-targeted drug, the anti-HER2 antibody, or the anti-HER2 antibody-containing compound may be included as active ingredients in a single formulation.

In addition, the present invention also includes an aspect in which the anti-HER2 antibody-drug conjugate is used in combination with two or more agents selected from an immune checkpoint inhibitor, a chemotherapeutic agent, a molecular-targeted drug, an anti-HER2 antibody, and an anti-HER2 antibody-containing compound. In this case, the two or more agents may be selected from agents belonging to the same category (e.g., two agents belonging to immune checkpoint inhibitors) or may be selected from agents belonging to different categories (e.g., an agent belonging to immune checkpoint inhibitors and an agent belonging to chemotherapeutic agents). For example, in one aspect of the present invention, the pharmaceutical composition containing the anti-HER2 antibody-drug conjugate can be used in combination with two or more agents selected from an immune checkpoint inhibitor, a chemotherapeutic agent, a molecular-targeted drug, an anti-HER2 antibody, and an anti-HER2 antibody-containing compound; in addition, the pharmaceutical composition containing an immune checkpoint inhibitor, a chemotherapeutic agent, a molecular-targeted drug, an anti-HER2 antibody, or an anti-HER2 antibody-containing compound can be used in combination with one or more additional agents selected from an immune checkpoint inhibitor, a chemotherapeutic agent, a molecular-targeted drug, an anti-HER2 antibody, and an anti-HER2 antibody-containing compound, in addition to the anti-HER2 antibody-drug conjugate. In another aspect of the present invention, the pharmaceutical composition containing the anti-HER2 antibody-drug conjugate further contains two or more agents selected from an immune checkpoint inhibitor, a chemotherapeutic agent, a molecular-targeted drug, an anti-HER2 antibody, and an anti-HER2 antibody-containing compound. In yet another aspect of the present invention, the pharmaceutical composition containing the anti-HER2 antibody-drug conjugate, and an immune checkpoint inhibitor, a chemotherapeutic agent, a molecular-targeted drug, an anti-HER2 antibody, or an anti-HER2 antibody-containing compound may be used in combination with one or more additional agents selected from an immune checkpoint inhibitor, a chemotherapeutic agent, a molecular-targeted drug, an anti-HER2 antibody, and an anti-HER2 antibody-containing compound.

The pharmaceutical composition and therapeutic method of the present invention can be used for the treatment of cancer, and preferably can be used for treating at least one cancer selected from the group consisting of lung cancer, renal cancer, urothelial carcinoma, colorectal cancer, prostate cancer, ovarian cancer, pancreatic cancer, breast cancer, melanoma, liver cancer, bladder cancer, gastric cancer, esophageal cancer, endometrial cancer, testicular cancer, cervical cancer, placental choriocarcinoma, glioblastoma multiforme, brain tumor, head and neck cancer, thyroid cancer, mesothelioma, gastrointestinal stromal tumor (GIST), gallbladder cancer, bile duct cancer, adrenal gland cancer, squamous cell carcinoma, leukemia, malignant lymphoma, plasmacytoma, myeloma, and sarcoma.

In another aspect, the pharmaceutical composition and therapeutic method of the present invention can be used for treating at least one cancer selected from the group consisting of lung cancer, colorectal cancer, prostate cancer, pancreatic cancer, ovarian cancer, breast cancer, bladder cancer, gastric cancer, esophageal cancer, endometrial cancer, cervical cancer, head and neck cancer, bile duct cancer, myeloma, and sarcoma.

In another aspect, the pharmaceutical composition and therapeutic method of the present invention can be used for treating lung cancer.

In another aspect, the pharmaceutical composition and therapeutic method of the present invention can be used for treating breast cancer.

In another aspect, the pharmaceutical composition and therapeutic method of the present invention can be used for treating gastric cancer.

In another aspect, the pharmaceutical composition and therapeutic method of the present invention can be used for treating colorectal cancer.

In another aspect, the pharmaceutical composition and therapeutic method of the present invention can be used for treating bladder cancer.

In one aspect, the present invention relates to a pharmaceutical composition containing the anti-HER2 antibody-drug conjugate, in which an immune checkpoint inhibitor, a chemotherapeutic agent, a molecular-targeted drug, an anti-HER2 antibody, or an anti-HER2 antibody-containing compound is used in combination.

In one aspect, the present invention relates to the anti-HER2 antibody-drug conjugate for use in treating cancer in combination with an immune checkpoint inhibitor, a chemotherapeutic agent, a molecular-targeted drug, an anti-HER2 antibody, or an anti-HER2 antibody-containing compound.

In one aspect, the present invention relates to use of the anti-HER2 antibody-drug conjugate in combination with an immune checkpoint inhibitor, a chemotherapeutic agent, a molecular-targeted drug, an anti-HER2 antibody, or an anti-HER2 antibody-containing compound for the manufacture of a medicament for treating cancer. In another aspect of the present invention, the present invention relates to use of an immune checkpoint inhibitor, a chemotherapeutic agent, a molecular-targeted drug, an anti-HER2 antibody, or an anti-HER2 antibody-containing compound in combination with the anti-HER2 antibody-drug conjugate for the manufacture of a medicament for treating cancer. In another aspect of the present invention, the present invention relates to use of the anti-HER2 antibody-drug conjugate, and an immune checkpoint inhibitor, a chemotherapeutic agent, a molecular-targeted drug, an anti-HER2 antibody, or an anti-HER2 antibody-containing compound in producing a pharmaceutical for combination cancer treatment; use of the anti-HER2 antibody-drug conjugate in combination with an immune checkpoint inhibitor, a chemotherapeutic agent, a molecular-targeted drug, an anti-HER2 antibody, or an anti-HER2 antibody-containing compound for the manufacture of a medicament for treating cancer; or use of an immune checkpoint inhibitor, a chemotherapeutic agent, a molecular-targeted drug, an anti-HER2 antibody, or an anti-HER2 antibody-containing compound in combination with the anti-HER2 antibody-drug conjugate for the manufacture of a medicament for treating cancer.

In one aspect, the present invention relates to a method of treating a disease, in which the anti-HER2 antibody-drug conjugate, and an immune checkpoint inhibitor, a chemotherapeutic agent, a molecular-targeted drug, an anti-HER2 antibody, or an anti-HER2 antibody-containing compound are administered in combination to a subject in need of treatment of the disease.

In one aspect, the present invention relates to a pharmaceutical composition containing the anti-HER2 antibody-drug conjugate, the pharmaceutical composition for use in treating cancer in combination with an immune checkpoint inhibitor, a chemotherapeutic agent, a molecular-targeted drug, an anti-HER2 antibody, or an anti-HER2 antibody-containing compound. In another aspect, the present invention relates to a pharmaceutical composition for use in treating cancer in combination with the anti-HER2 antibody-drug conjugate, the composition containing an immune checkpoint inhibitor, a chemotherapeutic agent, a molecular-targeted drug, an anti-HER2 antibody, or an anti-HER2 antibody-containing compound.

In one aspect, the present invention relates to a cancer therapeutic agent used in combination with an immune checkpoint inhibitor, a chemotherapeutic agent, a molecular-targeted drug, an anti-HER2 antibody, or an anti-HER2 antibody-containing compound, the therapeutic agent containing the anti-HER2 antibody-drug conjugate as an active ingredient and intended for a patient undergoing treatment with the immune checkpoint inhibitor, the chemotherapeutic agent, the molecular-targeted drug, the anti-HER2 antibody, or the anti-HER2 antibody-containing compound. In another aspect, the present invention relates to a cancer therapeutic agent used in combination with the anti-HER2 antibody-drug conjugate, the therapeutic agent intended for a patient undergoing treatment with the anti-HER2 antibody-drug conjugate and containing an immune checkpoint inhibitor, a chemotherapeutic agent, a molecular-targeted drug, an anti-HER2 antibody, or an anti-HER2 antibody-containing compound as an active ingredient.

In one aspect, the present invention relates to a cancer therapeutic agent for use in cancer treatment and containing the anti-HER2 antibody-drug conjugate as an active ingredient, in which the treatment includes administration of an immune checkpoint inhibitor, a chemotherapeutic agent, a molecular-targeted drug, an anti-HER2 antibody, or an anti-HER2 antibody-containing compound in addition to the anti-HER2 antibody-drug conjugate. In another aspect, the present invention relates to a cancer therapeutic agent for use in cancer treatment and containing an immune checkpoint inhibitor, a chemotherapeutic agent, a molecular-targeted drug, an anti-HER2 antibody, or an anti-HER2 antibody-containing compound as an active ingredient, in which the treatment includes administration of the anti-HER2 antibody-drug conjugate in addition to the immune checkpoint inhibitor, the chemotherapeutic agent, the molecular-targeted drug, the anti-HER2 antibody, or the anti-HER2 antibody-containing compound.

The pharmaceutical composition and therapeutic method of the present invention can be suitably used when the expression of HER2 is confirmed in cancer.

The expression of HER2 can be confirmed, for example, by detection of HER2 at the level of a gene product (protein) by immunohistochemistry (IHC), with a flow cytometer, by western blot method, or the like; or by detection at the level of gene transcription by in situ hybridization method (ISH) or quantitative PCR (q-PCR).

As used herein, the "subject" or "patient" refers to a human or other animal in need of the treatment, and in one aspect, a human in need of the treatment.

The anti-tumor effect of the pharmaceutical composition and therapeutic method of the present invention can be confirmed, for example, by preparing a model in which cancer cells are implanted into a test animal, and measuring the reduction in tumor volume and the life-extending effect by administering the pharmaceutical composition and therapeutic method of the present invention. Then, the combined effect of the anti-HER2 antibody-drug conjugate and the immune checkpoint inhibitor, chemotherapeutic agent, molecular-targeted drug, anti-HER2 antibody, or anti-HER2 antibody-containing compound used in the present invention can be confirmed by comparing it with the anti-tumor effect of single-agent administration of each of the anti-HER2 antibody-drug conjugate, immune checkpoint inhibitor, chemotherapeutic agent, molecular-targeted drug, anti-HER2 antibody, or anti-HER2 antibody-containing compound used in the present invention.

In addition, the anti-tumor effect of the pharmaceutical composition and therapeutic method of the present invention can be confirmed in clinical studies by Response Evaluation Criteria in Solid Tumors (RECIST), WHO evaluation method, Macdonald evaluation method, body weight measurement, and other methods, and can be determined by indices, such as complete response (CR), partial response (PR), progressive disease (PD), objective response rate (ORR), duration of response (DoR), progression-free survival (PFS), and overall survival (OS).

The methods described above can confirm the superiority of the anti-tumor effect of the pharmaceutical composition and therapeutic method of the present invention over existing pharmaceutical compositions and therapeutic methods for the treatment of cancer.

The pharmaceutical composition and therapeutic method of the present invention can inhibit the growth of cancer cells and furthermore eliminate cancer cells. These actions can achieve relief from cancer-related symptoms and improvement of QOL in cancer patients, attaining a therapeutic effect while prolonging the survival of cancer patients. Even if cancer cells are not eliminated, the inhibition or control of cancer cell growth can achieve higher QOL and longer survival in cancer patients.

The pharmaceutical composition of the present invention can contain one or more pharmaceutically compatible ingredients and be administered. The pharmaceutically compatible ingredient can be appropriately selected and used from pharmaceutical excipients and the like commonly used in this field according to the dose, dose concentration, and the like of the anti-HER2 antibody-drug conjugate, immune checkpoint inhibitor, chemotherapeutic agent, molecular-targeted drug, anti-HER2 antibody, or anti-HER2 antibody-containing compound used in the present invention.

The pharmaceutical composition and therapeutic method of the present invention may further contain a cancer therapeutic agent other than the anti-HER2 antibody-drug conjugate, immune checkpoint inhibitor, chemotherapeutic agent, molecular-targeted drug, anti-HER2 antibody, or anti-HER2 antibody-containing compound according to the present invention. The pharmaceutical composition of the present invention can also be administered in combination with an additional cancer therapeutic agent, and this can enhance the anti-tumor effect. The additional cancer therapeutic agent used for such a purpose may be administered simultaneously with the pharmaceutical composition of the present invention, or at different times (sequentially or separately). When administered at different times, it may be administered continuously or non-continuously. When it is administered at different times, the administration interval and the administration order can be changed as appropriate. Such a cancer therapeutic agent is not limited as long as it has anti-tumor activity, but examples thereof include at least one selected from the group consisting of endocrine therapeutic agents; immunotherapeutic agents, such as interferon formulations or BCG; and antibodies other than anti-HER2 antibodies or antigen-binding fragments of the antibodies. Here, the antigen-binding fragment means a fragment of an antibody having antigen-binding activity, and includes Fab, F(ab')2, Fv, scFv, diabody, linear antibodies, and multispecific antibodies formed from antibody fragments. However, the antigen-binding fragment is not limited to these molecules as long as it has antigen-binding activity.

The pharmaceutical composition and therapeutic method of the present invention can also be used in combination with radiotherapy and/or cellular immunotherapy. For example, a cancer patient receives radiotherapy and/or cellular immunotherapy before and/or after, or simultaneously with receiving treatment with the pharmaceutical composition of the present invention.

The pharmaceutical composition and therapeutic method of the present invention can also be used as adjuvant chemotherapy in combination with surgery. The pharmaceutical composition of the present invention may be administered for the purpose of reducing the size of tumors before surgery (referred to as preoperative adjuvant chemotherapy or neoadjuvant therapy), or may be administered for the purpose of preventing recurrence of tumors after surgery (referred to as postoperative adjuvant chemotherapy or adjuvant therapy). The pharmaceutical composition and therapeutic method of the present invention can also be used as maintenance therapy.

In addition to the therapeutic use as described above, the pharmaceutical composition and therapeutic method of the present invention can be expected to have a preventive effect of inhibiting the growth of microscopic metastatic cancer cells and furthermore eliminating the cells. For example, the pharmaceutical composition and therapeutic method of the present invention can be expected to have an effect of inhibiting the growth of cancer cells in body fluid and eliminating the cancer cells in a metastasis process, and an effect of inhibiting the growth of microscopic cancer cells immediately after implantation in any tissue, eliminating the microscopic cancer cells, and the like. Thus, the pharmaceutical composition and therapeutic method of the present invention can be expected to have an effect of inhibiting and preventing cancer metastasis, particularly after surgical removal of cancer.

The pharmaceutical composition of the present invention can be administered as a pharmaceutical composition containing one or more pharmaceutically compatible ingredients. The substance used in the pharmaceutical composition of the present invention can be appropriately selected and used from pharmaceutical excipients and the like commonly used in this field in terms of the dose and dose concentration. For example, the pharmaceutical composition typically contains one or more pharmaceutical carriers (e.g., a sterilized liquid). Here, examples of the liquid include water and oils (petroleum, oils of animal, plant, or synthetic origin). The oil may be, for example, peanut oil, soybean oil, mineral oil, or sesame oil. Water is a more typical carrier when the pharmaceutical composition is administered intravenously. An saline, an aqueous dextrose solution, and an aqueous glycerol solution can also be used as liquid carriers, particularly for injectable solutions. An appropriate pharmaceutical filler can be appropriately selected from those known in this field. The pharmaceutical composition, if desired, can also contain a minor amount of a wetting agent or an emulsifier, or a pH buffering agent. Examples of the suitable pharmaceutical carrier are described in "Remington's Pharmaceutical Sciences" by E. W. Martin. The formulation corresponds to the mode of administration.

Various delivery systems are known and can be used to administer the pharmaceutical composition of the present invention. Examples of the route of introduction include, but are not limited to, intradermal, intramuscular, intraperitoneal, intravenous, and subcutaneous routes. Administration can be, for example, by infusion or bolus injection. In one preferred embodiment, the administration of the antibody-drug conjugate is by infusion. Parenteral administration is a preferred route of administration.

When the anti-HER2 antibody-drug conjugate used in the present invention is administered to a human, the dose and interval of the administration are not limited, and for example, about 0.001 to 100 mg/kg can be administered once or a plurality of times at an interval of once every 1 to 180 days.

The immune checkpoint inhibitor, chemotherapeutic agent, molecular-targeted drug, anti-HER2 antibody, or anti-HER2 antibody-containing compound used in the present invention can be administered at a dose of 0.1 mg to 3000 mg per administration, and can be administered preferably at a dose of 50 mg to 2000 mg per administration.

The immune checkpoint inhibitor, anti-HER2 antibody, or anti-HER2 antibody-containing compound according to the present invention can be administered to a human at an interval of 1 to 4 times per 1 to 56 days, and can be administered preferably at an interval of once every 7 days, once every 14 days, once every 21 days, once every 28 days, or once every 42 days. The chemotherapeutic agent or the molecular-targeted drug can be administered to a human at an interval of 1 to 3 times a day or 1 to 4 times per 1 to 56 days, and can be administered preferably at an interval of once a day, twice a day, or once every 7 days, once every 14 days, once every 21 days, once every 28 days, or once every 42 days.

### Examples

Hereinafter, the present invention will be described in detail with reference to examples, but the present invention is not limited to these. In addition, these are not to be construed as limiting in any sense.

### Production Example 1: Preparation of Anti-HER2 Antibody-Drug Conjugate

In accordance with the production method described in WO 2020/050406, an anti-HER2 antibody-drug conjugate represented by the formula below was produced using an anti-HER2 antibody (referred to as "anti-HER2 antibody 1" in the present invention) comprising a heavy chain consisting of the amino acid sequence represented by SEQ ID NO: 1 (FIG. 1) and a light chain consisting of the amino acid sequence represented by SEQ ID NO: 2 (FIG. 2): 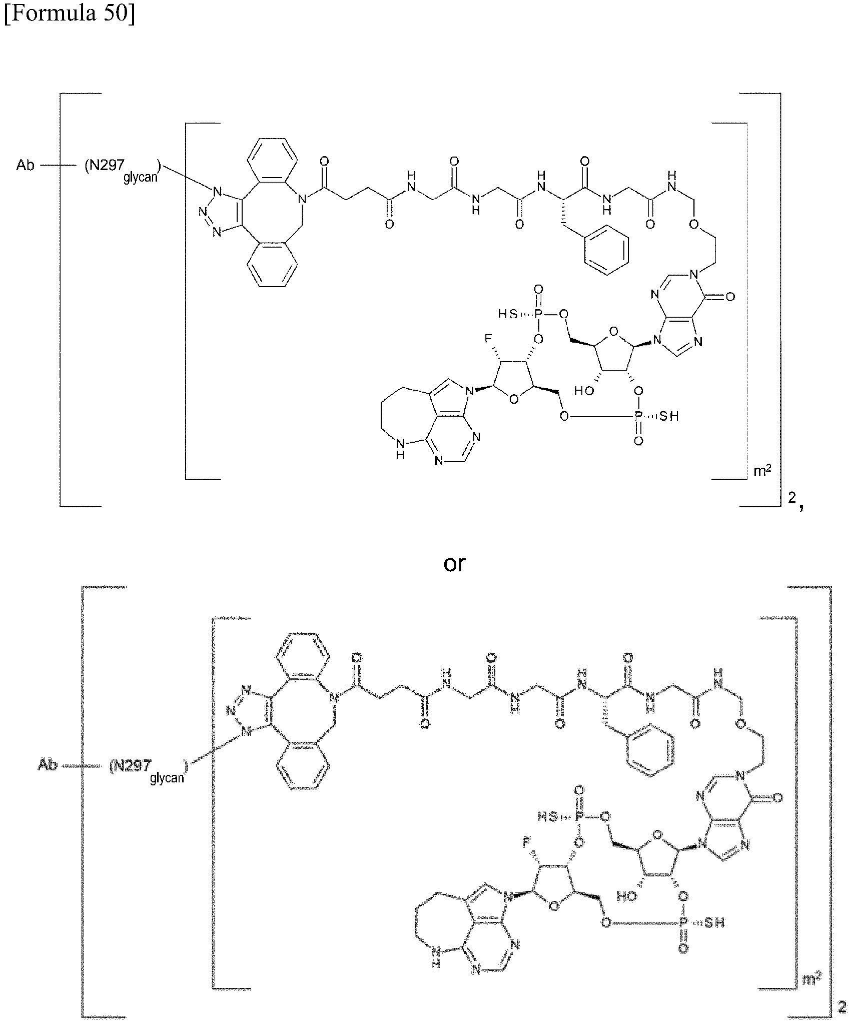 wherein the N297 glycan is [N297-(Fuc)SG]. The anti-HER2 antibody-drug conjugate with m² = 2 is referred to as "HER2-ADC (1)", and the anti-HER2 antibody-drug conjugate with m² = 1 is referred to as "HER2-ADC (2)". Similarly, an anti-HER2 antibody-drug conjugate with m² = 1 ("HER2-ADC (3)") was produced using an anti-HER2 antibody (referred to as "anti-HER2 antibody 2" in the present invention) comprising a heavy chain consisting of the amino acid sequence represented by SEQ ID NO: 8 (FIG. 4) and a light chain consisting of the amino acid sequence represented by SEQ ID NO: 9 (FIG. 5).

### Anti-tumor Test

Measurement and equation: In all studies, the major and minor axes of tumors were measured twice a week with an electronic digital caliper (CD-15CX, Mitutoyo Corp.), and tumor volume (mm³) was calculated. The equation is as shown below: $Tumor volume \left({mm}^{3}\right) = 1 / 2 \times major axis \left(mm\right) \times {\left[minor axis \left(mm\right)\right]}^{2}$

In the test examples below, an individual with a tumor volume of 50 mm³ or less is referred to as a "CR individual".

### Test Example 1: Anti-tumor Test (1)

CT26.WT-HER2 cells were prepared by introducing a human HER2 gene into the mouse colorectal cancer cell line CT26.WT purchased from the American Type Culture Collection.

The CT26.WT-HER2 cells were implanted subcutaneously into the right axillary region of BALB/c mice (Day 0), and after 7 days, the mice were randomly divided into groups. HER2-ADC (1) was administered intravenously via the tail vein at a dose of 0.4 mg/kg on Day 7, once in total. Trastuzumab was administered intravenously via the tail vein at a dose of 15 mg/kg on Day 7 and Day 10, twice in total. The number of mice in each group was 10.

The results are shown in FIG. 10. In the graph, the line with black circles represents a vehicle group, the line with black squares represents a HER2-ADC (1) administration group, the line with black triangles represents a trastuzumab administration group, and the line with black inverted triangles represents a combination administration group of HER2-ADC (1) and trastuzumab. The vertical axis represents tumor volume (mm³), and the horizontal axis represents days after tumor implantation. Tumor growth progressed in the vehicle group and the trastuzumab administration group. Tumor growth was inhibited in the HER2-ADC (1) administration group; the number of CR individuals was 3 out of 10 on Day 22. In contrast, tumor growth was significantly inhibited in the combination administration group of HER2-ADC (1) and trastuzumab; the number of CR individuals was 10 out of 10 on Day 22. The above results confirmed a strong combined anti-tumor effect of HER2-ADC (1) and trastuzumab.

### Test Example 2: Anti-tumor Test (2)

The human breast cancer cell line JIMT-1 purchased from DSMZ was implanted subcutaneously into the right axillary region of BALB/c-nu mice (Day 0), and after 11 days, the mice were randomly divided into groups. HER2-ADC (1) was administered intravenously via the tail vein at a dose of 0.04 mg/kg on Day 11, once in total. Trastuzumab was administered intravenously via the tail vein at a dose of 10 mg/kg on Day 11, Day 18, and Day 25, three times in total. The number of mice in each group was 5.

The results are shown in FIG. 11. In the graph, the line with black circles represents a vehicle group, the line with black squares represents a HER2-ADC (1) administration group, the line with black triangles represents a trastuzumab administration group, and the line with black inverted triangles represents a combination administration group of HER2-ADC (1) and trastuzumab. The vertical axis represents tumor volume (mm³), and the horizontal axis represents days after tumor implantation. Tumor growth progressed in the vehicle group. On Day 45, the tumor volume was 20% smaller in the trastuzumab administration group than in the vehicle administration group, and the tumor volume was almost unchanged in the HER2-ADC (1) administration group. In contrast, tumor growth was significantly inhibited in the combination administration group of HER2-ADC (1) and trastuzumab; the tumor volume on Day 45 was 63% smaller than in the vehicle administration group.

The above results confirmed a strong combined anti-tumor effect of HER2-ADC (1) and trastuzumab.

### Test Example 3: Anti-tumor Test (3)

The human gastric cancer cell line NCI-N87 purchased from the American Type Culture Collection was implanted subcutaneously into the right axillary region of BALB/c-nu mice (Day 0), and after 7 days, the mice were randomly divided into groups. HER2-ADC (1) was administered intravenously via the tail vein at a dose of 0.4 mg/kg on Day 7, once in total. Trastuzumab was administered intravenously via the tail vein at a dose of 10 mg/kg on Day 7, Day 14, and Day 21, three times in total. The number of mice in each group was 6.

The results are shown in FIG. 12. In the graph, the line with black circles represents a vehicle group, the line with black squares represents a HER2-ADC (1) administration group, the line with black triangles represents a trastuzumab administration group, and the line with black inverted triangles represents a combination administration group of HER2-ADC (1) and trastuzumab. The vertical axis represents tumor volume (mm³), and the horizontal axis represents days after tumor implantation. Tumor growth progressed in the vehicle group. Tumor growth was inhibited in the HER2-ADC (1) administration group and the trastuzumab administration group; however, the number of CR individuals was 0 out of 6 on Day 27 in both groups. In contrast, tumor growth was significantly inhibited in the combination administration group of HER2-ADC (1) and trastuzumab; the number of CR individuals was 6 out of 6 on Day 27.

The above results confirmed a strong combined anti-tumor effect of HER2-ADC (1) and trastuzumab.

### Test Example 4: Anti-tumor Test (4)

The human breast cancer cell line KPL-4 provided by Kawasaki Medical School was implanted subcutaneously into the right axillary region of BALB/c-nu mice (Day 0), and after 14 days, the mice were randomly divided into groups. HER2-ADC (3) was administered intravenously via the tail vein at a dose of 0.1 mg/kg on Day 14, Day 21, and Day 28, three times in total. T-DM1 was administered intravenously via the tail vein at a dose of 3 mg/kg on Day 14, Day 21, and Day 28, three times in total. The number of mice in each group was 6.

The results are shown in FIG. 13. In the graph, the line with black circles represents a vehicle group, the line with black squares represents a HER2-ADC (3) administration group, the line with black triangles represents a T-DM1 administration group, and the line with black inverted triangles represents a combination administration group of HER2-ADC (3) and T-DM1. The vertical axis represents tumor volume (mm³), and the horizontal axis represents days after tumor implantation. Tumor growth progressed in the vehicle group. Tumor growth was inhibited in the HER2-ADC (3) administration group and the T-DM1 administration group; however, the number of CR individuals was 0 out of 6 on Day 42. In contrast, tumor growth was significantly inhibited in the combination administration group of HER2-ADC (3) and T-DM1; the number of CR individuals was 4 out of 6 on Day 42.

The above results confirmed a strong combined anti-tumor effect of HER2-ADC (3) and T-DM1.

### Test Example 5: Anti-tumor Test (5)

The human gastric cancer cell line NCI-N87 purchased from the American Type Culture Collection was implanted subcutaneously into the right axillary region of BALB/c-nu mice (Day 0), and after 7 days, the mice were randomly divided into groups. HER2-ADC (1) was administered intravenously via the tail vein at a dose of 0.4 mg/kg on Day 7, once in total. T-DM1 was administered intravenously via the tail vein at a dose of 3 mg/kg on Day 7, Day 14, and Day 21, three times in total. The number of mice in each group was 6.

The results are shown in FIG. 14. In the graph, the line with black circles represents a vehicle group, the line with black squares represents a HER2-ADC (1) administration group, the line with black triangles represents a T-DM1 administration group, and the line with black inverted triangles represents a combination administration group of HER2-ADC (1) and T-DM1. The vertical axis represents tumor volume (mm³), and the horizontal axis represents days after tumor implantation. Tumor growth progressed in the vehicle group. Tumor growth was inhibited in the HER2-ADC (1) administration group and the T-DM1 administration group; however, the number of CR individuals was 0 out of 6 on Day 27 in both groups. In contrast, tumor growth was significantly inhibited in the combination administration group of HER2-ADC (1) and T-DM1; the number of CR individuals was 6 out of 6 on Day 27.

The above results confirmed a strong combined anti-tumor effect of HER2-ADC (1) and T-DM1.

### Test Example 6: Anti-tumor Test (6)

LL/2-HER2 cells were prepared by introducing a human HER2 gene into the mouse lung cancer cell line LL/2 purchased from the American Type Culture Collection.

The LL/2-HER2 cells were implanted subcutaneously into the right axillary region of C57BL/6 mice (Day 0), and after 10 days, the mice were randomly divided into groups. HER2-ADC (1) was administered intravenously via the tail vein at a dose of 0.4 mg/kg on Day 10, once in total. An anti-PD-1 surrogate antibody (Anti-mPD-1 mIgG1e3, InvivoGen) was administered intravenously via the tail vein at a dose of 5 mg/kg on Day 10 and Day 14, twice in total. The number of mice in each group was 6.

The results are shown in FIG. 15. In the graph, the line with black circles represents a vehicle group, the line with black squares represents a HER2-ADC (1) administration group, the line with black triangles represents an anti-PD-1 surrogate antibody administration group, and the line with black inverted triangles represents a combination administration group of HER2-ADC (1) and the anti-PD-1 surrogate antibody. The vertical axis represents tumor volume (mm³), and the horizontal axis represents days after tumor implantation. Tumor growth progressed in the vehicle group and the anti-PD-1 surrogate antibody administration group, and individuals with a tumor volume exceeding 2500 mm³ were observed on Day 17. Thus, the measurement was terminated from the viewpoint of the humane endpoint. Tumor growth was inhibited in the HER2-ADC (1) administration group; however, the number of CR individuals was 0 out of 6 on Day 22. In contrast, tumor growth was significantly inhibited in the combination administration group of HER2-ADC (1) and the anti-PD-1 surrogate antibody; the number of CR individuals was 2 out of 6 on Day 22.

The above results confirmed a strong combined anti-tumor effect of HER2-ADC (1) and the anti-PD-1 surrogate antibody.

### Test Example 7: Anti-tumor Test (7)

CT26.WT-chimera HER2 cells were prepared by introducing a human-mouse chimera HER2 gene, in which the epitope site of the anti-HER2 antibody was replaced with a human type, into the mouse colorectal cancer cell line CT26.WT purchased from the American Type Culture Collection.

The CT26.WT-chimera HER2 cells were implanted subcutaneously into the right axillary region of BALB/c mice (Day 0), and after 6 days, the mice were randomly divided into groups. HER2-ADC (1) was administered intravenously via the tail vein at a dose of 0.4 mg/kg on Day 6, once in total. An anti-PD-1 surrogate antibody (Anti-mPD-1 mIgG1e3, InvivoGen) was administered intravenously via the tail vein at a dose of 5 mg/kg on Day 6, Day 9, and Day 13, three times in total. The number of mice in each group was 7.

The results are shown in FIG. 16. In the graph, the line with black circles represents a vehicle group, the line with black squares represents a HER2-ADC (1) administration group, the line with black triangles represents an anti-PD-1 surrogate antibody administration group, and the line with black inverted triangles represents a combination administration group of HER2-ADC (1) and the anti-PD-1 surrogate antibody. The vertical axis represents tumor volume (mm³), and the horizontal axis represents days after tumor implantation. Tumor growth progressed in the vehicle group and the anti-PD-1 surrogate antibody administration group, and individuals with a tumor volume exceeding 2500 mm³ were observed on Day 13. Thus, the measurement was terminated from the viewpoint of the humane endpoint. Tumor growth was inhibited in the HER2-ADC (1) administration group; however, the number of CR individuals was 0 out of 7 on Day 17. In contrast, tumor growth was significantly inhibited in the combination administration group of HER2-ADC (1) and the anti-PD-1 surrogate antibody; the number of CR individuals was 3 out of 7 on Day 17.

The above results confirmed a strong combined anti-tumor effect of HER2-ADC (1) and the anti-PD-1 surrogate antibody.

### Test Example 8: Anti-tumor Test (8)

The CT26.WT-HER2 cells prepared in Test Example 1 were implanted subcutaneously into the right axillary region of BALB/c mice (Day 0), and after 8 days, the mice were randomly divided into groups. HER2-ADC (1) was administered intravenously via the tail vein at a dose of 0.3 mg/kg on Day 8, once in total. An anti-PD-L1 surrogate antibody (clone number 10F.9G2, Bio X Cell) was administered intravenously via the tail vein at a dose of 10 mg/kg on Day 8, once in total. The number of mice in each group was 7.

The results are shown in FIG. 17. In the graph, the line with black circles represents a vehicle group, the line with black squares represents a HER2-ADC (1) administration group, the line with black triangles represents an anti-PD-L1 surrogate antibody administration group, and the line with black inverted triangles represents a combination administration group of HER2-ADC (1) and the anti-PD-L1 surrogate antibody. The vertical axis represents tumor volume (mm³), and the horizontal axis represents days after tumor implantation. Tumor growth progressed in the vehicle group and the anti-PD-L1 surrogate antibody administration group. Tumor growth was inhibited in the HER2-ADC (1) administration group; however, the number of CR individuals was 0 out of 7 on Day 25. In contrast, tumor growth was significantly inhibited in the combination administration group of HER2-ADC (1) and the anti-PD-L1 surrogate antibody; the number of CR individuals was 3 out of 7 on Day 25.

The above results confirmed a strong combined anti-tumor effect of HER2-ADC (1) and the anti-PD-L1 surrogate antibody.

### Test Example 9: Life Extension Test (1)

The CT26.WT-chimera HER2 cells prepared in Test Example 7 were implanted subcutaneously into the right axillary region of BALB/c mice (Day 0), and after 7 days, the mice were randomly divided into groups. HER2-ADC (1) was administered intravenously via the tail vein at a dose of 0.4 mg/kg on Day 7, once in total. An anti-CTLA-4 surrogate antibody (clone number 9D9, Bio X Cell) was administered intravenously via the tail vein at a dose of 5 mg/kg on Day 7, Day 10, Day 14, and Day 17, four times in total. The number of mice in each group was 6.

The results are shown in FIG. 18. In the graph, the line with black circles represents a vehicle group, the line with black squares represents a HER2-ADC (1) administration group, the line with black triangles represents an anti-CTLA-4 surrogate antibody administration group, and the line with black inverted triangles represents a combination administration group of HER2-ADC (1) and the anti-CTLA-4 surrogate antibody. The vertical axis represents a survival rate (%), and the horizontal axis represents days after tumor implantation. For individuals with a tumor volume exceeding 2500 mm³, the measurement was terminated from the viewpoint of the humane endpoint, that is, they were considered dead. In the vehicle group and the anti-CTLA-4 surrogate antibody administration group, tumor growth progressed, dead individuals were observed from Day 14, and the measurement was terminated for all individuals on Day 17. In the HER2-ADC (1) administration group, tumor growth was inhibited, dead individuals were observed from Day 17, and the measurement was terminated for all individuals on Day 26. In contrast, in the combination administration group of HER2-ADC (1) and the anti-CTLA-4 surrogate antibody, tumor growth was inhibited, dead individuals were observed from Day 22, and the measurement was terminated for all individuals on Day 33. That is, the period until the first dead individual was observed was not extended in the anti-CTLA-4 surrogate antibody administration group compared with the vehicle group, extended by 3 days in the HER2-ADC (1) administration group, and extended by 8 days in the combination administration group of HER2-ADC (1) and the anti-CTLA-4 surrogate antibody.

The above results confirmed a strong combined anti-tumor effect of HER2-ADC (1) and the anti-CTLA-4 surrogate antibody.

### Test Example 10: Anti-tumor Test (9)

The human breast cancer cell line KPL-4 provided by Kawasaki Medical School was implanted subcutaneously into the right axillary region of BALB/c-nu mice (Day 0), and after 14 days, the mice were randomly divided into groups. HER2-ADC (2) was administered intravenously via the tail vein at a dose of 0.3 mg/kg on Day 14 and Day 21, twice in total. Paclitaxel was administered intravenously via the tail vein at a dose of 15 mg/kg on Day 14 and Day 21, twice in total. The number of mice in each group was 6.

The results are shown in FIG. 19. In the graph, the line with black circles represents a vehicle group, the line with black squares represents a HER2-ADC (2) administration group, the line with black triangles represents a paclitaxel administration group, and the line with black inverted triangles represents a combination administration group of HER2-ADC (2) and paclitaxel. The vertical axis represents tumor volume (mm³), and the horizontal axis represents days after tumor implantation. Tumor growth progressed in the vehicle group. Tumor growth was inhibited in the HER2-ADC (2) administration group and the paclitaxel administration group. On Day 42, the number of CR individuals was 1 out of 6 in the HER2-ADC (2) administration group, and 0 out of 6 in the paclitaxel administration group. In contrast, tumor growth was significantly inhibited in the combination administration group of HER2-ADC (2) and paclitaxel; the number of CR individuals was 6 out of 6 on Day 42.

The above results confirmed a strong combined anti-tumor effect of HER2-ADC (2) and paclitaxel.

### Test Example 11: Anti-tumor Test (10)

The human breast cancer cell line KPL-4 provided by Kawasaki Medical School was implanted subcutaneously into the right axillary region of BALB/c-nu mice (Day 0), and after 14 days, the mice were randomly divided into groups. HER2-ADC (2) was administered intravenously via the tail vein at a dose of 0.3 mg/kg on Day 14 and Day 21, twice in total. Capecitabine was orally administered at a dose of 540 mg/kg every day from Day 14 to Day 18 and every day from Day 21 to Day 25, 10 times in total. The number of mice in each group was 6.

The results are shown in FIG. 20. In the graph, the line with black circles represents a vehicle group, the line with black squares represents a HER2-ADC (2) administration group, the line with black triangles represents a capecitabine administration group, and the line with black inverted triangles represents a combination administration group of HER2-ADC (2) and capecitabine. The vertical axis represents tumor volume (mm³), and the horizontal axis represents days after tumor implantation. Tumor growth progressed in the vehicle group. Tumor growth was inhibited in the HER2-ADC (2) administration group and the capecitabine administration group. On Day 42, the number of CR individuals was 1 out of 6 in the HER2-ADC (2) administration group, and 0 out of 6 in the capecitabine administration group. In contrast, tumor growth was significantly inhibited in the combination administration group of HER2-ADC (2) and capecitabine; the number of CR individuals was 3 out of 6 on Day 42.

The above results confirmed a strong combined anti-tumor effect of HER2-ADC (2) and capecitabine.

### Test Example 12: Anti-tumor Test (11)

The human gastric cancer cell line NCI-N87 purchased from the American Type Culture Collection was implanted subcutaneously into the right axillary region of BALB/c-nu mice (Day 0), and after 7 days, the mice were randomly divided into groups. HER2-ADC (1) was administered intravenously via the tail vein at a dose of 0.4 mg/kg on Day 7, once in total. Capecitabine was orally administered at a dose of 360 mg/kg every day from Day 7 to Day 11 and every day from Day 14 to Day 18, 10 times in total. The number of mice in each group was 6.

The results are shown in FIG. 21. In the graph, the line with black circles represents a vehicle group, the line with black squares represents a HER2-ADC (1) administration group, the line with black triangles represents a capecitabine administration group, and the line with black inverted triangles represents a combination administration group of HER2-ADC (1) and capecitabine. The vertical axis represents tumor volume (mm³), and the horizontal axis represents days after tumor implantation. Tumor growth progressed in the vehicle group. Tumor growth was inhibited in the HER2-ADC (1) administration group and the capecitabine administration group. On Day 42, the number of CR individuals was 0 out of 6 in the HER2-ADC (1) administration group, and 1 out of 6 in the capecitabine administration group. In contrast, tumor growth was significantly inhibited in the combination administration group of HER2-ADC (1) and capecitabine; the number of CR individuals was 6 out of 6 on Day 42.

The above results confirmed a strong combined anti-tumor effect of HER2-ADC (1) and capecitabine.

### Test Example 13: Anti-tumor Test (12)

The human gastric cancer cell line NCI-N87 purchased from the American Type Culture Collection was implanted subcutaneously into the right axillary region of BALB/c-nu mice (Day 0), and after 7 days, the mice were randomly divided into groups. HER2-ADC (1) was administered intravenously via the tail vein at a dose of 0.4 mg/kg on Day 7, once in total. Paclitaxel was administered intravenously via the tail vein at a dose of 15 mg/kg on Day 7, Day 14, and Day 21, three times in total. The number of mice in each group was 6.

The results are shown in FIG. 22. In the graph, the line with black circles represents a vehicle group, the line with black squares represents a HER2-ADC (1) administration group, the line with black triangles represents a paclitaxel administration group, and the line with black inverted triangles represents a combination administration group of HER2-ADC (1) and paclitaxel. The vertical axis represents tumor volume (mm³), and the horizontal axis represents days after tumor implantation. Tumor growth progressed in the vehicle group. Tumor growth was inhibited in the HER2-ADC (1) administration group and the paclitaxel administration group. On Day 42, the number of CR individuals was 0 out of 6 in both the HER2-ADC (1) administration group and the paclitaxel administration group. In contrast, tumor growth was significantly inhibited in the combination administration group of HER2-ADC (1) and paclitaxel; the number of CR individuals was 5 out of 6 on Day 42.

The above results confirmed a strong combined anti-tumor effect of HER2-ADC (1) and paclitaxel.

### Test Example 14: Anti-tumor Test (13)

The human gastric cancer cell line NCI-N87 purchased from the American Type Culture Collection was implanted subcutaneously into the right axillary region of BALB/c-nu mice (Day 0), and after 7 days, the mice were randomly divided into groups. HER2-ADC (1) was administered intravenously via the tail vein at a dose of 0.4 mg/kg on Day 7, once in total. Irinotecan was administered intravenously via the tail vein at a dose of 60 mg/kg on Day 7, Day 14, and Day 21, three times in total. The number of mice in each group was 6.

The results are shown in FIG. 23. In the graph, the line with black circles represents a vehicle group, the line with black squares represents a HER2-ADC (1) administration group, the line with black triangles represents an irinotecan administration group, and the line with black inverted triangles represents a combination administration group of HER2-ADC (1) and irinotecan. The vertical axis represents tumor volume (mm³), and the horizontal axis represents days after tumor implantation. Tumor growth progressed in the vehicle group. Tumor growth was inhibited in the HER2-ADC (1) administration group and the irinotecan administration group. On Day 42, the number of CR individuals was 0 out of 6 in both the HER2-ADC (1) administration group and the irinotecan administration group. In contrast, tumor growth was significantly inhibited in the combination administration group of HER2-ADC (1) and irinotecan; the number of CR individuals was 2 out of 6 on Day 42.

The above results confirmed a strong combined anti-tumor effect of HER2-ADC (1) and irinotecan.

In the combination administration groups of Test Examples 1 to 14, no significant weight loss was observed compared with each single administration group, confirming that the combination administration poses no safety problem.

### Sequence Listing Free Text

SEQ ID NO: 1: Amino acid sequence of heavy chain of anti-HER2 antibody 1
SEQ ID NO: 2: Amino acid sequence of light chain of anti-HER2 antibody 1
SEQ ID NO: 3: Amino acid sequence of CDRH1 of anti-HER2 antibody 1
SEQ ID NO: 4: Amino acid sequence of CDRH2 of anti-HER2 antibody 1
SEQ ID NO: 5: Amino acid sequence of CDRH3 of anti-HER2 antibody 1
SEQ ID NO: 6: Amino acid sequence of CDRL1 of anti-HER2 antibody 1
SEQ ID NO: 7: Amino acid sequence of CDRL3 of anti-HER2 antibody 1
SEQ ID NO: 8: Amino acid sequence of heavy chain of anti-HER2 antibody 2
SEQ ID NO: 9: Amino acid sequence of light chain of anti-HER2 antibody 2
SEQ ID NO: 10: Amino acid sequence of CDRH1 of anti-HER2 antibody 2
SEQ ID NO: 11: Amino acid sequence of CDRH2 of anti-HER2 antibody 2
SEQ ID NO: 12: Amino acid sequence of CDRH3 of anti-HER2 antibody 2
SEQ ID NO: 13: Amino acid sequence of CDRL1 of anti-HER2 antibody 2
SEQ ID NO: 14: Amino acid sequence of CDRL3 of anti-HER2 antibody 2
SEQ ID NO: 15: Amino acid sequence of heavy chain of anti-HER2 antibody 3
SEQ ID NO: 16: Amino acid sequence of light chain of anti-HER2 antibody 3
SEQ ID NO: 17: Amino acid sequence of CDRH1 of anti-HER2 antibody 3
SEQ ID NO: 18: Amino acid sequence of CDRH2 of anti-HER2 antibody 3
SEQ ID NO: 19: Amino acid sequence of CDRH3 of anti-HER2 antibody 3
SEQ ID NO: 20: Amino acid sequence of CDRL1 of anti-HER2 antibody 3
SEQ ID NO: 21: Amino acid sequence of CDRL3 of anti-HER2 antibody 3

## Claims

1. A pharmaceutical composition comprising a) an anti-HER2 antibody-drug conjugate, wherein
a) the anti-HER2 antibody-drug conjugate, and
b) one or more selected from an immune checkpoint inhibitor, a chemotherapeutic agent, a molecular-targeted drug, an anti-HER2 antibody, and an anti-HER2 antibody-containing compound, provided that the anti-HER2 antibody-drug conjugate described in a) and anti-HER2 antibody-deruxtecan are excluded,
are used in combination, and
the anti-HER2 antibody-drug conjugate is represented by Formula (I) below: 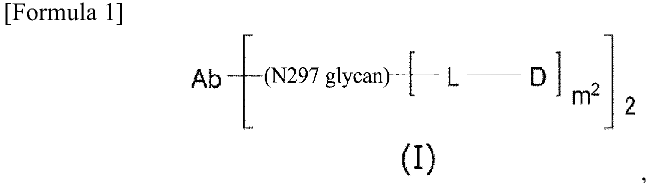
wherein m² represents an integer of 1 or 2;
L represents a linker connecting the N297 glycan and a drug D, and represents -Lb-La-Lp-Lc-*, wherein
the asterisk represents binding to the drug D;
Lb represents: 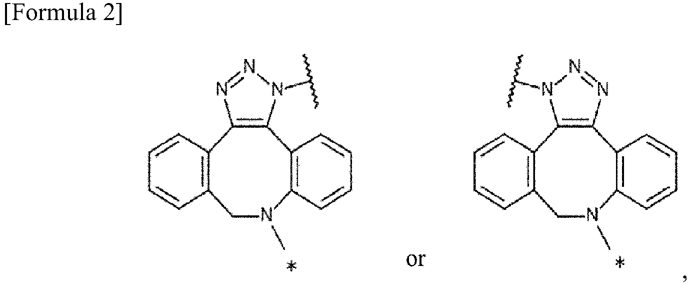
wherein in the structural formula of Lb shown above, the asterisk represents binding to La, and the wavy line represents binding to the N297 glycan;
La represents -C(=O)-CH₂CH₂-C(=O)-;
Lp represents -GGFG- or -GGPI-; and
Lc represents -NH-CH₂-;
Ab is an anti-HER2 antibody comprising a heavy chain comprising CDRH1 consisting of the amino acid sequence represented by SEQ ID NO: 3, CDRH2 consisting of the amino acid sequence represented by SEQ ID NO: 4, and CDRH3 consisting of the amino acid sequence represented by SEQ ID NO: 5, and a light chain comprising CDRL1 consisting of the amino acid sequence represented by SEQ ID NO: 6, CDRL2 consisting of the amino acid sequence represented by SAS, and CDRL3 consisting of the amino acid sequence represented by SEQ ID NO: 7, or an antigen-binding fragment of the antibody;
the N297 glycan is N297-(Fuc)MSG1 having a structure represented by a formula below: 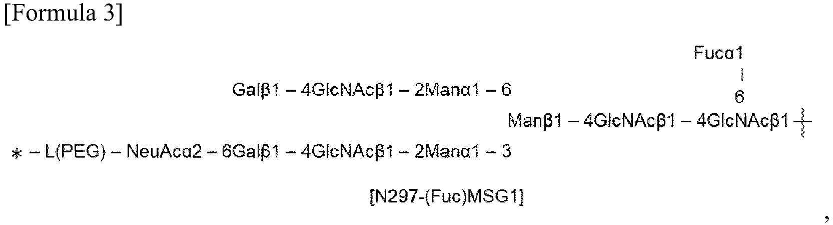
wherein the wavy line represents binding to Asn297 of Ab;
L(PEG) represents -(CH₂-CH₂-O)n⁵-CH₂-CH₂-NH-, and the amino group at the right end of the L(PEG) is amide-linked to the carboxyl group at the 2-position of the non-reducing-end sialic acid on the β-Man 1-3 branch chain of the N297 glycan;
the asterisk represents binding to the nitrogen atom at the 1-position or 3-position on the 1,2,3-triazole ring of Lb in the linker L; and
n⁵ represents an integer of 2 to 5; or
N297-(Fuc)SG having a structure represented by the following formula: 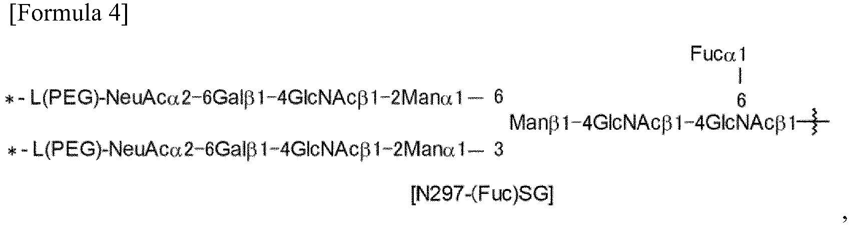
wherein the wavy line represents binding to Asn297 of Ab;
L(PEG) represents -(CH₂-CH₂-O)n⁵-CH₂-CH₂-NH-, and the amino group at the right end of the L(PEG) is amide-linked to the carboxyl group at the 2-position of the non-reducing-end sialic acid on each of the β-Man 1-3 and 1-6 branch chains of the N297 glycan;
the asterisk represents binding to the nitrogen atom at the 1-position or 3-position on the 1,2,3-triazole ring of Lb in the linker L; and
n⁵ represents an integer of 2 to 5; and
D is represented by 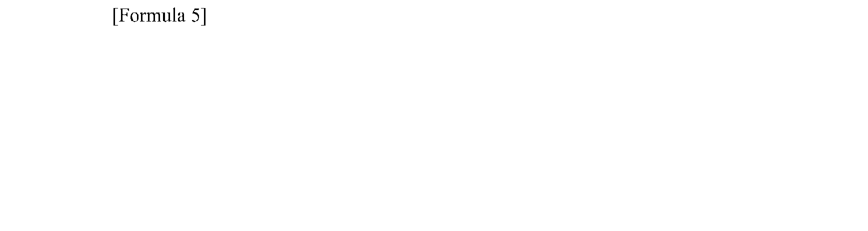 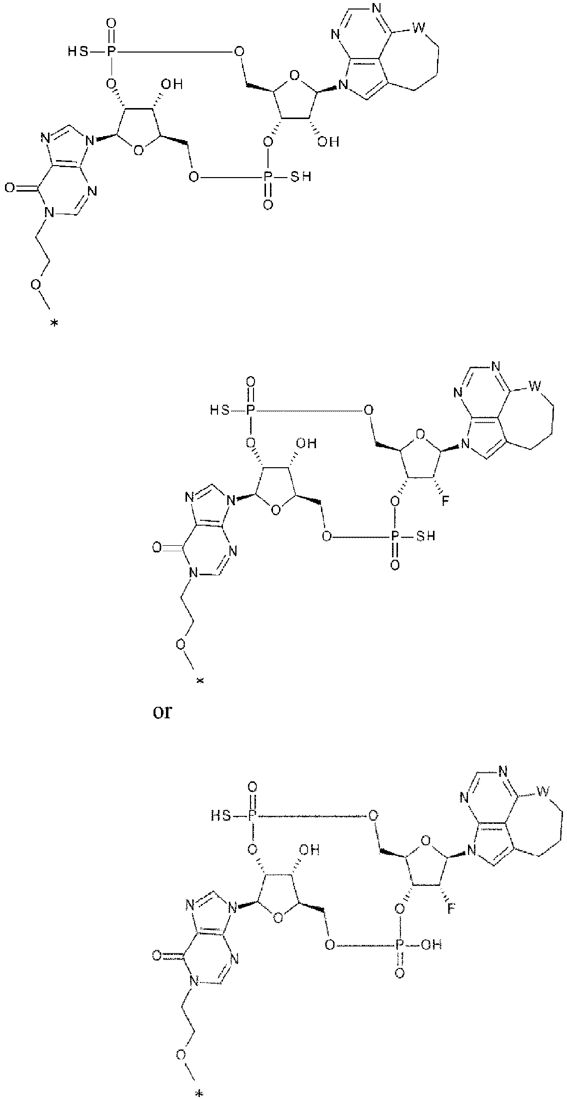
wherein the asterisk represents binding to L, and W represents -NH-.

2. The pharmaceutical composition according to claim 1, wherein
D is represented by 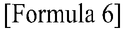 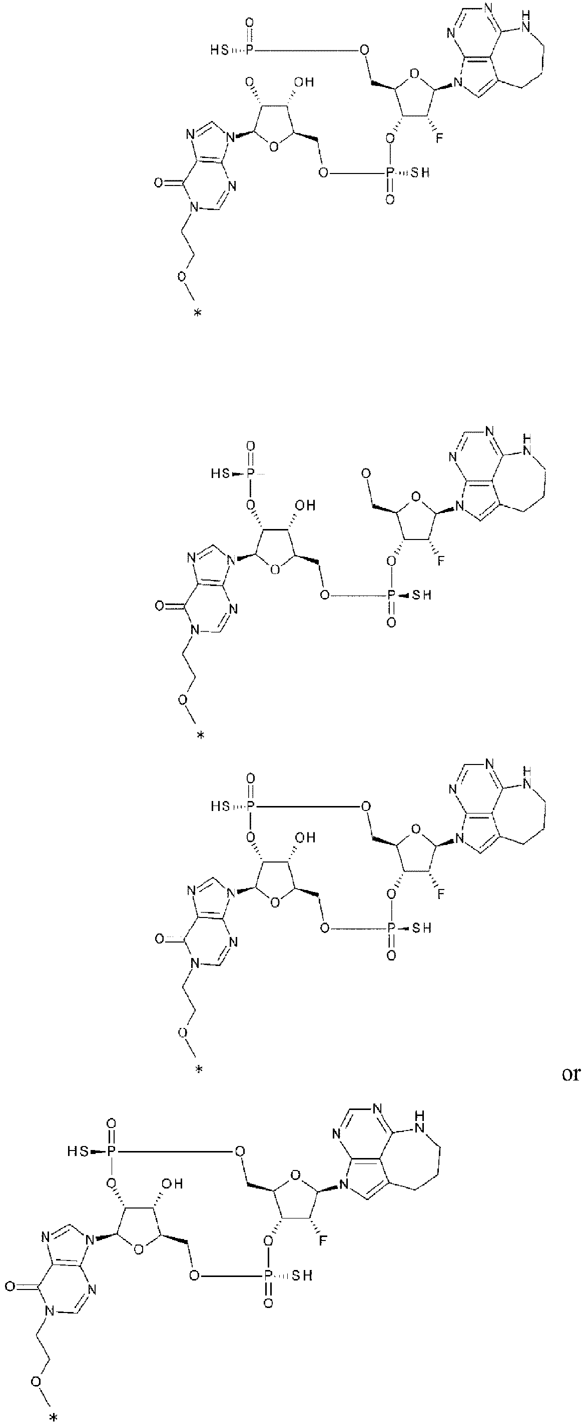
wherein the asterisk represents binding to L.

3. The pharmaceutical composition according to claim 1, wherein the anti-HER2 antibody-drug conjugate is represented by a formula below: 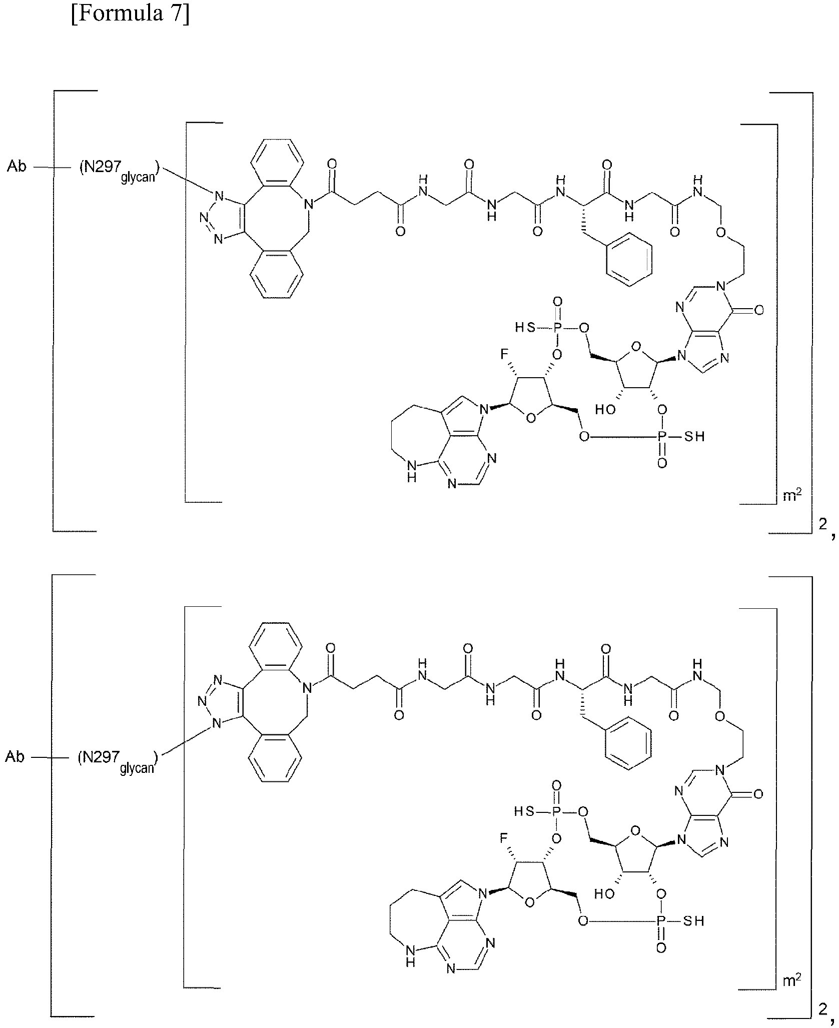 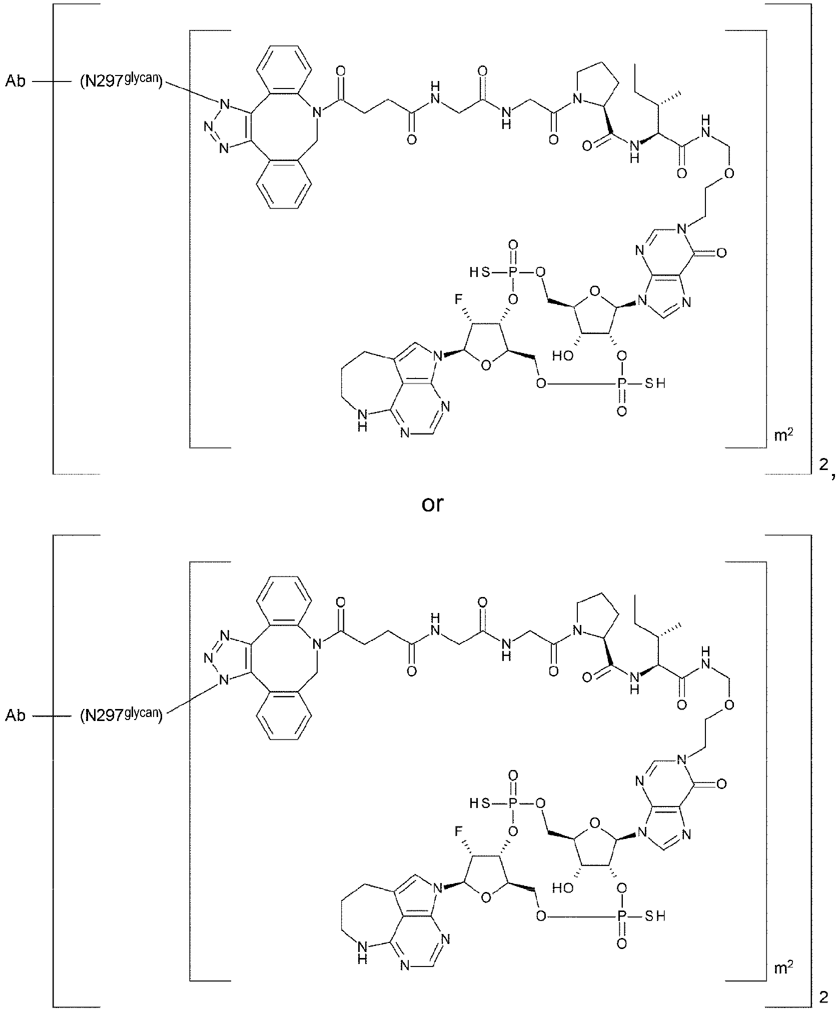 wherein Ab, the N297 glycan, and m² are as defined above.

4. The pharmaceutical composition according to any one of claims 1 to 3, wherein
the anti-HER2 antibody-drug conjugate is represented by a formula below: 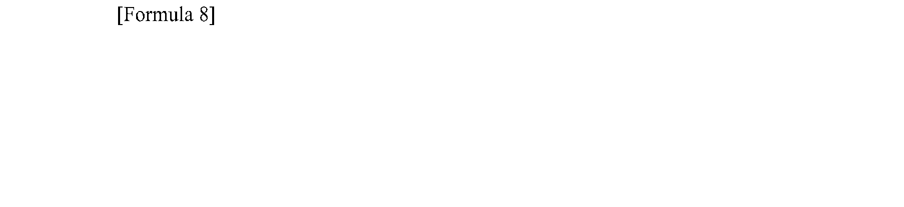 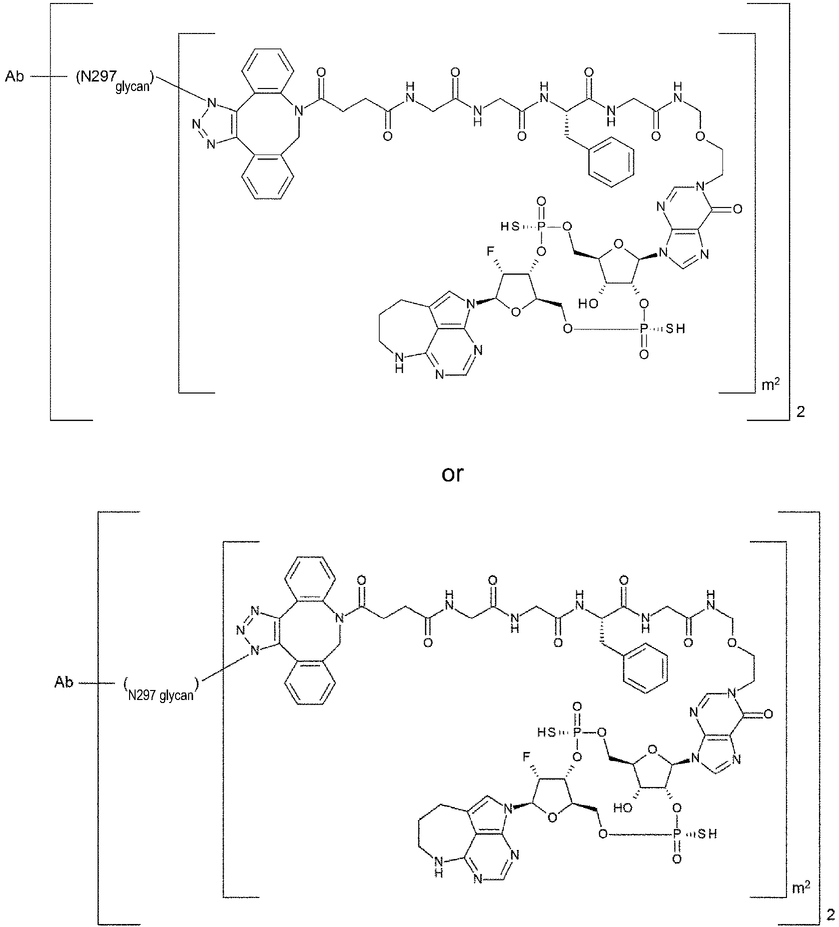
wherein m² is 2;
Ab comprises a heavy chain variable region consisting of an amino acid sequence having 80% or more, preferably 90% or more, and more preferably 95% or more identity with the amino acid sequence represented by the amino acid residues 1 to 119 of SEQ ID NO: 1, and a light chain variable region consisting of an amino acid sequence having 80% or more, preferably 90% or more, and more preferably 95% or more identity with the amino acid sequence represented by the amino acid residues 1 to 108 of SEQ ID NO: 2; and
the N297 glycan is N297-(Fuc)SG having a structure represented by a formula below: 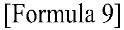 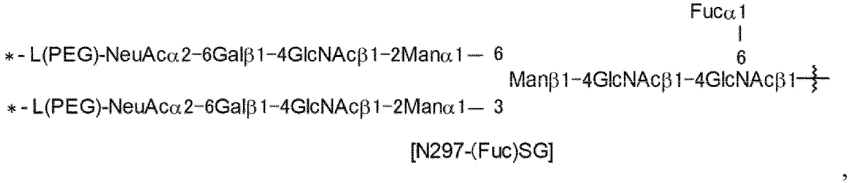
wherein the wavy line represents binding to Asn297 of Ab,
L(PEG) represents -(CH₂-CH₂-O)n⁵-CH₂-CH₂-NH-, and the amino group at the right end of the L(PEG) is amide-linked to the carboxyl group at the 2-position of the non-reducing-end sialic acid on each of the β-Man 1-3 and 1-6 branch chains of the N297 glycan,
the asterisk represents binding to the nitrogen atom at the 1-position or 3-position on the 1,2,3-triazole ring, and
n⁵ represents an integer of 3.

5. The pharmaceutical composition according to any one of claims 1 to 4, wherein Ab comprises a heavy chain variable region consisting of the amino acid sequence represented by the amino acid residues 1 to 119 of SEQ ID NO: 1; and a light chain variable region consisting of the amino acid sequence represented by the amino acid residues 1 to 108 of SEQ ID NO: 2.

6. The pharmaceutical composition according to any one of claims 1 to 5, wherein Ab comprises a heavy chain consisting of an amino acid sequence having 80% or more, preferably 90% or more, and more preferably 95% or more identity with the amino acid sequence represented by SEQ ID NO: 1, and a light chain consisting of an amino acid sequence having 80% or more, preferably 90% or more, and more preferably 95% or more identity with the amino acid sequence represented by SEQ ID NO: 2.

7. The pharmaceutical composition according to any one of claims 1 to 6, wherein Ab comprises a heavy chain consisting of the amino acid sequence represented by SEQ ID NO: 1, and a light chain consisting of the amino acid sequence represented by SEQ ID NO: 2.

8. The pharmaceutical composition according to any one of claims 1 to 5, wherein Ab comprises (i) a heavy chain consisting of an amino acid sequence in which one or two amino acids are deleted from the carboxyl terminus of the amino acid sequence of the heavy chain included in the antibody or the antigen-binding fragment of the antibody included in the pharmaceutical composition described in any one of claims 1 to 7; and (ii) the light chain included in the antibody or the antigen-binding fragment of the antibody included in the pharmaceutical composition described in any one of claims 1 to 7.

9. The pharmaceutical composition according to any one of claims 1 to 8, wherein a) the anti-HER2 antibody-drug conjugate and b) an immune checkpoint inhibitor are used in combination; and the immune checkpoint inhibitor is an antibody selected from the group consisting of an anti-PD-1 antibody, an anti-PD-L1 antibody, an anti-CTLA-4 antibody, an anti-TIGIT antibody, and an anti-LAG-3 antibody; an antigen-binding fragment of the antibody; or a compound including either the antibody or the antigen-binding fragment.

10. The pharmaceutical composition according to claim 9, wherein the anti-PD-1 antibody is nivolumab, pembrolizumab, spartalizumab, cemiplimab, or dostarlimab.

11. The pharmaceutical composition according to claim 9, wherein the anti-PD-L1 antibody is atezolizumab, durvalumab, or avelumab.

12. The pharmaceutical composition according to claim 9, wherein the anti-CTLA-4 antibody is ipilimumab or tremelimumab.

13. The pharmaceutical composition according to claim 9, wherein the anti-TIGIT antibody is tiragolumab or vibostolimab.

14. The pharmaceutical composition according to claim 9, wherein the anti-LAG-3 antibody is relatlimab.

15. The pharmaceutical composition according to any one of claims 1 to 8, wherein a) the anti-HER2 antibody-drug conjugate and b) a chemotherapeutic agent are used in combination, and the chemotherapeutic agent is an antimetabolite, a tubulin inhibitor, an alkylating agent, a platinum-based agent, or a topoisomerase inhibitor.

16. The pharmaceutical composition according to claim 15, wherein the antimetabolite is capecitabine or a pharmaceutically acceptable salt of capecitabine.

17. The pharmaceutical composition according to claim 15, wherein the tubulin inhibitor is paclitaxel or a pharmaceutically acceptable salt of paclitaxel.

18. The pharmaceutical composition according to claim 15, wherein the topoisomerase inhibitor is irinotecan or a pharmaceutically acceptable salt of irinotecan.

19. The pharmaceutical composition according to any one of claims 1 to 8, wherein a) the anti-HER2 antibody-drug conjugate and b) an anti-HER2 antibody are used in combination, and the anti-HER2 antibody described in b) is trastuzumab, pertuzumab, or margetuximab.

20. The pharmaceutical composition according to any one of claims 1 to 8, wherein a) the anti-HER2 antibody-drug conjugate and b) an anti-HER2 antibody-containing compound are used in combination, and the anti-HER2 antibody-containing compound is ado-trastuzumab emtansine (T-DM1), disitamab vedotin, or vic-trastuzumab duocarmazine.

21. The pharmaceutical composition according to any one of claims 1 to 20, wherein
a) the anti-HER2 antibody-drug conjugate; and
b) the immune checkpoint inhibitor, the chemotherapeutic agent, the molecular-targeted drug, the anti-HER2 antibody, or the anti-HER2 antibody-containing compound
are each included as an active ingredient in different formulations and are administered simultaneously or at different times (sequentially or separately).

22. The pharmaceutical composition according to any one of claims 1 to 21, for the treatment of cancer.

23. The pharmaceutical composition according to claim 22, wherein the cancer is at least one selected from the group consisting of lung cancer, colorectal cancer, prostate cancer, pancreatic cancer, ovarian cancer, breast cancer, bladder cancer, gastric cancer, esophageal cancer, endometrial cancer, cervical cancer, head and neck cancer, bile duct cancer, myeloma, and sarcoma.

24. The pharmaceutical composition according to claim 23, wherein the cancer is lung cancer.

25. The pharmaceutical composition according to claim 23, wherein the cancer is colorectal cancer.

26. The pharmaceutical composition according to claim 23, wherein the cancer is breast cancer.

27. The pharmaceutical composition according to claim 23, wherein the cancer is gastric cancer.

28. The pharmaceutical composition according to claim 23, wherein the cancer is bladder cancer.

29. An anti-HER2 antibody-drug conjugate for use in treating cancer in combination with one or more selected from an immune checkpoint inhibitor, a chemotherapeutic agent, a molecular-targeted drug, an anti-HER2 antibody, and an anti-HER2 antibody-containing compound, provided that the anti-HER2 antibody-drug conjugate represented by Formula (I) described below and anti-HER2 antibody-deruxtecan are excluded, wherein
the anti-HER2 antibody-drug conjugate is represented by Formula (I) below: 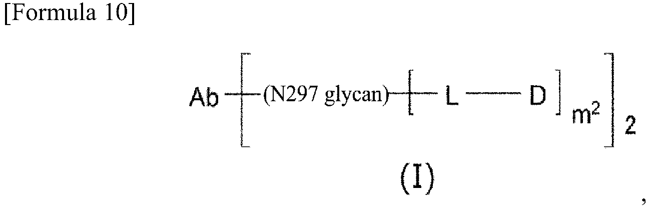
wherein m² represents an integer of 1 or 2;
L represents a linker connecting the N297 glycan and a drug D, and represents -Lb-La-Lp-Lc-*, wherein
the asterisk represents binding to the drug D;
Lb represents: 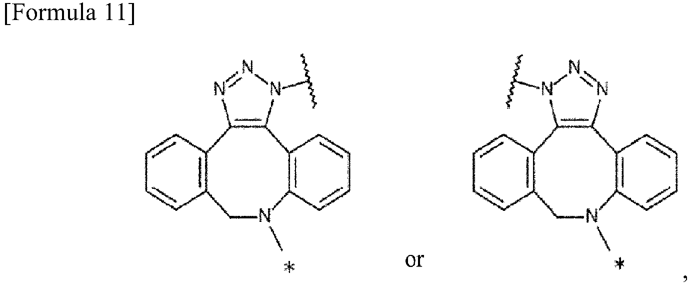
wherein in the structural formula of Lb shown above, the asterisk represents binding to La, and the wavy line represents binding to the N297 glycan;
La represents -C(=O)-CH₂CH₂-C(=O)-;
Lp represents -GGFG- or -GGPI-; and
Lc represents -NH-CH₂-;
Ab is an anti-HER2 antibody comprising a heavy chain comprising CDRH1 consisting of the amino acid sequence represented by SEQ ID NO: 3, CDRH2 consisting of the amino acid sequence represented by SEQ ID NO: 4, and CDRH3 consisting of the amino acid sequence represented by SEQ ID NO: 5, and a light chain comprising CDRL1 consisting of the amino acid sequence represented by SEQ ID NO: 6, CDRL2 consisting of the amino acid sequence represented by SAS, and CDRL3 consisting of the amino acid sequence represented by SEQ ID NO: 7, or an antigen-binding fragment of the antibody;
the N297 glycan is N297-(Fuc)MSG1 having a structure represented by a formula below: 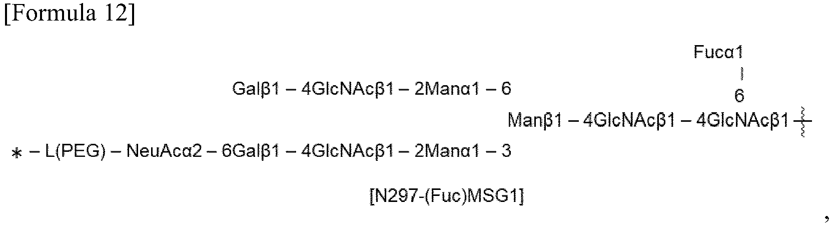
wherein the wavy line represents binding to Asn297 of Ab;
L(PEG) represents -(CH₂-CH₂-O)n⁵-CH₂-CH₂-NH-, and the amino group at the right end of the L(PEG) is amide-linked to the carboxyl group at the 2-position of the non-reducing-end sialic acid on the β-Man 1-3 branch chain of the N297 glycan;
the asterisk represents binding to the nitrogen atom at the 1-position or 3-position on the 1,2,3-triazole ring of Lb in the linker L; and
n⁵ represents an integer of 2 to 5; or
N297-(Fuc)SG having a structure represented by the following formula: 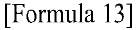 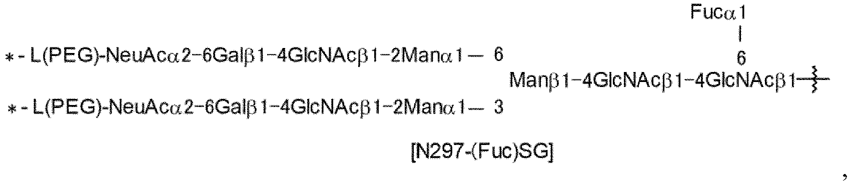
wherein the wavy line represents binding to Asn297 of Ab;
L(PEG) represents -(CH₂-CH₂-O)n⁵-CH₂-CH₂-NH-, and the amino group at the right end of the L(PEG) is amide-linked to the carboxyl group at the 2-position of the non-reducing-end sialic acid on each of the β-Man 1-3 and 1-6 branch chains of the N297 glycan;
the asterisk represents binding to the nitrogen atom at the 1-position or 3-position on the 1,2,3-triazole ring of Lb in the linker L; and
n⁵ represents an integer of 2 to 5; and
D is 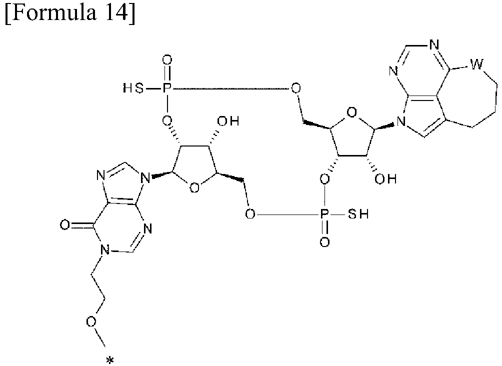 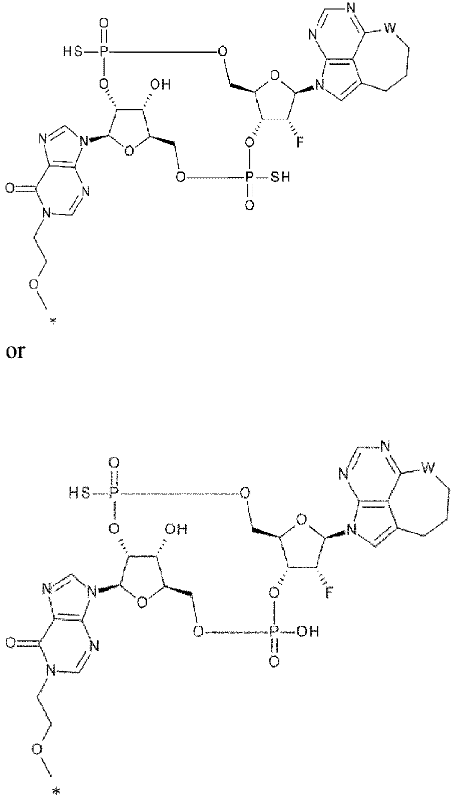
wherein the asterisk represents binding to L, and W represents -NH-.

30. The anti-HER2 antibody-drug conjugate according to claim 29, wherein
D is represented by 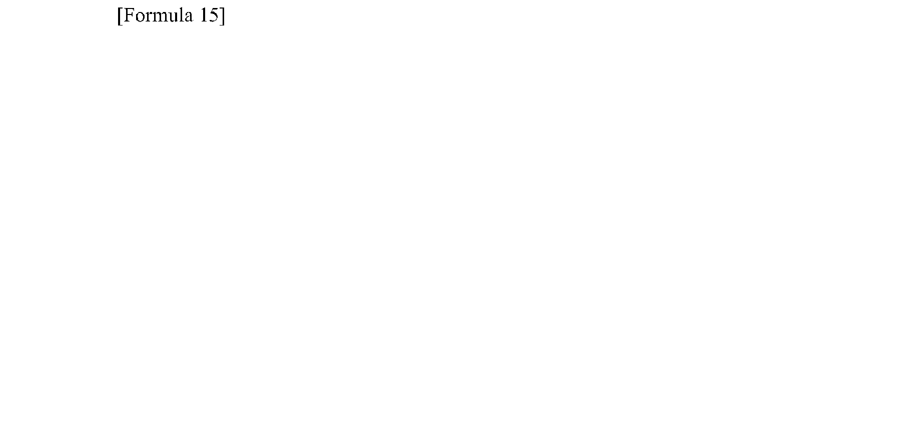 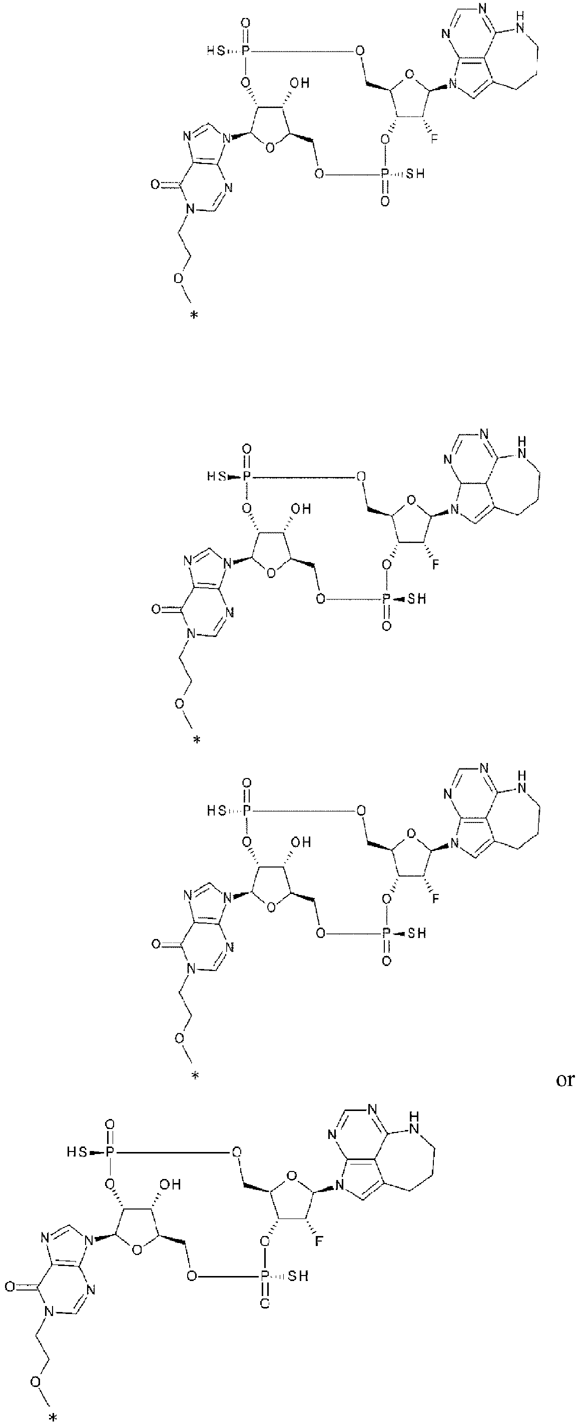
wherein the asterisk represents binding to L.

31. The anti-HER2 antibody-drug conjugate according to claim 29, wherein the anti-HER2 antibody-drug conjugate is represented by a formula below: 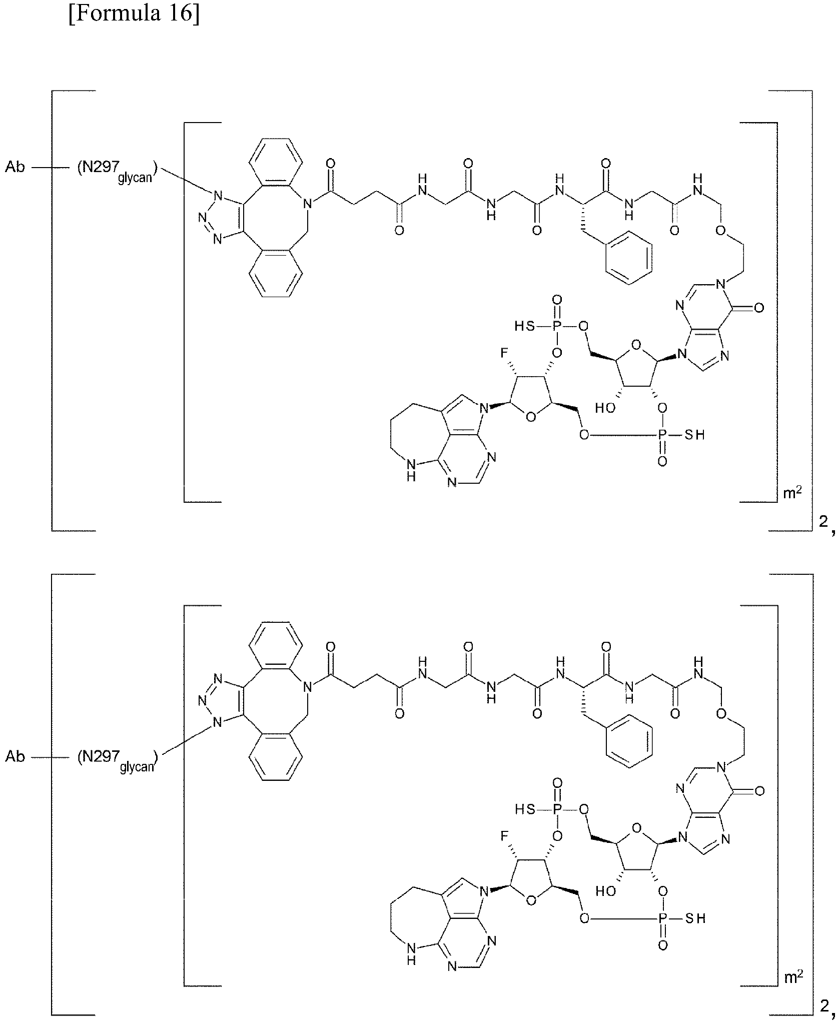 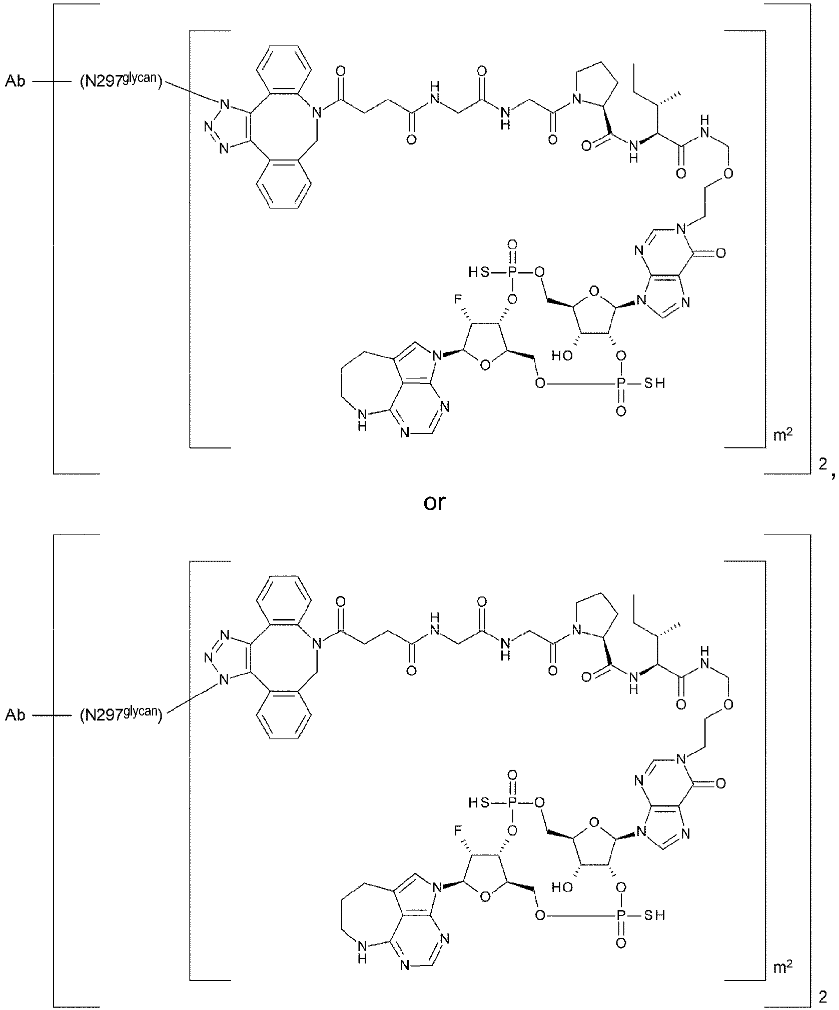 wherein Ab, the N297 glycan, and m² are as defined above.

32. The anti-HER2 antibody-drug conjugate according to any one of claims 29 to 31, wherein
the anti-HER2 antibody-drug conjugate is represented by a formula below: 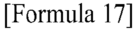 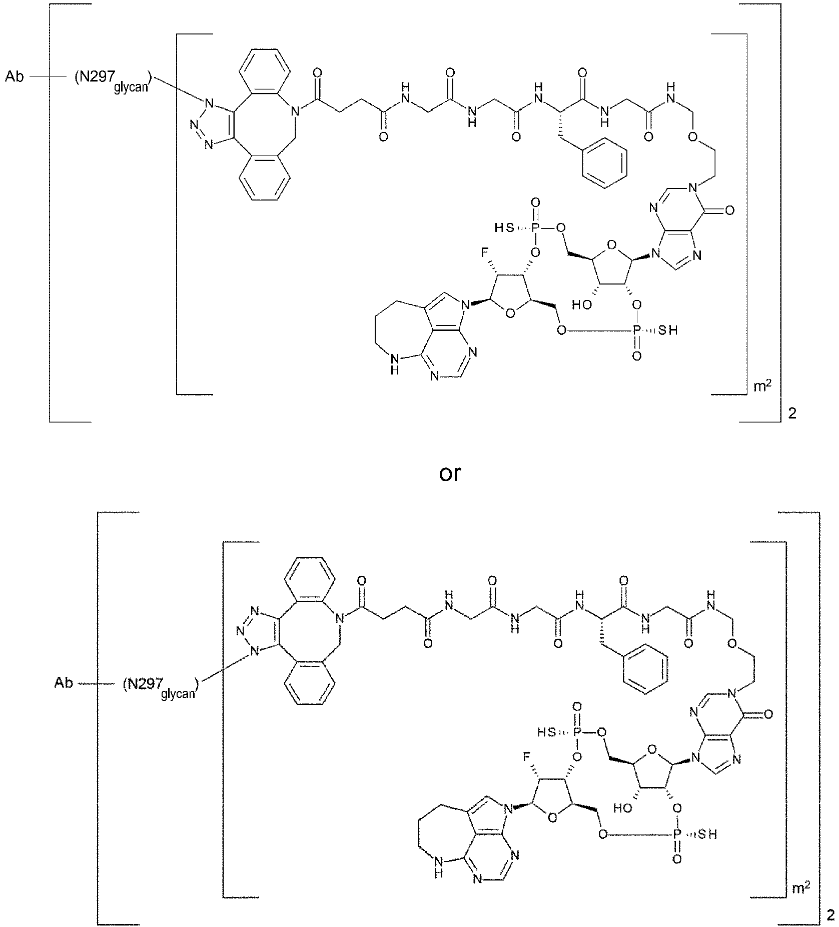
wherein m² is 2;
Ab comprises a heavy chain variable region consisting of an amino acid sequence having 80% or more, preferably 90% or more, and more preferably 95% or more identity with the amino acid sequence represented by the amino acid residues 1 to 119 of SEQ ID NO: 1, and a light chain variable region consisting of an amino acid sequence having 80% or more, preferably 90% or more, and more preferably 95% or more identity with the amino acid sequence represented by the amino acid residues 1 to 108 of SEQ ID NO: 2; and
the N297 glycan is N297-(Fuc)SG having a structure represented by a formula below: 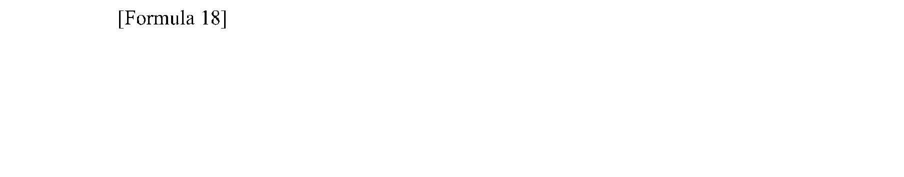 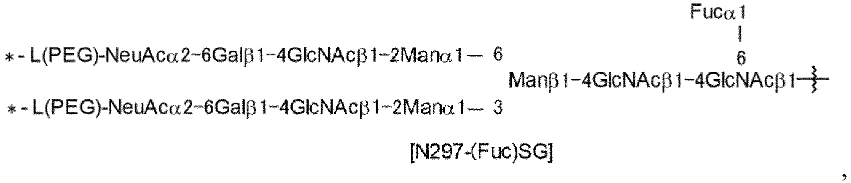
wherein the wavy line represents binding to Asn297 of Ab,
L(PEG) represents -(CH₂-CH₂-O)n⁵-CH₂-CH₂-NH-, and the amino group at the right end of the L(PEG) is amide-linked to the carboxyl group at the 2-position of the non-reducing-end sialic acid on each of the β-Man 1-3 and 1-6 branch chains of the N297 glycan,
the asterisk represents binding to the nitrogen atom at the 1-position or 3-position on the 1,2,3-triazole ring, and
n⁵ represents an integer of 3.

33. The anti-HER2 antibody-drug conjugate according to any one of claims 29 to 32,
wherein Ab comprises a heavy chain variable region consisting of the amino acid sequence represented by the amino acid residues 1 to 119 of SEQ ID NO: 1, and a light chain variable region consisting of the amino acid sequence represented by the amino acid residues 1 to 108 of SEQ ID NO: 2.

34. The anti-HER2 antibody-drug conjugate according to any one of claims 29 to 33,
wherein Ab comprises a heavy chain consisting of an amino acid sequence having 80% or more, preferably 90% or more, and more preferably 95% or more identity with the amino acid sequence represented by SEQ ID NO: 1, and a light chain consisting of an amino acid sequence having 80% or more, preferably 90% or more, and more preferably 95% or more identity with the amino acid sequence represented by SEQ ID NO: 2.

35. The anti-HER2 antibody-drug conjugate according to any one of claims 29 to 34,
wherein Ab comprises a heavy chain consisting of the amino acid sequence represented by SEQ ID NO: 1, and a light chain consisting of the amino acid sequence represented by SEQ ID NO: 2.

36. The anti-HER2 antibody-drug conjugate according to any one of claims 29 to 33,
wherein Ab comprises (i) a heavy chain consisting of an amino acid sequence in which one or two amino acids are deleted from the carboxyl terminus of the amino acid sequence of the heavy chain included in the antibody or the antigen-binding fragment of the antibody included in the anti-HER2 antibody-drug conjugate described in any one of claims 29 to 35; and (ii) the light chain included in the antibody or the antigen-binding fragment of the antibody included in the anti-HER2 antibody-drug conjugate described in any one of claims 29 to 35.

37. The anti-HER2 antibody-drug conjugate according to any one of claims 29 to 36, for use in treating cancer in combination with an immune checkpoint inhibitor, wherein the immune checkpoint inhibitor is an antibody selected from the group consisting of an anti-PD-1 antibody, an anti-PD-L1 antibody, an anti-CTLA-4 antibody, an anti-TIGIT antibody, and an anti-LAG-3 antibody; an antigen-binding fragment of the antibody; or a compound including either the antibody or the antigen-binding fragment.

38. The anti-HER2 antibody-drug conjugate according to claim 37, wherein the anti-PD-1 antibody is nivolumab, pembrolizumab, spartalizumab, cemiplimab, or dostarlimab.

39. The anti-HER2 antibody-drug conjugate according to claim 37, wherein the anti-PD-L1 antibody is atezolizumab, durvalumab, or avelumab.

40. The anti-HER2 antibody-drug conjugate according to claim 37, wherein the anti-CTLA-4 antibody is ipilimumab or tremelimumab.

41. The anti-HER2 antibody-drug conjugate according to claim 37, wherein the anti-TIGIT antibody is tiragolumab or vibostolimab.

42. The anti-HER2 antibody-drug conjugate according to claim 37, wherein the anti-LAG-3 antibody is relatlimab.

43. The anti-HER2 antibody-drug conjugate according to any one of claims 29 to 36, for use in treating cancer in combination with a chemotherapeutic agent, wherein the chemotherapeutic agent is an antimetabolite, a tubulin inhibitor, an alkylating agent, a platinum-based agent, or a topoisomerase inhibitor.

44. The anti-HER2 antibody-drug conjugate according to claim 43, wherein the antimetabolite is capecitabine or a pharmaceutically acceptable salt of capecitabine.

45. The anti-HER2 antibody-drug conjugate according to claim 43, wherein the tubulin inhibitor is paclitaxel or a pharmaceutically acceptable salt of paclitaxel.

46. The anti-HER2 antibody-drug conjugate according to claim 43, wherein the topoisomerase inhibitor is irinotecan or a pharmaceutically acceptable salt of irinotecan.

47. The anti-HER2 antibody-drug conjugate according to any one of claims 29 to 36, for use in treating cancer in combination with an anti-HER2 antibody, wherein the anti-HER2 antibody used in combination with the anti-HER2 antibody-drug conjugate is trastuzumab, pertuzumab, or margetuximab.

48. The anti-HER2 antibody-drug conjugate according to any one of claims 29 to 36, for use in treating cancer in combination with an anti-HER2 antibody-containing compound, wherein the anti-HER2 antibody-containing compound is ado-trastuzumab emtansine (T-DM1), disitamab vedotin, or vic-trastuzumab duocarmazine.

49. The anti-HER2 antibody-drug conjugate according to any one of claims 29 to 48, wherein
the anti-HER2 antibody-drug conjugate; and
the immune checkpoint inhibitor, the chemotherapeutic agent, the molecular-targeted drug, the anti-HER2 antibody, or the anti-HER2 antibody-containing compound
are each included as an active ingredient in different formulations and are administered simultaneously or at different times (sequentially or separately).

50. The anti-HER2 antibody-drug conjugate according to any one of claims 29 to 49, wherein the cancer is selected from the group consisting of lung cancer, colorectal cancer, prostate cancer, pancreatic cancer, ovarian cancer, breast cancer, bladder cancer, gastric cancer, esophageal cancer, endometrial cancer, cervical cancer, head and neck cancer, bile duct cancer, myeloma, and sarcoma.

51. The anti-HER2 antibody-drug conjugate according to claim 50, wherein the cancer is lung cancer.

52. The anti-HER2 antibody-drug conjugate according to claim 50, wherein the cancer is colorectal cancer.

53. The anti-HER2 antibody-drug conjugate according to claim 50, wherein the cancer is breast cancer.

54. The anti-HER2 antibody-drug conjugate according to claim 50, wherein the cancer is gastric cancer.

55. The anti-HER2 antibody-drug conjugate according to claim 50, wherein the cancer is bladder cancer.

56. A method of treating a disease, the method comprising administering in combination to a subject in need of treatment of the disease:
a) an anti-HER2 antibody-drug conjugate; and
b) one or more selected from an immune checkpoint inhibitor, a chemotherapeutic agent, a molecular-targeted drug, an anti-HER2 antibody, and an anti-HER2 antibody-containing compound, provided that the anti-HER2 antibody-drug conjugate described in a) and anti-HER2 antibody-deruxtecan are excluded, wherein
the anti-HER2 antibody-drug conjugate is represented by Formula (I) below: 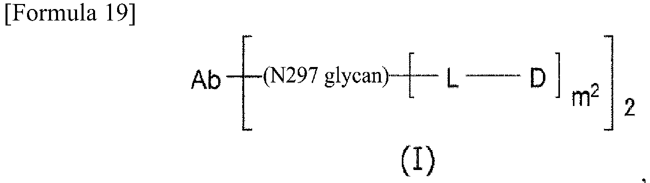
wherein m² represents an integer of 1 or 2;
L represents a linker connecting the N297 glycan and a drug D, and represents -Lb-La-Lp-Lc-*, wherein
the asterisk represents binding to the drug D;
Lb represents: 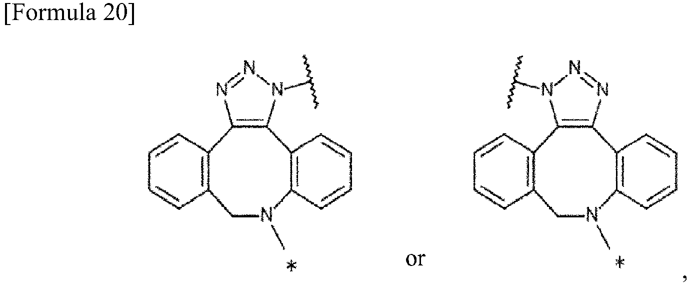
wherein in the structural formula of Lb shown above, the asterisk represents binding to La, and the wavy line represents binding to the N297 glycan;
La represents -C(=O)-CH₂CH₂-C(=O)-;
Lp represents -GGFG- or -GGPI-; and
Lc represents -NH-CH₂-;
Ab is an anti-HER2 antibody comprising a heavy chain comprising CDRH1 consisting of the amino acid sequence represented by SEQ ID NO: 3, CDRH2 consisting of the amino acid sequence represented by SEQ ID NO: 4, and CDRH3 consisting of the amino acid sequence represented by SEQ ID NO: 5, and a light chain comprising CDRL1 consisting of the amino acid sequence represented by SEQ ID NO: 6, CDRL2 consisting of the amino acid sequence represented by SAS, and CDRL3 consisting of the amino acid sequence represented by SEQ ID NO: 7, or an antigen-binding fragment of the antibody;
the N297 glycan is N297-(Fuc)MSG1 having a structure represented by a formula below: 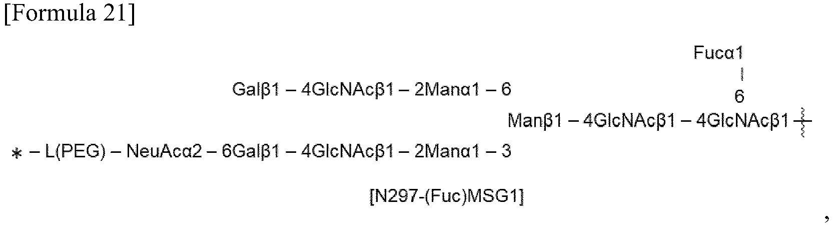
wherein the wavy line represents binding to Asn297 of Ab;
L(PEG) represents -(CH₂-CH₂-O)n⁵-CH₂-CH₂-NH-, and the amino group at the right end of the L(PEG) is amide-linked to the carboxyl group at the 2-position of the non-reducing-end sialic acid on the β-Man 1-3 branch chain of the N297 glycan;
the asterisk represents binding to the nitrogen atom at the I-position or 3-position on the 1,2,3-triazole ring of Lb in the linker L; and
n⁵ represents an integer of 2 to 5; or
N297-(Fuc)SG having a structure represented by the following formula: 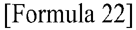 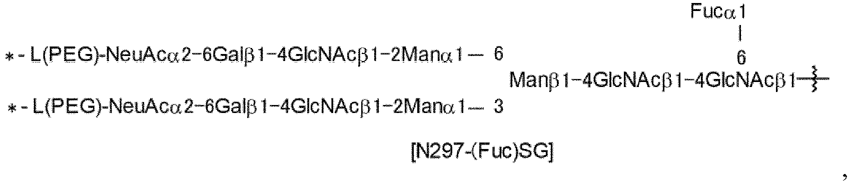
wherein the wavy line represents binding to Asn297 of Ab;
L(PEG) represents -(CH₂-CH₂-O)n⁵-CH₂-CH₂-NH-, and the amino group at the right end of the L(PEG) is amide-linked to the carboxyl group at the 2-position of the non-reducing-end sialic acid on each of the β-Man 1-3 and 1-6 branch chains of the N297 glycan;
the asterisk represents binding to the nitrogen atom at the 1-position or 3-position on the 1,2,3-triazole ring of Lb in the linker L; and
n⁵ represents an integer of 2 to 5; and
D is 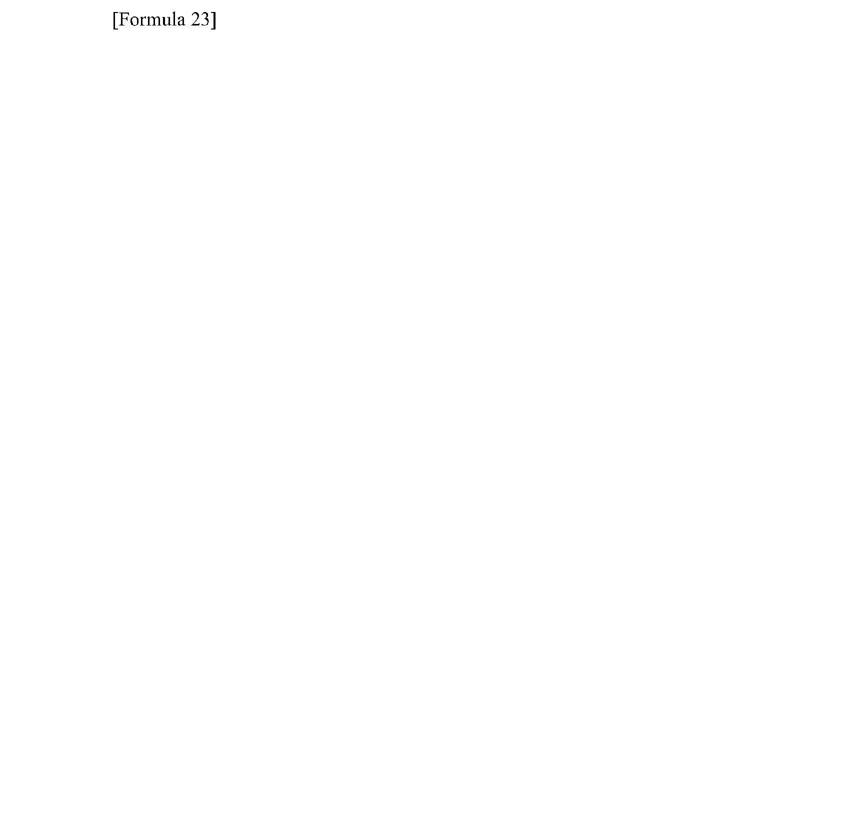 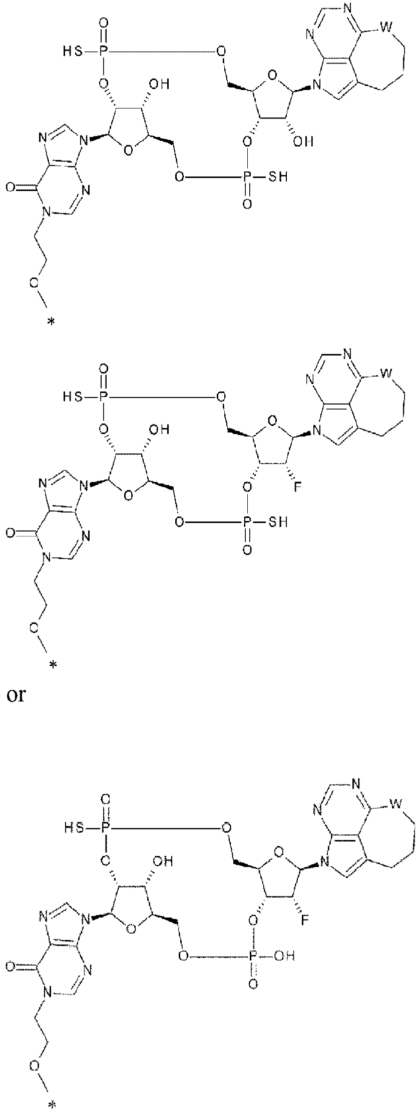
wherein the asterisk represents binding to L, and W represents -NH-.

57. The method according to claim 56, wherein
D is represented by 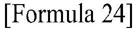 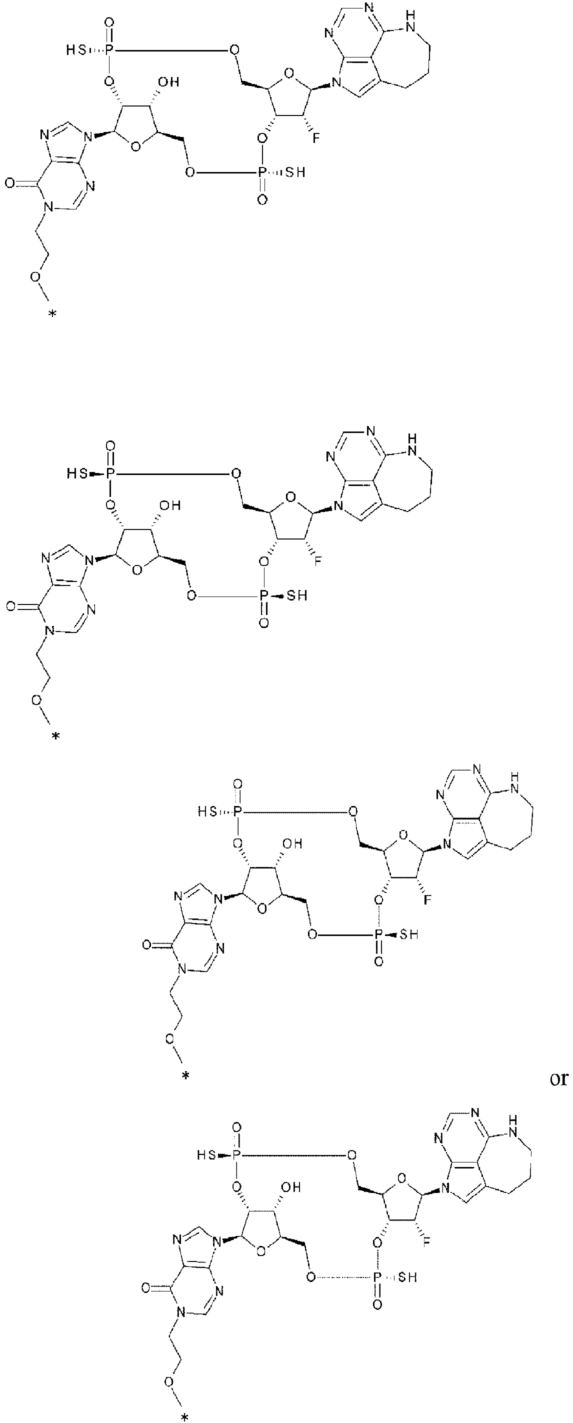
wherein the asterisk represents binding to L.

58. The method according to claim 56, wherein the anti-HER2 antibody-drug conjugate is represented by a formula below: 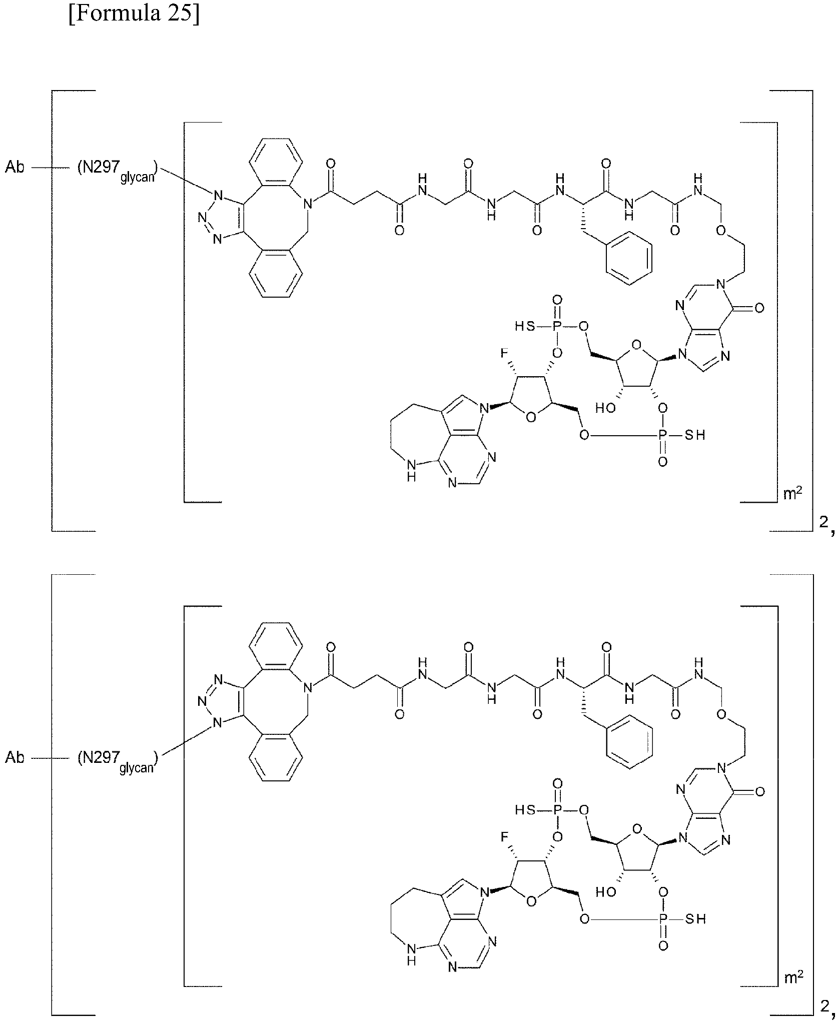 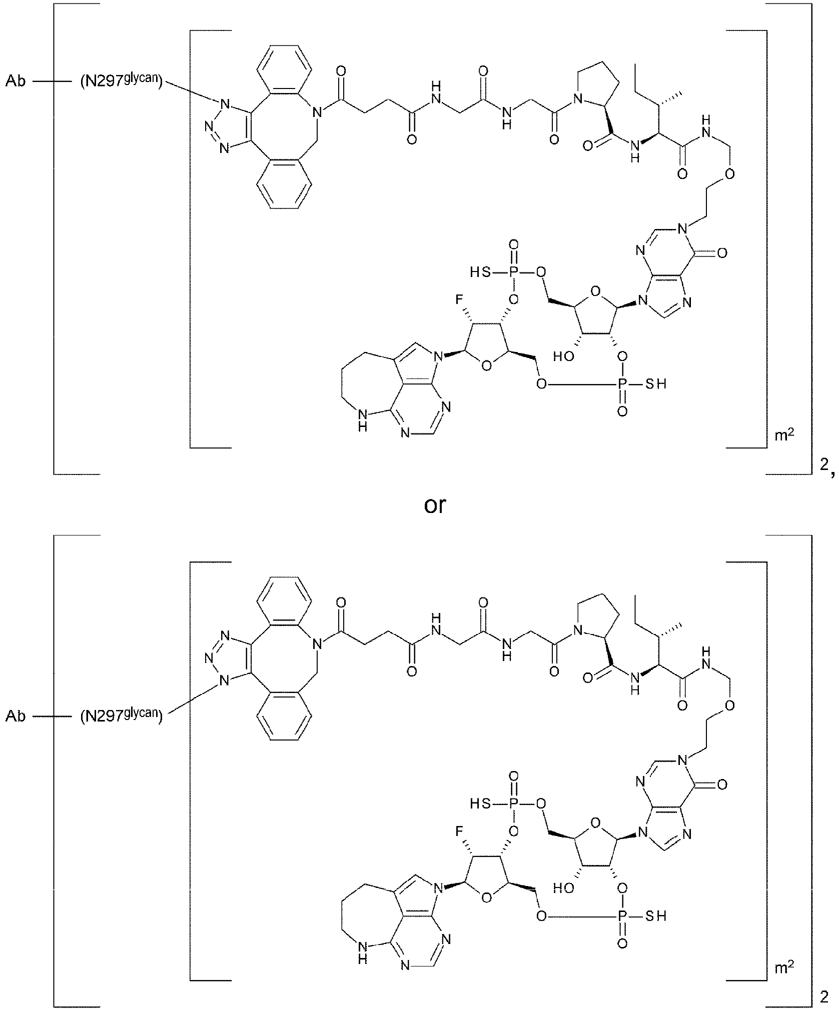 wherein Ab, the N297 glycan, and m² are as defined above.

59. The method according to any one of claims 56 to 58, wherein
the anti-HER2 antibody-drug conjugate is represented by a formula below: 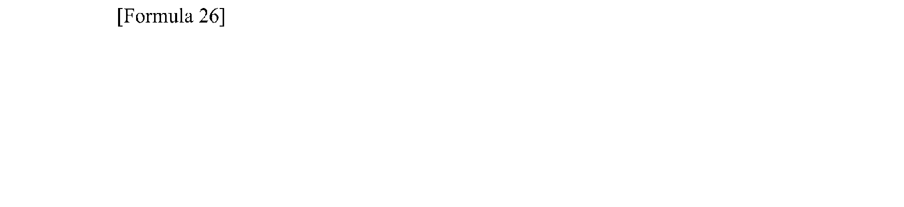 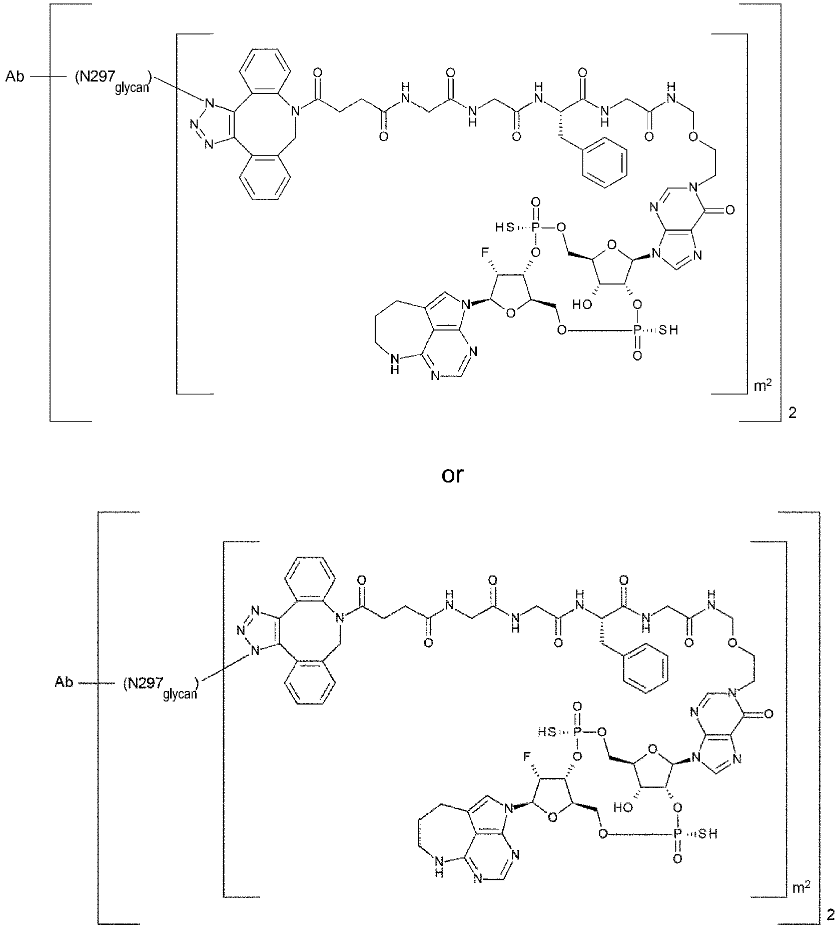
wherein m² is 2;
Ab comprises a heavy chain variable region consisting of an amino acid sequence having 80% or more, preferably 90% or more, and more preferably 95% or more identity with the amino acid sequence represented by the amino acid residues 1 to 119 of SEQ ID NO: 1, and a light chain variable region consisting of an amino acid sequence having 80% or more, preferably 90% or more, and more preferably 95% or more identity with the amino acid sequence represented by the amino acid residues 1 to 108 of SEQ ID NO: 2; and
the N297 glycan is N297-(Fuc)SG having a structure represented by a formula below: 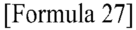 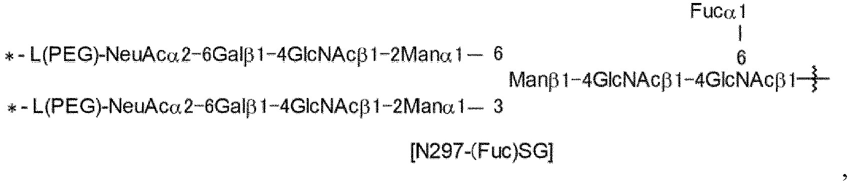
wherein the wavy line represents binding to Asn297 of Ab,
L(PEG) represents -(CH₂-CH₂-O)n⁵-CH₂-CH₂-NH-, and the amino group at the right end of the L(PEG) is amide-linked to the carboxyl group at the 2-position of the non-reducing-end sialic acid on each of the β-Man 1-3 and 1-6 branch chains of the N297 glycan,
the asterisk represents binding to the nitrogen atom at the 1-position or 3-position on the 1,2,3-triazole ring, and
n⁵ represents an integer of 3.

60. The method according to any one of claims 56 to 59, wherein Ab comprises a heavy chain variable region consisting of the amino acid sequence represented by the amino acid residues 1 to 119 of SEQ ID NO: 1, and a light chain variable region consisting of the amino acid sequence represented by the amino acid residues 1 to 108 of SEQ ID NO: 2.

61. The method according to any one of claims 56 to 60, wherein Ab comprises a heavy chain consisting of an amino acid sequence having 80% or more, preferably 90% or more, and more preferably 95% or more identity with the amino acid sequence represented by SEQ ID NO: 1, and a light chain consisting of an amino acid sequence having 80% or more, preferably 90% or more, and more preferably 95% or more identity with the amino acid sequence represented by SEQ ID NO: 2.

62. The method according to any one of claims 56 to 61, wherein Ab comprises a heavy chain consisting of the amino acid sequence represented by SEQ ID NO: 1 and a light chain consisting of the amino acid sequence represented by SEQ ID NO: 2.

63. The method according to any one of claims 56 to 60, wherein Ab comprises (i) a heavy chain consisting of an amino acid sequence in which one or two amino acids are deleted from the carboxyl terminus of the amino acid sequence of the heavy chain included in the antibody or the antigen-binding fragment of the antibody included in the anti-HER2 antibody-drug conjugate described in any one of claims 56 to 62; and (ii) the light chain included in the antibody or the antigen-binding fragment of the antibody included in the anti-HER2 antibody-drug conjugate described in any one of claims 56 to 62.

64. The method of treating a disease according to any one of claims 56 to 63 comprising administering in combination to a subject in need of treatment of the disease: a) the anti-HER2 antibody-drug conjugate; and b) an immune checkpoint inhibitor, wherein the immune checkpoint inhibitor is an antibody selected from the group consisting of an anti-PD-1 antibody, an anti-PD-L1 antibody, an anti-CTLA-4 antibody, an anti-TIGIT antibody, and an anti-LAG-3 antibody; an antigen-binding fragment of the antibody; or a compound including either the antibody or the antigen-binding fragment.

65. The method according to claim 64, wherein the anti-PD-1 antibody is nivolumab, pembrolizumab, spartalizumab, cemiplimab, or dostarlimab.

66. The method according to claim 64, wherein the anti-PD-L1 antibody is atezolizumab, durvalumab, or avelumab.

67. The method according to claim 64, wherein the anti-CTLA-4 antibody is ipilimumab or tremelimumab.

68. The method according to claim 64, wherein the anti-TIGIT antibody is tiragolumab or vibostolimab.

69. The method according to claim 64, wherein the anti-LAG-3 antibody is relatlimab.

70. The method of treating a disease according to any one of claims 56 to 63 comprising administering in combination to a subject in need of treatment of the disease: a) the anti-HER2 antibody-drug conjugate; and b) a chemotherapeutic agent, wherein the chemotherapeutic agent is an antimetabolite, a tubulin inhibitor, an alkylating agent, a platinum-based agent, or a topoisomerase inhibitor.

71. The method according to claim 70, wherein the antimetabolite is capecitabine or a pharmaceutically acceptable salt of capecitabine.

72. The method according to claim 70, wherein the tubulin inhibitor is paclitaxel or a pharmaceutically acceptable salt of paclitaxel.

73. The method according to claim 70, wherein the topoisomerase inhibitor is irinotecan or a pharmaceutically acceptable salt of irinotecan.

74. The method of treating a disease according to any one of claims 56 to 63 comprising administering in combination to a subject in need of treatment of the disease: a) the anti-HER2 antibody-drug conjugate; and b) an anti-HER2 antibody, wherein the anti-HER2 antibody described in b) is trastuzumab, pertuzumab, or margetuximab.

75. The method of treating a disease according to any one of claims 56 to 63 comprising administering in combination to a subject in need of treatment of the disease: a) the anti-HER2 antibody-drug conjugate; and b) an anti-HER2 antibody-containing compound, wherein the anti-HER2 antibody-containing compound is ado-trastuzumab emtansine (T-DM1), disitamab vedotin, or vic-trastuzumab duocarmazine.

76. The method according to any one of claims 56 to 75, wherein
the anti-HER2 antibody-drug conjugate; and
the immune checkpoint inhibitor, the chemotherapeutic agent, the molecular-targeted drug, the anti-HER2 antibody, or the anti-HER2 antibody-containing compound
are each included as an active ingredient in different formulations and are administered simultaneously or at different times (sequentially or separately).

77. The method according to any one of claims 56 to 76, wherein the disease is cancer.

78. The method according to claim 77, wherein the cancer is selected from the group consisting of lung cancer, colorectal cancer, prostate cancer, pancreatic cancer, ovarian cancer, breast cancer, bladder cancer, gastric cancer, esophageal cancer, endometrial cancer, cervical cancer, head and neck cancer, bile duct cancer, myeloma, and sarcoma.

79. The method according to claim 77, wherein the cancer is lung cancer.

80. The method according to claim 77, wherein the cancer is colorectal cancer.

81. The method according to claim 77, wherein the cancer is breast cancer.

82. The method according to claim 77, wherein the cancer is gastric cancer.

83. The method according to claim 77, wherein the cancer is bladder cancer.
